# EUROPEAN PATENT APPLICATION

(11) **EP 4 793 640 A1**
(43) Date of publication of application: **19.08.2026**
(21) Application number: 24853838.1
(22) Date of filing: 14.08.2024
(51) Int. Cl.: G01N 33/48

(54) **MYOCARDIAL DAMAGE ASSESSMENT METHOD, APPARATUS, SAMPLE ANALYSIS SYSTEM AND USE THEREOF**

(30) Priority: 14.08.2023 CN 202311024062
(71) Applicant: Shenzhen Mindray Bio-Medical Electronics Co., Ltd., Shenzhen, Guangdong 518057 (CN)
(72) Inventor: ZHANG, Yi, Shenzhen, Guangdong 518057 (CN); LIU, Yuqing, Shenzhen, Guangdong 518057 (CN); LI, Ke, Shenzhen, Guangdong 518057 (CN); KATRUKHA, Ivan, Moscow 117186 (RU); KOGAN, Alexander E., Moscow 117186 (RU)
(74) Representative: KIPA AB
(86) International application number: PCT/CN2024/112205
(87) International publication number: WO 2025/036431

(57) **Abstract**

The present application relates to a method, device, sample analysis system and use thereof for assessing myocardial injury. The method, device and sample analysis system can rapidly and accurately assess the cause and risk of individual myocardial injury and guide diagnostic and treatment decisions.

## Description

The present application is based on the application with CN application number of 202311024062.1 and application date of August 14, 2023, and claims its priority. The disclosure of the CN application is hereby introduced into the present application in its entirety.

### Technical Field

The present application belongs to the field of disease diagnosis, and specifically relates to a method for *in vitro* assessment of myocardial injury in a subject, a device and a sample analysis system for obtaining characteristic parameters for assessment of myocardial injury in a subject, and use of a reagent for quantitative detection of a large-size cardiac troponin ternary complex and/or a reagent for quantitative detection of a total cardiac troponin ternary complex in the preparation of a kit.

### Background Art

Cardiovascular diseases seriously threaten human health. In China, the incidence and mortality of cardiovascular diseases have been increasing year by year. Chest pain is a common symptom of various cardiovascular diseases, accompanied by various clinical manifestations, often associated with dyspnea. The risk of acute fatal chest pain is extremely high. For emergency department chest pain patients, accurate risk stratification, diagnosis, and the establishment of a rapid and reasonable diagnostic protocol are essential for correct management and personalized treatment decisions. Distinguishing the causes of chest pain, especially acute vs. non-acute myocardial injury, is of important clinical significance and meets urgent clinical needs.

Cardiac troponin (cTn) is a highly specific and sensitive biomarker of myocardial injury, widely used to detect myocardial injury during or after myocardial infarction.

Cardiac troponin contains three subunits, namely cardiac troponin I (cTnI), cardiac troponin T (cTnT), and troponin C (TnC). Serum concentrations of cTnI and cTnT are highly correlated with the severity of myocardial injury. Troponin typically exists bound to actin filaments as a ternary complex (cTnITC). Upon myocardial injury, troponin is released from myofilaments and degraded into the bloodstream. In cells or circulation, cTnITC is proteolytically degraded into various forms. Studies have shown that blood troponin presence may be associated with an individual's physiological and pathological status.

An in-depth understanding of the association between different troponin forms and diseases, correct interpretation of cTn elevation causes, and differentiation between acute and non-acute myocardial injury are crucial for rapid risk stratification and accurate clinical diagnosis of chest pain patients.

### Contents of the Invention

In order to solve the above technical problems, in the first aspect, the present application provides a method for assessment of myocardial injury in a subject *in vitro*, comprising:
detecting the concentration of one or more myocardial injury markers in a sample from the subject;
obtaining a characteristic parameter for assessment of myocardial injury based on the concentration of the one or more myocardial injury markers;
comparing the characteristic parameter with a reference value of the characteristic parameter;
assessing myocardial injury of the subject based on the result of the comparison;
wherein the one or more myocardial injury markers comprise a large-size cardiac troponin ternary complex (large-size cTnITC, large-size ITC complex) and/or a total cardiac troponin ternary complex (total cTnITC, total ITC complex). The large-size cardiac troponin ternary complex may also be referred to as long cardiac troponin ternary complex (long cTnITC, long ITC complex).

In the second aspect, the present application provides a device for obtaining a characteristic parameter for assessment of myocardial injury in a subject, comprising:
a data receiving module, configured to receive concentration data of one or more myocardial injury markers obtained from a sample from a subject, wherein the one or more myocardial injury markers comprise a large-size cardiac troponin ternary complex and/or a total cardiac troponin ternary complex;
a data processing module, configured to process the data of the concentration of one or more myocardial injury markers received by the receiving module to obtain a characteristic parameter for assessment of myocardial injury; and
an output module, configured to output the characteristic parameter.

In the third aspect, the present application provides a sample analysis system, comprising:
a sample carrying portion, configured to carry a container containing a sample from a subject;
a sample dispensing portion, configured to pipette the sample from the subject from the sample carrying portion and discharge it into a reaction cup to be added with the sample;
a reagent carrying portion, configured to carry a detection reagent;
a reagent dispensing portion, configured to pipet the detection reagent from the reagent carrying portion and discharge it into the reaction cup to be added with the reagent;
a reaction portion, configured to place a reaction cup so as to incubate a test solution obtained by reacting the sample from the subject with the detection reagent in the reaction cup;
a detection portion, which has a signal detector and is configured to detect a signal of the test solution in the reaction cup to determine and output the concentration of one or more myocardial injury markers in the sample from the subject, wherein the one or more myocardial injury markers comprise a large-size cardiac troponin ternary complex and/or a total cardiac troponin ternary complex;
a data processing portion, which comprises a processor and a computer-readable storage medium on which the computer-readable instructions are stored, characterized in that the computer-readable instructions cause the processor to implement the following steps when executed by the processor:
   receiving and processing the concentration of the one or more myocardial injury markers to obtain a characteristic parameter for assessment of myocardial injury; and
   outputting the characteristic parameter.

In a fourth aspect, the present application provides use of a reagent for quantitatively detecting a large-size cardiac troponin ternary complex and/or a reagent for quantitatively detecting a total cardiac troponin ternary complex in a sample in the manufacture of a kit, wherein the kit is used for assessing myocardial injury in a subject.

In a fifth aspect, the present application provides a reagent for quantitative detection of a large-size cardiac troponin ternary complex and/or a reagent for quantitative detection a total cardiac troponin ternary complex in a sample, for use in assessing myocardial injury in a subject.

In various aspects of the present application, the characteristic parameter obtained based on the concentration of large-size cardiac troponin ternary complex and/or total cardiac troponin ternary complex can be used to assess myocardial injury faster and more accurately.

### Brief Description of the Drawings

The drawings described herein are used to provide a further understanding of the present invention and constitute a part of the present application. The exemplary embodiments of the present invention and descriptions thereof are used to explain the present invention and do not constitute an improper limitation of the present invention. In the drawings:
Figure 1 shows a schematic block diagram of a device for obtaining characteristic parameters for assessing myocardial injury in a subject provided in an example of the present application.
Figure 2 shows a schematic diagram of a sample analysis system provided in an example of the present application.
Figure 3 shows a signal-to-noise ratio analysis of large-size cTnITC detection kits, in which the signal-to-noise ratio data of each group of samples correspond to Kits **1** to **8** from left to right.
Figure 4 shows a signal-to-noise ratio analysis of total cTnITC detection kits, in which the signal-to-noise ratio data of each group of samples correspond to Kits **1** to **12** from left to right.
Figure 5 shows a validation of the specificity of large-size cTnITC detection kits using serum samples.
Figure 6 shows a validation of the specificity of total cTnITC detection kits using serum samples.
Figure 7 shows a linearity analysis of the large-size cTnITC detection Kit **3**.
Figure 8 shows a linearity analysis of the large-size cTnITC detection Kit **4**.
Figure 9 shows a linearity analysis of the total cTnITC detection Kit **3**.
Figure 10 shows a linearity analysis of the total cTnITC detection Kit **4**.
Figure 11 shows the enrollment and detection process for patients with early myocardial infarction.
Figure 12 shows the ratios of large-size cTnITC/total complex, total cTnITC/total complex, and cTnT/total complex in patients with different chest pain durations. The *P* values between the groups were compared using the Kruskal-Wallis test. **P*<0.05, ***P*<0.01, ****P*<0.001, *****P*<0.0001, ns: no significant difference.
Figure 13 shows the ratios of large-size cTnITC/total complex, total cTnITC/total complex, cTnT/total complex, large-size cTnITC/cTnT, and total cTnITC/cTnT in patients with Type 1 myocardial infarction and Type 2 myocardial infarction. The *P* values between Type 1 myocardial infarction and Type 2 myocardial infarction were compared using the Mann-Whitney U test. **P*<0.05, ***P*<0.01, ns: no significant difference.
Figure 14 shows the enrollment and detection process for patients with acute myocardial infarction (Type 1) in Example 3-1.
Figure 15 shows the enrollment and detection process for patients with chronic cardiac events (cardiomyopathy or chronic heart failure) in Example 3-1.
Figure 16 shows the enrollment and detection process for patients with chronic cardiac events (pneumonia) in Example 3-1.
Figure 17 shows the ratios of large-size cTnITC/total complex, total cTnITC/total complex, cTnT/total complex, large-size cTnITC/cTnT, and total cTnITC/cTnT in acute myocardial infarction (Type 1) and chronic cardiac event samples. The values were shown as median ± interquartile range. The *P* values between patients with acute myocardial infarction (Type 1), cardiomyopathy or chronic heart failure, and pneumonia were compared using the Kruskal-Wallis test. ***P*<0.01, ****P*<0.001, *****P*<0.0001, ns: no significant difference.
Figure 18 shows the enrollment and detection process for patients with invasive procedure.
Figure 19 shows the enrollment and detection process for patients with chronic cardiac events.
Figure 20 shows the ratios of large-size cTnITC/total complex, total cTnITC/total complex, cTnT/total complex, large-size cTnITC/cTnT, and total cTnITC/cTnT in patients with myocardial injury due to invasive procedure and patients with chronic cardiac events. The values were shown as median ± interquartile range. The *P* values between patients with chronic cardiac events, surgical patients, and interventional surgery patients were compared using the Kruskal-Wallis test. ***P*<0.01, ****P*<0.001, *****P*<0.0001, ns: no significant difference.
Figure 21 shows the enrollment and detection process for cardiac surgery patients.
Figure 22 shows the survival curves of cardiac surgery patients in different troponin fragment concentration groups.
Figure 23 shows the enrollment and detection process for patients with acute myocardial infarction (Type 1).
Figure 24 shows the survival curves of patients with acute myocardial infarction (Type 1) in different troponin fragment concentration groups.
Figure 25 shows the enrollment and detection process for patients with chronic cardiac events (cardiomyopathy or chronic heart failure).
Figure 26 shows the survival curves of patients with chronic cardiac events (cardiomyopathy or chronic heart failure) in different troponin fragment concentration groups.
Figure 27 shows the enrollment and detection process for patients with acute chest pain suspected of having coronary syndrome.

The technical solution in the embodiments will be described clearly and completely in conjunction with the accompanying drawings. Obviously, the described embodiments are merely part of the embodiments of the present invention, rather than all of them. The following description of the embodiments is for illustrative purpose only and in no way limits the present invention. Based on the embodiments provided, all other embodiments obtained by those skilled in the art without creative efforts shall fall within the scope of protection of the present invention.

As used herein, unless otherwise specified, scientific and technical terms shall have meanings that are commonly understood by those skilled in the art. In addition, the immunological laboratory procedures described herein are all routine procedures widely used in the relevant art. Meanwhile, for a better understanding of the embodiments of the present invention, definitions and explanations of relevant terms are provided below.

As used herein, the terms "include", "comprise" or any other variants thereof are intended to cover a non-exclusive inclusion, such that a method or device comprising a series of elements includes not only those explicitly recited, but also other elements not explicitly listed, or further includes elements inherent to the practice of such method or device. In the absence of further limitations, an element defined by the phrase "comprise a ..." does not preclude the presence of additional related elements in the method or device that includes the element.

As used herein, the term "at least one" means one or more, such as 2, 3, 4, 5 or 10, etc., under reasonable circumstances.

As used herein, the terms "first" and "second" are used merely to distinguish similar objects and do not denote a specific order for such objects. It is understood that "first" and "second" may be interchanged in terms of a specific order or sequence where permitted. It should be understood that the objects distinguished by "first" and "second" may be interchangeable where appropriate, such that the embodiments of the present application described herein can be practiced in an order other than those illustrated or described herein.

As used herein, the terms "individual" and "subject" preferably refer to a mammal. Mammals include, but are not limited to, domestic animals (e.g., cattle, sheep, cats, dogs, and horses), primates (e.g., humans and non-human primates such as monkeys), rabbits, and rodents (e.g., mice and rats), with a preference for humans. In some embodiments, the terms specifically refer to patients with specific clinical symptoms, such as patients with chest pain.

As used herein, the term "specific binding" refers to a non-random binding reaction between two molecules (i.e., a binding molecule and a target molecule), such as a reaction between an antibody and its cognate antigen. The binding affinity between two molecules can be characterized by a K_{D} value. The K_{D} value refers to a dissociation constant obtained from the ratio of kd (the dissociation rate of a specific binding molecule-target molecule interaction; also known as koff) to ka (the association rate of a specific binding molecule-target molecule interaction; also known as kon), or refers to a molar concentration (M) expression of kd/ka. The smaller the K_{D} value, the tighter the binding between the two molecules and the higher the binding affinity. In certain embodiments, an antibody capable of specifically binding to an antigen (or being specific for an antigen) means that the antibody binds to the antigen with a K_{D} of less than about 10⁻⁵ M, such as less than about 10⁻⁶ M, 10⁻⁷ M, 10⁻⁸ M, 10⁻⁹ M, or 10⁻¹⁰ M or less. Corresponding methods for analyzing antibody specificity are described, for example, in Harlow & Lane (1988) Antibodies: A Laboratory Manual, Cold Spring Harbor Laboratory Press, and Harlow & Lane (1999) Using Antibodies: A Laboratory Manual, Cold Spring Harbor Laboratory Press. Non-limiting examples of suitable assays include, for example, binding assays, blocking and competition assays using molecules with close structural and/or functional homology. These assays can be performed using methods such as fluorescence activated cell sorting (FACS) analysis, flow cytometry titration (FACS titration) analysis, surface plasmon resonance (SPR, as used for example), isothermal titration calorimetry (ITC), fluorescence titration or radiolabeled ligand binding assay. Additional methods include, for example, Western Blot, ELISA (including competitive ELISA), RIA, ECL and IRMA.

As used herein, the term "diagnosis" refers to a method by which it can be assessed and/or determined whether a patient suffers from a specific disease or condition.

As used herein, the term "prognosis" is typically determined by one or more markers or characteristic parameters. Such markers or characteristic parameters are indicative of the probability that a particular outcome or process will occur.

As used herein, the term "sample", "specimen" refers to a body fluid or tissue sample from a subject, such as whole blood, serum, plasma (including lithium heparin plasma, EDTA plasma), urine, saliva, biological tissue or cells, preferably whole blood, serum or plasma.

As used herein, the term "troponin" or "Tn" refers to a protein on actin that modulates calcium-mediated interactions between actin and myosin in myocytes. It is present in cardiac and skeletal muscle and is composed of three subunits: troponin T (TnT), troponin I (TnI) and troponin C (TnC). Among them, TnT is a tropomyosin-binding subunit that interacts with actin and tropomyosin; TnI is an inhibitory subunit that inhibits the ATPase activity of actomyosin; TnC is the only subunit that binds calcium and the binding can trigger contraction of skeletal or cardiac muscle. The term "cardiac troponin" or "cTn" refers to all troponin isoforms expressed in cardiac cells, preferably subendocardial cells. These isoforms have been well characterized in the art, for example, as described in Anderson 1995, Circulation Research, vol. 76, no. 4: 681-686 and Ferrieres 1998, Clinical Chemistry, 44: 487-493. The term "cardiac troponin" also includes variants of a particular cardiac troponin, such variants having at least the same basic biological and immunological properties as those of the particular cardiac troponin. In particular, if they are detected with the same specificity as mentioned herein, such variants share the same basic biological and immunological properties. It should be understood that the isoforms of troponin can be detected in combination (simultaneously or sequentially) or individually (i.e., without detecting other isoforms at all).

As used herein, the term "cardiac troponin T", "cTnT" refers to the cardiac troponin T subunit, the amino acid sequence of which is disclosed in the UniProt database, number P45379.

As used herein, the term "cardiac troponin I" and "cTnI" refer to the cardiac troponin I subunit, the amino acid sequence of which is disclosed in the UniProt database, number P19429.

As used herein, the term "troponin C" and "TnC" refer to the troponin C subunit, the amino acid sequence of which is disclosed in the UniProt database, number P63316.

As used herein, the term "cardiac troponin binary complex" comprises a binary complex composed of full-length troponin C or any fragments thereof and full-length cardiac troponin I or any fragments thereof.

As used herein, the term "large-size cardiac troponin ternary complex" or "large-size cTnITC (large-size ITC complex)" is used interchangeably herein and is intended to include a complex formed by any of full-length TnC or fragments thereof, full-length cTnI or fragments thereof, one or more fragments of amino acid residues 223-287 of cTnT, and one or more fragments of amino acid residues 1-222 of cTnT.

As used herein, the term "total cardiac troponin ternary complex" or "total cTnITC (total ITC complex)" is used interchangeably herein and is intended to include a complex formed by all of full-length TnC or fragments thereof, full-length cTnI or fragments thereof, one or more fragments of amino acid residues 223-287 of cTnT, and optionally one or more fragments of amino acid residues 1-222 of cTnT.

As used herein, the term "total cardiac troponin complex" or "total complex" comprises a total cardiac troponin ternary complex, and a binary complex composed of full-length troponin C or any fragments thereof and full-length cardiac troponin I or any fragments thereof.

### Differential evaluation and diagnostic methods

Cardiac troponin is a highly specific and sensitive biomarker of myocardial injury, and is widely used for the detection of myocardial injury following or during myocardial infarction. However, current troponin assays are unable to distinguish different types of myocardial injury, and there is still a lack of effective means in the art to assess myocardial injury, distinguish different types of myocardial injury, and assist clinicians in rapidly and accurately diagnosing individual pathological conditions, thereby guiding prognosis.

The first aspect of the present application provides a method for assessment of myocardial injury in a subject *in vitro*, comprising:
detecting the concentration of one or more myocardial injury markers in a sample from the subject;
obtaining a characteristic parameter for assessment of myocardial injury based on the concentration of the one or more myocardial injury markers;
comparing the characteristic parameter with the reference value of the characteristic parameter;
assessing myocardial injury of the subject based on the result of the comparison;
wherein, the one or more myocardial injury markers comprise a large-size cardiac troponin ternary complex and/or a total cardiac troponin ternary complex.

The term "myocardial injury" is known in the art and refers to a pathological change in myocardial cells, which cause abnormal cardiac manifestations. Patients may experience symptoms such as chest pain, palpitations, dyspnea, and palpitations. cTn level above the 99^{th}URL is indicative of myocardial injury. If accompanied by a rising or falling in cTn levels, the condition is considered as acute myocardial injury; if cTn shows a persistent elevation with an increase of less than <20%, it may indicate chronic myocardial injury. Myocardial injury can occur in a variety of cardiac and non-cardiac diseases.

In the context, assessment of myocardial injury comprises diagnosing (e.g., diagnosing the cause or type), grading (e.g., grading the severity or stage of myocardial injury), or monitoring (e.g., prognosis) myocardial injury.

A differential evaluation or diagnosis refers to a method of diagnosing a specific disease and/or specific condition underlying the symptoms in a particular individual, based on a comparison between observable characteristic properties in the individual and characteristic properties of potential disease. Depending on the range of diseases and conditions that must be taken into account in the differential evaluation or diagnosis, the type and number of laboratory analyses that a physician must perform can be very extensive. For example, in the case of chest pain, the physician may select the following laboratory analyses, including physical examination, echocardiography, electrocardiography, high-sensitivity troponin I detection, high-sensitivity troponin T detection, and coronary angiography. However, the physician must integrate the information obtained from a set of tests to arrive at a clinical diagnosis that most closely represents the range of symptoms and/or diagnostic test results from the subject.

The present application describes markers and characteristic parameters that can be used for or to assist in the assessment of myocardial injury. By detecting the concentration of one or more myocardial injury markers in a sample and further obtaining a characteristic parameter for assessing myocardial injury, the value of the characteristic parameter is compared with that in an individual suffering from a specific condition or at risk of a specific condition, or with that in an individual known not suffering from the condition. Such comparison result is linked to an assessment or diagnosis to generate a response.

### Marker and characteristic parameter for myocardial injury

In the present application, the markers for assessing myocardial injury include at least a large-size cardiac troponin ternary complex and/or a total cardiac troponin ternary complex. Based on the diagnostic scenario of application, the markers may further include one or more of cTnI, cTnT, TnC, cardiac troponin binary complex and total cardiac troponin complex.

The concentrations of markers are particularly preferably concentrations, which can be determined by immunoassay. Examples of such immunoassays are enzyme-linked immunosorbent assay (ELISA), enzyme immunoassay (EIA), radioimmunoassay (RIA) or immunoassay based on luminescence, fluorescence, chemiluminescence or electrochemiluminescence. In some embodiments, quantitative detection of each marker is particularly preferably performed by ELISA method, for example, using a commercially available ELISA kit for detection.

### Comparison with reference value

"Comparison" refers to comparing a characteristic parameter obtained from a subject with a reference value thereof. It should be understood that the comparison used herein generally refers to the comparison of the value of the corresponding characteristic parameter. The comparison can be performed manually or computer-assisted. Therefore, the comparison can be implemented by a computer device (e.g., the device or analysis system disclosed herein). The characteristic parameter and the reference value can be compared with each other, for example, the comparison can be automatically implemented by a computer program that executes an algorithm for comparison. The computer program implementing the evaluation will provide the desired evaluation in a suitable output form. For computer-assisted comparison, the characteristic parameter can be compared with corresponding suitable reference value, which is stored in a database by a computer program. The computer program can further evaluate the comparison result, that is, automatically provide the desired evaluation in a suitable output form.

The reference value can be used to define and establish a threshold value. The threshold value is preferably used to assess/diagnose the subject as described herein. The diagnosis or assessment can be provided by the data processing module of the device or system described herein based on the calculated "value" and the reference value or threshold value. For example, the data processing module of the system can provide indicators in the form of text, symbols or numerical values, which indicate a diagnosis or assessment. The reference value applicable to the subject may vary, depending on the markers selected and determination method thereof. Suitable reference value can be determined from the reference sample to be analyzed together with the test sample (i.e., simultaneously or sequentially).

In principle, the reference level of a patient group suffering from a specific disease or at least one abnormality or not suffering from the above-mentioned disease or abnormality can be calculated based on the mean or median value of a specific marker by applying standard statistical methods. In some embodiments, Graphpad Prism, SPSS and Excel software are used for statistical analysis. Statistical differences between different subgroups are analyzed by Mann-Whitney U test (for comparison between two groups) and Kruskal-Wallis test (for comparison among multiple groups) to determine statistical significance. *P* value <0.05 is considered significant for the variable.

Specifically, the accuracy of the test method (e.g., for diagnostic events) is preferably described by the ROC (receiver operator characteristic curve) (see Zweig 1993, Clin. Chem. 39: 561-577). The ROC curve is a curve plotted according to a series of different binary classification methods (cutoff thresholds), with the true positive rate (TPR) as the ordinate and the false positive rate (FPR) as the abscissa. ROC_AUC (area under the curve) represents the area enclosed by the ROC curve and the horizontal axis. In some embodiments, SPSS software is used to generate the ROC curve to obtain the area under the curve, AUC. Different TPRs and FPRs can be obtained on the ROC curve by adjusting the threshold. The higher the threshold, the smaller the FPR and the larger the TPR. Conversely, the larger the FPR, the smaller the TPR. Youden's J statistic is a statistic for evaluating the performance of a classifier, which is equal to TPR - FPR, that is, sensitivity. The larger its value, the better the performance of the classifier. On the ROC curve, the point where Youden's J statistic takes the maximum value is called Youden index point, and the threshold value corresponding to it is called Youden threshold. In some embodiments, the Youden threshold is used as the optimal threshold point (CUTOFF value).

Depending on the target confidence interval, the threshold value can be derived from the ROC curve, which enables diagnosis or prediction of a given event by achieving an appropriate balance between sensitivity and specificity, respectively. Therefore, the reference value used in the present invention may preferably be a threshold value or CUTOFF value, and may preferably be determined by constructing an ROC curve for the group and deriving a threshold therefrom as described above. Depending on the desired sensitivity and specificity of the diagnostic method, the ROC graph allows for derivation of a suitable cutoff threshold.

The effectiveness of the diagnostic method and the predictive value of marker parameters for the primary endpoint event are described by the receiver operator characteristic (ROC) curve, which is plotted according to the sensitivity and specificity obtained by continuously adjusting the decision threshold within the observed data range, and the Y axis is sensitivity, the X axis is 1-specificity, and the closer the curve is to the upper left corner, the higher the diagnostic accuracy. The diagnostic performance or accuracy is indicated by the area under the receiver operating curve (AUC). The significance test *P* value <0.05 is considered indicative of a statistically significant variable. Since the ROC curve is composed of multiple critical values representing the sensitivity and specificity of each, the best diagnostic limit value of a diagnostic method can be selected with the help of the ROC curve. The closer the ROC curve is to the upper left corner, the higher the test sensitivity, the lower the false positive rate, and the better the performance of the diagnostic method. It can be seen that the point on the ROC curve closest to the upper left corner corresponds to the maximum sum of sensitivity and specificity, and this point or the value corresponding to its adjacent point is often used as a diagnostic reference value (also called a diagnostic threshold or judgment threshold or preset condition or preset range).

In one embodiment, the term "reference value" can be a predetermined value. As will be appreciated by those skilled in the art, the reference value is predetermined and set to meet conventional criteria with respect to, for example, specificity and/or sensitivity. These requirements may vary, for example, across regulatory authorities. For example, when determining sensitivity or specificity, they must be set to defined levels, such as 80%, 90%, 95% or 98%, respectively. These requirements can also be limited in terms of positive or negative predictive values. Nevertheless, based on the teachings of the present invention, it is easy to establish reference values that satisfy these criteria. The reference value is derived from individuals suffering from the disease or abnormality as mentioned herein, or suffering from severe or mild forms of the condition.

In some embodiments, the reference value has been predetermined in a reference sample from subjects with the disease of interest. In some embodiments, the reference value can be set, for example, to any percentage between 25th and 75th percentage of the overall distribution of the characteristic parameter in the target disease population. In other embodiments, the reference value can be set, for example, to the median, tertile or quartile calculated from the overall distribution of the reference sample from the target disease population. In some embodiments, the reference value can be set to the median determined from the overall distribution of the characteristic parameter in the disease under study. In some embodiments, continuous variables are presented as medians (25%-75% quartiles); categorical variables are presented as counts (percentages).

In the present application, the characteristic parameter for assessing myocardial injury can be selected from the concentration of large-size cardiac troponin ternary complex, the concentration of total cardiac troponin ternary complex, and the concentration of large-size cardiac troponin ternary complex and/or the concentration of total cardiac troponin ternary complex in combination with the concentration of other myocardial injury markers (e.g., cTnI, cTnT or total cardiac troponin complex).

In some embodiments, based on the concentration of the detected marker, the characteristic parameter for assessing myocardial injury is obtained, comprising:
determining the concentration of the large-size cardiac troponin ternary complex or the total cardiac troponin ternary complex as the characteristic parameter for assessing myocardial injury; or
obtaining the characteristic parameter for assessing myocardial injury based on the concentration of the large-size cardiac troponin ternary complex and the concentration of the total cardiac troponin ternary complex;
for example, determining the characteristic parameter for assessing myocardial injury based on the concentration change (including difference and ratio) of the large-size cardiac troponin ternary complex or the total cardiac troponin ternary complex before and after the event.

In some embodiments, the one or more myocardial injury markers preferably further comprise at least one of cTnI, cTnT and the total cardiac troponin complex.

In some embodiments, the step of obtaining the characteristic parameter for assessing myocardial injury based on the concentrations of the one or more myocardial injury markers comprises: inputting the concentrations of the multiple myocardial injury markers into a preset function model to obtain the output of the preset function model as a characteristic parameter for assessing myocardial injury. The input preset function model can be, for example, a linear function model, a nonlinear function model or a machine learning model. In some embodiments, a binary logistic regression model is used to construct a combination of variables for establishing a combination parameter. In some embodiments, a binary logistic regression analysis is used to analyze the correlation between marker parameter and the occurrence of primary endpoint event. *P* value <0.05 is considered statistically significant for the variable. Cox regression analysis is used to determine the association between marker parameters (or their combinations) and the outcome of study subjects, and the hazard ratio (HR) is calculated using the Cox proportional hazard model to evaluate the risk stratification of different risk groups defined by marker parameter values, and the fold increase in the risk of endpoint event relative to the baseline group. *P* value <0.05 is considered statically significant for the variable.

In one example, the concentrations of multiple myocardial injury markers can be directly input as variables into a preset function model, for example, the concentration of large-size cardiac troponin ternary complex or total cardiac troponin ternary complex and the concentration of at least one of cTnI, cTnT and total cardiac troponin complex are input as variables into a preset function model to obtain the output of the preset function model as a characteristic parameter for assessing myocardial injury.

In another example, the ratio between the concentrations of multiple myocardial injury markers can also be input as a variable into a preset function model, for example, the ratio of the concentration of large-size cardiac troponin ternary complex and/or total cardiac troponin ternary complex to the concentration of at least one of cTnI, cTnT and total cardiac troponin complex is input as a variable into a preset function model to obtain the output of the preset function model as a characteristic parameter for assessment of myocardial injury.

As used herein, when describing the ratio of the concentration of a certain myocardial injury marker to that of another myocardial injury marker, it includes both the ratio of the concentration of the former myocardial injury marker to the concentration of the latter myocardial injury marker, and the ratio of the concentration of the latter myocardial injury marker to the concentration of the former myocardial injury marker.

In some embodiments, the characteristic parameter is obtained by the ratio between the concentrations of multiple myocardial injury markers, that is, the step of obtaining the characteristic parameter for assessing myocardial injury based on the concentration of one or more myocardial injury markers comprises: determining one of the following ratio parameters as the characteristic parameter for assessment of myocardial injury, or obtaining the characteristic parameter for assessment of myocardial injury based on at least two of the following ratio parameters, or obtaining the characteristic parameter for assessment of myocardial injury based on at least one of the following ratio parameters and the ratio of the concentration of cTnT to the concentration of total cardiac troponin complex;
the ratio parameters comprise: the ratio of the concentration of large-size cardiac troponin ternary complex to the concentration of cTnI, the ratio of the concentration of large-size cardiac troponin ternary complex to the concentration of cTnT, the ratio of the concentration of large-size cardiac troponin ternary complex to the concentration of total cardiac troponin complex, the ratio of the concentration of total cardiac troponin ternary complex to the concentration of cTnI, the ratio of the concentration of total cardiac troponin ternary complex to the concentration of cTnT, the ratio of the concentration of total cardiac troponin ternary complex to the concentration of total cardiac troponin complex, and the ratio of the concentration of large-size cardiac troponin ternary complex to the concentration of total cardiac troponin ternary complex.

In some embodiments, the ratio parameter is the ratio of the concentration of large-size cardiac troponin ternary complex to the concentration of cTnI, cTnT or total cardiac troponin complex; optionally, the ratio parameter further comprises the ratio of the concentration of total cardiac troponin ternary complex to the concentration of cTnI, cTnT or total cardiac troponin complex, and/or the ratio of the concentration of cTnT to the concentration of cTnI or total cardiac troponin complex.

For example, the ratio of the concentration of large-size cardiac troponin ternary complex or total cardiac troponin ternary complex to the concentration of one of cTnI, cTnT and total cardiac troponin complex can be determined as a characteristic parameter for assessing myocardial injury.

Based on the above information, the present application can be used for the assessment of myocardial injury.

Large-size cardiac troponin ternary complex or total cardiac troponin ternary complex can be used for the diagnosis of myocardial injury. Specifically, it can be used in the following clinical scenarios.

### (1) Diagnosis of early acute myocardial infarction disease state (staging of myocardial infarction patients)

Regarding the existing forms of troponin complex components in patients with acute myocardial infarction, it has been reported in the literature that the concentration of ternary complex is found to be higher in patients with early onset of myocardial infarction symptoms, and the proportion of total troponin I is larger. However, there is still no relevant research that uses the analysis of large-size cardiac troponin ternary complex or total cardiac troponin ternary complex to assist in the diagnosis of myocardial infarction. Large-size cardiac troponin ternary complex or total cardiac troponin ternary complex can provide information for the judgment of acute phase of myocardial infarction alone or in combination with other clinical myocardial injury indicators, rapidly identify high-risk patients, and help customize clinical decisions.

As used herein, the marker for diagnosing early acute myocardial infarction comprises large-size cardiac troponin ternary complex and/or total cardiac troponin ternary complex, as well as one or more of cTnI, cTnT, TnC, cardiac troponin binary complex and total cardiac troponin complex.

In some embodiments, the marker for diagnosing early acute myocardial infarction comprises large-size cardiac troponin ternary complex, or a combination thereof with cTnI, cTnT or total cardiac troponin complex.

In some embodiments, the marker for diagnosing early acute myocardial infarction comprises large-size cardiac troponin ternary complex and/or total cardiac troponin ternary complex, and one or more of cTnI, cTnT, TnC, cardiac troponin binary complex and total cardiac troponin complex, in particular large-size cardiac troponin ternary complex and/or total cardiac troponin ternary complex, and one or two of cTnT and total cardiac troponin complex.

In some embodiments, the step of obtaining a characteristic parameter for assessing myocardial injury based on the concentration of the one or more myocardial injury markers comprises:
determining the concentration of the large-size cardiac troponin ternary complex as a characteristic parameter for assessing myocardial injury; or
obtaining a characteristic parameter for assessing myocardial injury based on the concentration of the large-size cardiac troponin ternary complex and the concentration of at least one of cTnI, cTnT, total cardiac troponin ternary complex and total cardiac troponin complex, preferably calculating the characteristic parameter for assessing myocardial injury based on the concentration of the total cardiac troponin ternary complex and the concentration of cTnT;
obtaining a characteristic parameter for assessing myocardial injury based on the ratio of the concentration of the large-size cardiac troponin ternary complex or the concentration of the total cardiac troponin ternary complex to the concentration of at least one of cTnI, cTnT and total cardiac troponin complex, and optionally the ratio of the concentration of cTnT to the concentration of cTnI or total cardiac troponin complex, for example, determining the ratio of the concentration of the large-size cardiac troponin ternary complex or the concentration of the total cardiac troponin ternary complex to the concentration of cTnT as a characteristic parameter for assessing myocardial injury.

In some embodiments, a characteristic parameter based on a combination of multiple myocardial injury markers is constructed based on a logistic regression algorithm. One example is a combination of the concentration of large-size cardiac troponin ternary complex and the concentration of total cardiac troponin ternary complex, the concentration of cTnT or the concentration of total cardiac troponin complex, or a combination of the concentration of large-size cardiac troponin ternary complex, the concentration of total cardiac troponin ternary complex, the concentration of cTnT and the concentration of total cardiac troponin complex; another example is a combination of the ratio of the concentration of large-size cardiac troponin ternary complex to the concentration of total cardiac troponin complex, the ratio of the concentration of total cardiac troponin ternary complex to the concentration of total cardiac troponin complex, and the ratio of the concentration of cTnT to the concentration of total cardiac troponin complex, or a combination of the ratio of the concentration of large-size cardiac troponin ternary complex to the concentration of cTnT, and the ratio of the concentration of total cardiac troponin ternary complex to the concentration of cTnT.

In some embodiments, a ROC curve is plotted to establish a prediction model for the characteristic parameter, and the optimal threshold (cutoff) is calculated as a reference value.

In some embodiments, the step of assessing the myocardial injury of the subject based on the aforementioned comparison result comprises:
staging the subject for myocardial infarction based on the comparison result.

In some embodiments, when the characteristic parameter is higher than the reference value of the characteristic parameter, the subject is judged to be in the early stage of acute myocardial infarction, such as myocardial infarction within 72 hours, 30 hours to 72 hours, 10 hours to 30 hours, 10 hours to 72 hours, or within 10 hours of the onset of chest pain.

In one embodiment, it also comprises hospitalizing the subject who is judged to be in the early stage of acute myocardial infarction.

The term "higher than" reference value means that the level of such parameter in the sample is higher than the reference value or the level of such parameter in the reference sample. For example, a higher amount of a marker or an increased level of a marker can be detected in a sample from an individual with a given disease compared to the same sample from an individual without the disease.

### (2) Differentiation between acute myocardial infarction (Type 1) and chronic cardiac events

Currently, troponin I and troponin T are specific markers of myocardial injury. When myocardial injury occurs, the marker levels rise, making it impossible to differentiate between acute and chronic injuries. In order to identify acute myocardial injury, continuous monitoring of changes in troponin concentration is usually required, which is difficult to judge through a single test and may lead to delayed diagnosis and delayed treatment.

As used herein, "acute cardiac event" refers to an acute condition, disease or dysfunction of heart, especially acute heart failure, such as myocardial infarction (MI) or arrhythmia. Depending on the degree of MI, it can be followed by LVD and CHF. "Chronic cardiac event" is a weakening of cardiac function, such as due to ischemia of heart, coronary artery disease or previous, especially small, myocardial infarction (which may be followed by progressive LVD). It can also be a weakening caused by inflammatory disease, heart valve defect (e.g., mitral valve defect), dilated cardiomyopathy, hypertrophic cardiomyopathy, heart rhythm defect (arrhythmia) and chronic obstructive pulmonary disease. Therefore, it is clear that chronic cardiac events can also include patients who have suffered from acute coronary syndromes such as MI but are not currently experiencing an acute cardiac event.

It is important to differentiate between acute cardiac events and chronic cardiac events because acute cardiac events and chronic cardiac events may require distinctly different treatment plans. For example, for patients who present with acute myocardial infarction, early treatment for reperfusion may be crucial. However, reperfusion treatment for a patient with chronic heart failure may be harmless or only slightly harmful to this patient at best.

As used herein, the markers used to distinguish between acute myocardial infarction and chronic cardiac events include large-size cardiac troponin ternary complex and/or total cardiac troponin ternary complex, and one or more of cTnI, cTnT, TnC, cardiac troponin binary complex, and total cardiac troponin complex.

In some embodiments, the markers used to distinguish between acute myocardial infarction and chronic cardiac events include large-size cardiac troponin ternary complex or total cardiac troponin ternary complex, or a combination of large-size cardiac troponin ternary complex and cTnT.

In some embodiments, the markers for distinguishing between acute myocardial infarction and chronic cardiac events include a combination of large-size cardiac troponin ternary complex and one or more of total cardiac troponin ternary complex, cTnT, and total cardiac troponin complex.

In some embodiments, the step of obtaining the characteristic parameter for assessing myocardial injury based on the concentration of the one or more myocardial injury markers comprises:
determining the concentration of the large-size cardiac troponin ternary complex or the concentration of the total cardiac troponin ternary complex as a characteristic parameter for assessing myocardial injury; or
calculating the characteristic parameter for assessing myocardial injury based on the concentration of the large-size cardiac troponin ternary complex and the concentration of the total cardiac troponin ternary complex; or
obtaining the characteristic parameter for assessing myocardial injury based on the concentration of the large-size cardiac troponin ternary complex or the concentration of the total cardiac troponin ternary complex and the concentration of cTnT or total cardiac troponin complex.

In one embodiment, the characteristic parameter for assessing myocardial injury is obtained based on the ratio of the concentration of the large-size cardiac troponin ternary complex or the concentration of the total cardiac troponin ternary complex to the concentration of at least one of cTnI, cTnT and total cardiac troponin complex, and optionally the ratio of the concentration of cTnT to the concentration of cTnI or total cardiac troponin complex.

In one example, the concentration of the large-size cardiac troponin ternary complex or the concentration of the total cardiac troponin ternary complex and the concentration of cTnT or total cardiac troponin complex can be directly used as variables to calculate the characteristic parameter for assessing myocardial injury.

In another example, the ratio of the concentration of the large-size cardiac troponin ternary complex and/or the concentration of the total cardiac troponin ternary complex to the concentration of cTnT or total cardiac troponin complex can be used as a variable to calculate the characteristic parameter for assessing myocardial injury.

In some embodiments, based on a logistic regression algorithm, a characteristic parameter based on a combination of multiple myocardial injury markers is constructed. One example is a combination of the concentration of large-size cardiac troponin ternary complex and the concentration of total cardiac troponin ternary complex, the concentration of cTnT or the concentration of total cardiac troponin complex, or a combination of the concentration of large-size cardiac troponin ternary complex, the concentration of total cardiac troponin ternary complex, the concentration of cTnT and the concentration of total cardiac troponin complex; another example is a combination of the ratio of the concentration of large-size cardiac troponin ternary complex to the concentration of total cardiac troponin complex, the ratio of the concentration of total cardiac troponin ternary complex to the concentration of total cardiac troponin complex, and the ratio of the concentration of cTnT to the concentration of total cardiac troponin complex, or a combination of the ratio of the concentration of large-size cardiac troponin ternary complex to the concentration of cTnT, and the ratio of the concentration of total cardiac troponin ternary complex to the concentration of cTnT.

In some embodiments, a ROC curve is plotted to establish a prediction model for the characteristic parameter, and the optimal threshold (cutoff) is calculated as the reference value.

In some embodiments, the aforementioned comparison is performed, and the step of assessing the myocardial injury of the subject based on the result of the comparison comprises:
determining whether the subject has a type I myocardial infarction or a chronic cardiac event based on the result of the comparison.

In some embodiments, when the characteristic parameter is higher than the reference value of the characteristic parameter, the subject is judged to have a type I myocardial infarction.

In some embodiments, it further comprises hospitalizing the subject who has been judged to have a type I myocardial infarction.

Using large-size cardiac troponin ternary complex and/or total cardiac troponin ternary complex enables more rapid differentiation of the type of myocardial injury through a single time point test, accelerated treatment of patients with acute injury, rapid exclusion of patients with chronic injury, and accelerated clinical turnover.

### (3) Excluding patients without occurrence of myocardial injury

Current research focuses on the use of continuous monitoring of troponin I and troponin T to exclude patients without occurrence of myocardial injury. For patients with chest pain duration of less than 3 hours, continuous monitoring of troponin concentration changes is required even if the troponin level is very low. The use of large-size cardiac troponin ternary complex and/or total cardiac troponin ternary complex enables the optimization of the diagnostic process by excluding patients without occurrence of myocardial injury through a single-point test, such as patients with chest pain onset within 24 hours, such as within 12 hours.

As used herein, the markers used to exclude chest pain subjects from myocardial injury include large-size cardiac troponin ternary complex and/or total cardiac troponin ternary complex, as well as one or more of cTnI, cTnT, TnC, cardiac troponin binary complex and total cardiac troponin complex; preferably large-size cardiac troponin ternary complex.

Based on the above method, the characteristic parameter for differentiation is obtained, and based on the results of the above comparison, patients without occurrence of myocardial injury (e.g., myocardial infarction, especially NSTEMI) are excluded.

In some embodiments, when the characteristic parameter is lower than the reference value of the characteristic parameter, the chest pain subject is excluded from occurring myocardial injury event.

In some embodiments, it further comprises notifying the patient who has been excluded from myocardial injury to leave an emergency room.

In some embodiments, the step of obtaining the characteristic parameter for assessing myocardial injury based on the concentration of the one or more myocardial injury markers comprises:
determining the concentration of the large-size cardiac troponin ternary complex or the concentration of the total cardiac troponin ternary complex as a characteristic parameter for assessing myocardial injury; or
obtaining the characteristic parameter for assessing myocardial injury based on the concentration of the large-size cardiac troponin ternary complex or the concentration of the total cardiac troponin ternary complex and the concentration of the total cardiac troponin complex.

In some embodiments, a ROC curve is plotted to establish a prediction model for the characteristic parameter, and the optimal threshold (cutoff) is calculated as the reference value.

### (4) Distinction between type I and type II myocardial infarction

The distinction between type I and type II myocardial infarction is of great significance. Early distinction between type I and type II myocardial infarction can provide information for subsequent treatment decisions. At present, the distinction between Type 1 and Type 2 myocardial infarction is mainly achieved through imaging examinations, and there is no marker that can effectively distinguish between type I and type II myocardial infarction. Large-size cardiac troponin ternary complex and/or total cardiac troponin ternary complex can rapidly distinguish between type I and type II myocardial infarction, support customization of clinical decisions, and reduce unnecessary medical examinations.

Herein, the markers used to distinguish between type I and type II myocardial infarction comprise large-size cardiac troponin ternary complex and/or total cardiac troponin ternary complex, as well as one or more of cTnI, cTnT, TnC, cardiac troponin binary complex and total cardiac troponin complex.

Based on the above method, a characteristic parameter for the distinction is obtained, and based on the results of the above comparison, the myocardial injury of the subject is evaluated to determine whether the subject has type I myocardial infarction or type II myocardial infarction.

In some embodiments, when the characteristic parameter is higher than the reference value of the characteristic parameter, the subject is judged to have type I myocardial infarction.

In some embodiments, thrombolytic therapy is given to the subject judged to have type I myocardial infarction, and thrombolytic therapy is not given to the subject judged to have type II myocardial infarction, but oxygen supplementation is given to the subject.

### (5) Distinction between myocardial injury due to invasive procedure and chronic cardiac events

Currently, troponin I and troponin T are specific markers of myocardial injury. When myocardial injury occurs, the marker levels rise, but it is impossible to distinguish different types of injury. In order to identify myocardial injury due to invasive procedure, it is usually necessary to continuously monitor the changes in troponin concentration. The use of large-size cardiac troponin ternary complex and/or total cardiac troponin ternary complex can more rapidly distinguish different types of injury through a single time point test.

Herein, the markers used to distinguish myocardial injury due to invasive procedures from chronic cardiac events include large-size cardiac troponin ternary complex and/or total cardiac troponin ternary complex, as well as one or more of cTnI, cTnT, TnC, cardiac troponin binary complex and total cardiac troponin complex.

In some embodiments, the step of obtaining the characteristic parameter for assessing myocardial injury based on the concentration of the one or more myocardial injury markers comprises:
determining the concentration of the large-size cardiac troponin ternary complex or the concentration of the total cardiac troponin ternary complex as a characteristic parameter for assessing myocardial injury.

In some embodiments, the characteristic parameter for assessing myocardial injury is obtained based on the ratio of the concentration of the large-size cardiac troponin ternary complex or the concentration of the total cardiac troponin ternary complex to the concentration of at least one of cTnI, cTnT and total cardiac troponin complex, and optionally the ratio of the concentration of cTnT to the concentration of cTnI or total cardiac troponin complex.

In some embodiments, the characteristic parameter based on a combination of multiple myocardial injury markers is constructed based on a logistic regression algorithm. One example is a combination of the concentration of large-size cardiac troponin ternary complex and the concentration of total cardiac troponin ternary complex, the concentration of cTnT or the concentration of total cardiac troponin complex, or a combination of the concentration of large-size cardiac troponin ternary complex, the concentration of total cardiac troponin ternary complex, the concentration of cTnT and the concentration of total cardiac troponin complex; another example is a combination of the ratio of the concentration of large-size cardiac troponin ternary complex to the concentration of total cardiac troponin complex, the ratio of the concentration of total cardiac troponin ternary complex to the concentration of total cardiac troponin complex, and the ratio of the concentration of cTnT to the concentration of total cardiac troponin complex, or a combination of the ratio of the concentration of large-size cardiac troponin ternary complex to the concentration of cTnT, and the ratio of the concentration of total cardiac troponin ternary complex to the concentration of cTnT.

In some embodiments, a ROC curve is plotted to establish a prediction model for the characteristic parameter, and the optimal threshold (cutoff) is calculated as the reference value.

In some embodiments, the comparison described above is performed, and the myocardial injury of the subject is assessed based on the result of the comparison to determine whether the subject has a myocardial injury due to invasive procedure or a chronic cardiac event.

In some embodiments, when the characteristic parameter is higher than the reference value of the characteristic parameter, the subject is judged to have myocardial injury due to invasive procedure.

In some embodiments, for the subject who has been judged to have myocardial injury due to invasive surgery, the hospitalization time is extended, or cardiac monitoring is strengthened.

In some embodiments, the method can accurately distinguish myocardial injury due to invasive procedure from chronic heart-related diseases (e.g., cardiomyopathy, chronic heart failure, structural heart disease, infiltrative disease, stable coronary heart disease, and persistent arrhythmia). In other embodiments, the method can accurately distinguish myocardial injury due to invasive procedures from non-heart-related diseases (e.g., pneumonia, renal insufficiency).

In addition, the large-size cardiac troponin ternary complex or total cardiac troponin ternary complex is also used for the prognosis of myocardial injury. Specifically, it can be used in the following clinical scenarios.

### (6) Evaluating the prognosis of acute myocardial injury

Current research focuses on the use of troponin I and troponin T to evaluate the prognosis of patients with myocardial infarction. The use of large-size cardiac troponin ternary complex and/or total cardiac troponin ternary complex is expected to improve the accuracy of predicting future cardiac events or deaths related to myocardial injury in patients with acute myocardial injury, such as the prognosis of myocardial injury in patients undergoing cardiac surgery or the prognosis of myocardial injury in patients with myocardial infarction.

Herein, the markers used to evaluate the prognosis risk of patients with myocardial infarction include large-size cardiac troponin ternary complex and/or total cardiac troponin ternary complex, as well as one or more of cTnI, cTnT, TnC, cardiac troponin binary complex and total cardiac troponin complex.

Based on the above method, the characteristic parameter for distinction is obtained, and based on the result of the above comparison, the myocardial injury of the subject is assessed to determine the prognosis of the subject with myocardial infarction, such as the prognosis within one year, within half a year or within three months.

In some embodiments, when the characteristic parameter is higher than the reference value of the characteristic parameter, the prognosis is judged to be poor.

In some embodiments, the step of obtaining the characteristic parameter for assessing myocardial injury based on the concentration of the one or more myocardial injury markers comprises:
determining the concentration of the large-size cardiac troponin ternary complex or the total cardiac troponin ternary complex as the characteristic parameter for assessing myocardial injury.

In some embodiments, the change in the concentration of the large-size cardiac troponin ternary complex or the total cardiac troponin ternary complex in the plasma of the subject before and after cardiac surgery is determined as the characteristic parameter for assessing the prognostic risk of receiving surgery.

### Assessment of prognosis of myocardial injury in patients undergoing surgery (e.g., coronary artery bypass grafting (CABG) or heart valve replacement)

Based on the concentrations of the large-size cardiac troponin ternary complex or total cardiac troponin ternary complex before and after surgery or change thereof (e.g., the difference or ratio of the concentration of the large-size cardiac troponin ternary complex or total cardiac troponin ternary complex between the postoperative and preoperative period), a characteristic parameter for assessing myocardial injury is determined. In some embodiments, based on a logistic regression algorithm, a characteristic parameter based on a combination of multiple myocardial injury markers is constructed. One example is a combination of the concentration of large-size cardiac troponin ternary complex and the concentration of cTnT.

In some embodiments, a ROC curve is plotted to establish a prediction model for the characteristic parameter, and the optimal threshold (cutoff) is calculated as the reference value.

In some embodiments, a binary logistic regression analysis is used to assess the risk of a patient's composite endpoint event (selected from major clinical events including composite evens of all-cause mortality, myocardial infarction, and unplanned coronary revascularization, and minor clinical events including any combination of cardiovascular death, various components of major clinical events, stroke, heart failure hospitalization or emergency observation for 24 hours or more, cardiac arrest or malignant arrhythmia, and other cardiovascular disease hospitalization events).

In some embodiments, a COX regression is used to assess the risk of a patient's composite endpoint event (selected from major clinical events including composite events of all-cause mortality, myocardial infarction, and unplanned coronary revascularization, and minor clinical events including any combination of cardiovascular death, components of major clinical events, stroke, hospitalization for heart failure or emergency observation for 24 hours or more, cardiac arrest or malignant arrhythmia, and other cardiovascular disease hospitalization events).

### Assessment of prognosis of myocardial injury in patients with myocardial infarction

The concentration of the large-size cardiac troponin ternary complex or total cardiac troponin ternary complex is determined as the characteristic parameter for assessing myocardial injury.

In some embodiments, a ROC curve is plotted to establish a prediction model for the characteristic parameter, and the optimal threshold (cutoff) is calculated as the reference value.

In some embodiments, a binary logistic regression analysis is used to assess the risk of a composite endpoint event (selected from major clinical events including composite events of all-cause mortality, myocardial infarction, and unplanned coronary revascularization, and minor clinical events including any combination of cardiovascular death, components of major clinical events, stroke, hospitalization for heart failure or emergency observation for 24 hours or more, cardiac arrest or malignant arrhythmia, and other cardiovascular disease hospitalization events).

In some embodiments, a COX regression is used to assess the risk of a composite endpoint event.

### (7) Assessment of prognostic risk of patients with chronic myocardial injury

Current research focuses on using troponin I and troponin T to assess the prognosis of patients with chronic myocardial injury. The use of large-size cardiac troponin ternary complex and/or total cardiac troponin ternary complex is expected to improve the accuracy of prediction.

Herein, the markers for assessing the prognostic risk of patients with chronic myocardial injury include large-size cardiac troponin ternary complex and/or total cardiac troponin ternary complex, and one or more of cTnI, cTnT, TnC, cardiac troponin binary complex and total cardiac troponin complex.

Based on the above method, a characteristic parameter for distinction is obtained, and based on the result of the above comparison, the prognostic risk of subject with chronic myocardial injury (e.g., cardiomyopathy, chronic heart failure, structural heart disease, infiltrative disease, stable coronary heart disease, and persistent arrhythmia) is assessed.

In some embodiments, when the characteristic parameter is higher than the reference value of the characteristic parameter, the prognosis is judged to be poor.

In some embodiments, the step of obtaining the characteristic parameter for assessing myocardial injury based on the concentration of the one or more myocardial injury markers comprises:
determining the concentration of the large-size cardiac troponin ternary complex or total cardiac troponin ternary complex as the characteristic parameter for assessing myocardial injury.

The concentration of the large-size cardiac troponin ternary complex or total cardiac troponin ternary complex is determined as the characteristic parameter for assessing myocardial injury. In some embodiments, based on a logistic regression algorithm, the characteristic parameter based on a combination of multiple myocardial injury markers is constructed. One example is a combination of the concentration of large-size cardiac troponin ternary complex and the concentration of total cardiac troponin complex, or a combination of the concentration of large-size cardiac troponin ternary complex and the concentration of cTnT.

In some embodiments, a ROC curve is plotted to establish a prediction model for the characteristic parameter, and the optimal threshold (cutoff) is calculated as the reference value.

In some embodiments, a binary logistic regression analysis is used to assess the risk of a composite endpoint event in a patient.

In some embodiments, a COX regression is used to assess the risk of a composite endpoint event in a patient.

The method of the present application is preferably an ex vivo or *in vitro* method. In addition, it may comprise steps other than the steps explicitly mentioned above. For example, further steps may involve sample pretreatment and assessment of the result obtained by the method. The method may be performed manually or with the assistance of automation. Preferably, the detection step, calculation step and comparison step may be assisted in whole or in part by automation, for example, by a suitable robot and sensory device for detection, a calculation algorithm executed by a computer on a data processing device in the calculation step, or a comparison and/or diagnostic algorithm on a data processing device in the comparison step.

### Device and sample analysis system

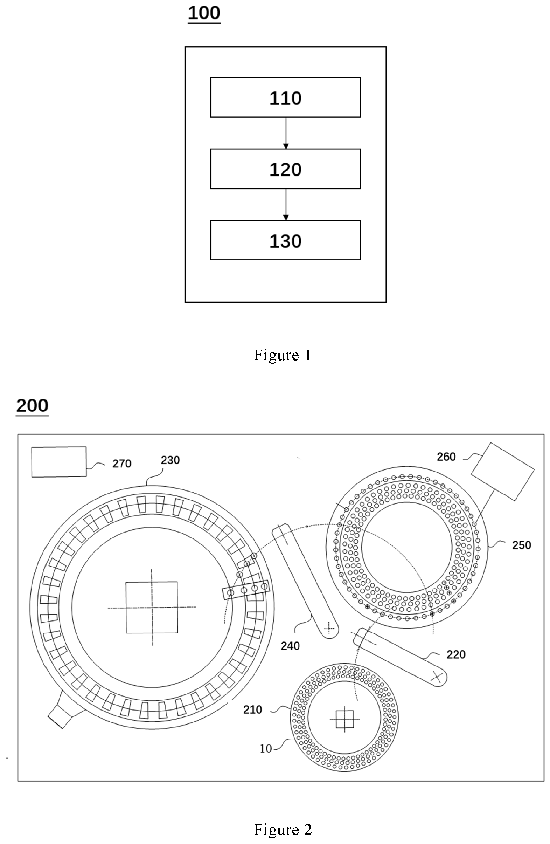

In a second aspect, the present application provides a device for obtaining a characteristic parameter for assessment of myocardial injury in a subject, as shown in Figure 1, the device 100 comprises:
a data receiving module 110, which is configured to receive concentration data of one or more myocardial injury markers obtained from a sample from the subject, wherein the one or more myocardial injury markers comprise a large-size cardiac troponin ternary complex and/or a total cardiac troponin ternary complex;
a data processing module 120, which is configured to process the concentration data of one or more myocardial injury markers received by the receiving module, and obtain the characteristic parameter for assessment of myocardial injury;
and, an output module 130, which is configured to output the characteristic parameter.

In some embodiments, the data processing module is configured to process the concentration of the one or more myocardial injury markers to obtain the characteristic parameter for assessment of myocardial injury, comprising:
being configured to determine the concentration of the large-size cardiac troponin ternary complex or total cardiac troponin ternary complex as the characteristic parameter for assessment of myocardial injury; or
being configured to obtain the characteristic parameter for assessment of myocardial injury based on the concentration of the large-size cardiac troponin ternary complex and the concentration of the total cardiac troponin ternary complex.

In some embodiments, the myocardial injury markers further comprise one or more of the following: cTnI, cTnT, TnC, cardiac troponin binary complex and total cardiac troponin complex.

In some embodiments, the one or more myocardial injury markers further comprise at least one of cTnI, cTnT and total cardiac troponin complex.

In some embodiments, the data processing module is configured to process the concentration of the one or more myocardial injury markers to obtain the characteristic parameter for assessment of myocardial injury, comprising: being configured to input the concentration of the multiple myocardial injury markers into a preset function model to obtain the output of the preset function model as the characteristic parameter for assessment of myocardial injury.

In some embodiments, the data processing module is configured to process the concentration of the one or more myocardial injury markers to obtain the characteristic parameter for assessment of myocardial injury, comprising: being configured to determine one of the following ratio parameters as the characteristic parameter for assessment of myocardial injury, or obtain the characteristic parameter for assessment of myocardial injury based on at least two of the following ratio parameters, or obtain the characteristic parameter for assessment of myocardial injury based on at least one of the following ratio parameters and the ratio of the concentration of cTnT to the concentration of total cardiac troponin complex;
the ratio parameters comprise: the ratio of the concentration of large-size cardiac troponin ternary complex to the concentration of cTnI, the ratio of the concentration of large-size cardiac troponin ternary complex to the concentration of cTnT, the ratio of the concentration of large-size cardiac troponin ternary complex to the concentration of total cardiac troponin complex, the ratio of the concentration of total cardiac troponin ternary complex to the concentration of cTnI, the ratio of the concentration of total cardiac troponin ternary complex to the concentration of cTnT, the ratio of the concentration of total cardiac troponin ternary complex to the concentration of total cardiac troponin complex, and the ratio of the concentration of large-size cardiac troponin ternary complex to the concentration of total cardiac troponin ternary complex.

In some embodiments, the ratio parameter is the ratio of the concentration of large-size cardiac troponin ternary complex to the concentration of cTnI, cTnT or total cardiac troponin complex; optionally, the ratio parameter further comprises the ratio of the concentration of total cardiac troponin ternary complex to the concentration of cTnI, cTnT or total cardiac troponin complex, and/or the ratio of the concentration of cTnT to the concentration of cTnI or total cardiac troponin complex.

In the third aspect, the present application provides a sample analysis system, comprising:
a sample carrying portion, which is used for carrying a container containing a sample from a subject;
a sample dispensing portion, which is used for pipetting the sample from the subject from the sample carrying portion and discharging it into a reaction cup to be added with the sample;
a reagent carrying portion, which is used for carrying a detection reagent;
a reagent dispensing portion, which is used for pipetting the detection reagent from the reagent carrying portion and discharging it into the reaction cup to be added with the reagent;
a reaction portion, which is used for placing the reaction cup so as to incubate a test solution obtained by reacting the sample from the subject with the detection reagent in the reaction cup;
a detection portion, which has a signal detector for detecting a signal of the test solution in the reaction cup to determine and output the concentration of one or more myocardial injury markers in the sample from the subject, wherein the one or more myocardial injury markers comprise a large-size cardiac troponin ternary complex and/or a total cardiac troponin ternary complex; and
a data processing portion, which comprises a processor and a computer-readable storage medium on which the computer-readable instructions are stored, characterized in that the computer-readable instructions cause the processor to implement the following steps when executed by the processor:
   receiving and processing the concentration of the one or more myocardial injury markers to obtain a characteristic parameter for assessment of myocardial injury; and
   outputting the characteristic parameter.

Figure 2 shows a sample analysis system 200 provided in an example of the present application, and the sample analysis system is particularly configured as a chemiluminescence analyzer or comprises a chemiluminescence analyzer. As shown in Figure 2, the sample analysis system 200 comprises a sample carrying portion 210, a sample dispensing portion 220, a reagent carrying portion 230, a reagent dispensing portion 240, a reaction portion 250, a detection portion 260, and a device 270 according to the second aspect.

The sample carrying portion 210 is used to carry a container containing a blood sample from a subject. For example, the sample carrying portion 210 can be configured as a sample tray, the sample tray comprises a plurality of sample positions where the container 10 can be placed, and the sample tray can be rotated to dispatch the container containing the blood sample to a corresponding position, for example, to a position for the sample dispensing portion 220 to pipette the blood sample.

The sample dispensing portion 220 is used to pipette the blood sample from the subject, such as serum, from the sample carrying portion 210 and discharge it into a reaction cup to be loaded with sample. For example, the sample dispensing portion 112 may comprise a sample needle which can move in two dimensions or three dimensions in space through a two-dimensional or three-dimensional driving mechanism, so that the sample needle can move to a position for pipetting blood sample and move to a reaction cup to be loaded with sample, and discharge the pipetted blood sample into the reaction cup.

The reagent carrying portion 230 is used to carry a detection reagent, such as a reagent for determining the concentration of one or more myocardial injury markers. For example, the reagent carrying portion 230 can be configured as a reagent tray with a disc-shaped structure, the reagent tray has a plurality of positions for carrying reagent containers, and the reagent carrying portion 230 can rotate and drive the reagent container it carries to rotate, so as to rotate the reagent container to a specific position, such as a position where the reagent is pipetted by the reagent dispensing portion 240. The number of reagent carrying portion 230 can be one or more.

The reagent dispensing portion 240 is used to pipette the detection reagent from the reagent carrying portion 230 and discharge it into the reaction cup to be loaded with the reagent. For example, the reagent dispensing portion 240 may comprise a reagent needle which can move in two dimensions or three dimensions in space through a two-dimensional or three-dimensional driving mechanism, so that the reagent needle can move to the position of pipetting the reagent and move to the reaction cup to be added with the reagent, and discharge the pipetted reagent to the reaction cup.

The reaction portion 250 is used to place the reaction cup so as to incubate a test solution obtained by the reaction of the blood sample from the subject and the detection reagent in the reaction cup. For example, the reaction portion 250 can be configured as a reaction tray with a disc-shaped structure which has one or more placement positions for placing the reaction cup, and the reaction tray can rotate and drive the reaction cup in its placement position to move, so as to dispatch the reaction cup in the reaction tray and incubate the test solution in the reaction cup.

The detection portion 260 has a signal detector, such as an optical detector, for detecting a signal of the test solution in the reaction cup to determine and output the concentration of one or more myocardial injury markers in the sample from the subject. The detection portion 260 is, for example, arranged outside the reaction portion 250, and the reaction portion 250 rotates so as to drive the container containing the test solution to move to the detection portion 260 for detection. In some embodiments, the detection portion 260 is configured as a photometric device.

For example, when the sample analysis system is configured as a chemiluminescence analyzer or comprises a chemiluminescence analyzer, the detection portion 260 is configured as a photometric device. A specific detection process of the chemiluminescence analyzer is as follows: the sample dispensing portion 220 pipettes the sample to be tested from the sample carrying portion 210 and discharges it to the reaction cup; the reagent dispensing portion 240 pipettes an enzyme-labeled reagent and a magnetic bead reagent from the reagent carrying portion 230 and discharges them to the reaction cup to which the sample has been added, so as to mix them with the sample; then the reaction cup is placed in the reaction portion 250 for reaction, incubation and magnetic separation and cleaning; then a luminescent substrate is added to the reaction cup that has completed the reaction, incubation and magnetic separation and cleaning, and incubated for a period of time; finally, the detection portion 260 detects the photons emitted by the substance to be tested in the sample to be tested under the action of the luminescent substrate, so as to calculate the concentration level of the substance to be tested by the measured number of photons. For other examples and advantages of the sample analysis system, please refer to the above description of the device 100.

### Detection kit and use

In the fourth aspect, the present application provides a use of a reagent for quantitative detection of a large-size cardiac troponin ternary complex and/or a reagent for quantitative detection of a total cardiac troponin ternary complex in a sample in the manufacture of a kit, wherein the kit is used for the assessment of myocardial injury described in the present application. Accordingly, the present application provides a kit comprising a reagent for quantitative detection of a large-size cardiac troponin ternary complex and/or a reagent for quantitative detection of a total cardiac troponin ternary complex in a sample.

In some embodiments, the kit further comprises a reagent for quantitative detection of a total cardiac troponin complex, a reagent for quantitative detection of cTnI, and/or a reagent for quantitative detection of cTnT.

The study found that the use of the kit for detecting large-size cardiac troponin ternary complex and total cardiac troponin ternary complex in the present application is beneficial for more rapidly and accurately obtaining the concentration of myocardial injury markers, and thereby obtaining a characteristic parameter for assessing myocardial injury.

In some embodiments, the reagent for quantitative detection of large-size cardiac troponin ternary complex in a sample comprises a first group of antibodies and a second group of antibodies, wherein,
the first group of antibodies comprises one or more antibodies 1-1, each of the antibodies 1-1 is independently selected from an antibody capable of specifically binding to any fragment of amino acid residues 67-222 of cTnT;
the second group of antibodies comprises one or more antibodies 1-2, each of the antibodies 1-2 is independently selected from an antibody capable of specifically binding to any fragment of the amino acid sequence of TnC.

In some embodiments, the first group of antibodies does not comprise an antibody capable of specifically binding to any fragment of amino acid residues 223-287 of cTnT.

In some embodiments, the second group of antibodies further comprises:
one or more antibodies 1-3, each of antibodies 1-3 is independently selected from an antibody capable of specifically binding to cTnIC; and/or,
one or more antibodies 1-4, each of antibodies 1-4 is independently selected from an antibody capable of specifically binding to any fragment of amino acid residues 18-210 of cTnI.

In some embodiments, the first group of antibodies comprises one or more of the antibodies 1-1, and the second group of antibodies comprises one or more of the antibodies 1-2 and one or more of the antibodies 1-3.

In some embodiments, each of the antibodies 1-1 is independently selected from an antibody capable of specifically binding to amino acids 67-86, amino acids 119-138, amino acids 132-151, amino acids 145-164, or amino acids 171-190 of cTnT. In some embodiments, each of the antibodies 1-1 is independently selected from an antibody capable of specifically binding to amino acids 119-138, amino acids 132-151, or amino acids 171-190 of cTnT.

In some embodiments, each of the antibodies 1-4 is independently selected from an antibody capable of specifically binding to amino acids 1-15, amino acids 13-22, amino acids 18-22, amino acids 18-28, amino acids 18-35, amino acids 22-31, amino acids 22-40, amino acids 23-29, amino acids 24-40, amino acids 25-40, amino acids 26-35, amino acids 34-37, amino acids 41-49, amino acids 83-89, amino acids 86-90, amino acids 87-90, amino acids 117-126, amino acids 130-145, amino acids 169-178, amino acids 186-192, amino acids 190-196, or amino acids 195-209 of cTnI. In some embodiments, each of the antibodies 1-4 is independently selected from an antibody capable of specifically binding to amino acids 22-40, amino acids 41-49, or amino acids 83-89 of cTnI. In some embodiments, each of the antibodies 1-4 is independently selected from an antibody capable of specifically binding to amino acids 41-49 of cTnI.

In some embodiments, the first group of antibodies are capture antibodies, and the second group of antibodies are detection antibodies.

In some embodiments, the reagent for quantitative detection of total cardiac troponin ternary complex in a sample comprises a first detection reagent and a second detection reagent, wherein,
the first detection reagent comprises one or more antibodies 2-1, each of the antibodies 2-1 is independently selected from an antibody capable of specifically binding to any fragment of amino acid residues 223-287 of cTnT;
the second detection reagent comprises one or more antibodies 2-2, each of the antibodies 2-2 is independently selected from an antibody capable of specifically binding to any fragment of the amino acid sequence of TnC.

In some embodiments, the first detection reagent further comprises one or more antibodies 2-3, each of the antibodies 2-3 is independently selected from an antibody capable of specifically binding to any fragment of amino acid residues 67-222 of cTnT.

In some embodiments, the second detection reagent further comprises:
one or more antibodies 2-4, each of the antibodies 2-4 is independently selected from an antibody capable of specifically binding to cTnIC; and/or
one or more antibodies 2-5, each of the antibodies 2-5 is independently selected from an antibody capable of specifically binding to any fragment of amino acid residues 18-210 of cTnI.

In some embodiments, the first detection reagent comprises one or more of the antibodies 2-1 and one or more of the antibodies 2-3; the second detection reagent comprises one or more of the antibodies 2-2, and one or more of the antibodies 2-4 and/or one or more of the antibodies 2-5.

In some embodiments, each of the antibodies 2-1 is independently selected from an antibody capable of specifically binding to amino acids 223-242 and amino acids 262-281 of cTnT. In some embodiments, the antibodies 2-1 are antibodies capable of specifically binding to amino acids 223-242 of cTnT.

In some embodiments, each of the antibodies 2-3 is independently selected from an antibody capable of specifically binding to amino acids 67-86, amino acids 119-138, amino acids 132-151, amino acids 145-164, or amino acids 171-190 of cTnT. In some embodiments, each of the antibodies 2-3 is independently selected from an antibody capable of specifically binding to amino acids 119-138 or amino acids 132-151 of cTnT.

In some embodiments, each of the antibodies 2-5 is independently selected from an antibody capable of specifically binding to amino acids 1-15, amino acids 13-22, amino acids 18-22, amino acids 18-28, amino acids 18-35, amino acids 22-31, amino acids 22-40, amino acids 23-29, amino acids 24-40, amino acids 25-40, amino acids 26-35, amino acids 34-37, amino acids 41-49, amino acids 83-89, amino acids 86-90, amino acids 87-90, amino acids 117-126, amino acids 130-145, amino acids 169-178, amino acids 186-192, amino acids 190-196, or amino acids 195-209 of cTnI. In some embodiments, each of the antibodies 2-5 is independently selected from an antibody capable of specifically binding to amino acids 22-40, amino acids 41-49, or amino acids 83-89 of cTnI. In some embodiments, each of the antibodies 2-5 is independently selected from an antibody capable of specifically binding to amino acids 41-49 of cTnI.

In some embodiments, the first detection reagent is a capture reagent, and the second detection reagent is a detection reagent.

In some embodiments, the kit is used for one or more of the following:
1) staging of myocardial infarction, especially judging whether the subject is in the early stage of myocardial infarction;
2) distinguishing between type I myocardial infarction and chronic cardiac events;
3) excluding the subject with chest pain who have not experienced myocardial injury events;
4) distinguishing between type I myocardial infarction and type II myocardial infarction;
5) distinguishing between myocardial injury due to invasive procedure and chronic cardiac events;
6) assessing the prognosis of acute myocardial injury; or
7) assessing the prognosis of chronic myocardial injury.

Beneficial effects
1) By detecting troponin complexes and fragments in patient samples, the cause and risk of individual myocardial injury can be assessed in the case of elevated troponin concentrations.
2) Avoiding the difficulties caused by continuous monitoring of troponin, improving clinical turnover, and helping to establish a faster diagnostic process.
3) Helping to stratify patients with chest pain and guide diagnostic and treatment decisions.

### Specific Models for Carrying Out the Invention

### Example 1. Detection kit and signal-to-noise ratio analysis

### I. Construction of kits

Those skilled in the art should understand that kits for quantitatively detecting myocardial injury markers, including commercially available kits, can be used to determine the concentration of myocardial injury markers. For non-limiting purposes, the following exemplary descriptions of detection kits for cTnI, cTnT, total cardiac troponin complex, large-size cTnITC and total cTnITC are provided.

Capture antibody-detection antibody (all antibodies used herein are from Hytest) were used in the double antibody sandwich chemiluminescent immunoassay method to construct each kit.
1. Construction of kits for detection of myocardial injury markers cTnI, cTnT and total cardiac troponin complex
   (1) Total complex detection kits:
      Capture antibody: Antibody 19C7cc capable of specifically binding to the 41-49 fragment of cTnI;
      Detection antibody: Antibody 20C6cc capable of specifically binding to the cTnIC complex epitope;
   (2) cTnT detection kit:
      Capture antibody: Antibody 329cc capable of specifically binding to the 119-138 fragment of cTnT;
      Detection antibody: Antibody 406cc capable of specifically binding to the 132-151 fragment of cTnT.
   (3) cTnI detection kit:
      Capture antibody: Antibody 19C7cc capable of specifically binding to the 41-49 fragment of cTnI;
      Detection antibody: Antibody RecR33 capable of specifically binding to the 24-40 fragment of cTnI.
2. Construction of large-size cTnITC detection kits
   (1) Large-size cTnITC Detection Kit **1**
      Capture antibody: Antibody **1-1**: Antibody 329cc capable of specifically binding to the 119-138 fragment of cTnT;
      Detection antibody: Antibody **1-3**: Antibody 20C6cc capable of specifically binding to the epitope of cTnIC complex.
   (2) Large-size cTnITC Detection Kit **2**
      Capture antibody: Antibody **1-1**: Antibody 329cc capable of specifically binding to the 119-138 fragment of cTnT;
      Detection antibody: Antibody **1-4**: Antibody 19C7cc capable of specifically binding to the 41-49 fragment of cTnI.
   (3) Large-size cTnITC Detection Kit **3**
      Capture antibody: Antibody **1-1**: Antibody 329cc capable of specifically binding to the 119-138 fragment of cTnT;
      Detection antibody: Antibody **1-2**: Antibody 7B9cc capable of specifically binding to TnC; and Antibody **1-3**: Antibody 20C6cc capable of specifically binding to the epitope of cTnIC complex.
   (4) Large-size cTnITC Detection Kit **4**
      Capture antibody: Antibody **1-1**: Antibody 329cc capable of specifically binding to the 119-138 fragment of cTnT;
      Detection antibody: Antibody **1-2**: Antibody 7B9cc capable of specifically binding to TnC; and Antibody **1-4**: Antibody 19C7cc capable of specifically binding to the 41-49 fragment of cTnI.
   (5) Large-size cTnITC Detection Kit **5**
      Capture antibody: Antibody **1-1**: Antibody 329cc capable of specifically binding to the 119-138 fragment of cTnT;
      Detection antibody: Antibody **1-2**: Antibody 7B9cc capable of specifically binding to TnC.
   (6) Large-size cTnITC Detection Kit **6**
      Capture antibody: Antibody **1-1**: Antibody 1C11cc capable of specifically binding to the 171-190 fragment of cTnT;
      Detection antibody: Antibody **1-2**: Antibody 7B9cc capable of specifically binding to TnC; and Antibody **1-3**: Antibody Tcom8 capable of specifically binding to the epitope of cTnIC complex.
   (7) Large-size cTnITC Detection Kit **7**
      Capture antibody: Antibody **1-1**: Antibody 406cc capable of specifically binding to the 132-151 fragment of cTnT;
      Detection antibody: Antibody **1-2**: Antibody 7B9cc capable of specifically binding to TnC; and Antibody **1-3**: Antibody 20C6cc capable of specifically binding to the epitope of cTnIC complex.
   (8) Large-size cTnITC Detection Kit **8**
      Capture antibody: Antibody **1-1**: Antibody 300cc capable of specifically binding to the 119-138 fragment of cTnT;
      Detection antibody: Antibody **1-2**: Antibody 7B9cc capable of specifically binding to TnC; and Antibody **1-3**: Antibody 20C6cc capable of specifically binding to the epitope of cTnIC complex.
      In addition to the above kits, kits, constructed by using the following antibodies as Antibody **1-1** of the large-size cTnITC detection Kits **3** to **8**, were also employed in the experiment: Antibody 7F4 or 7G7 with a specific binding site for the 67-86 fragment of cTnT, Antibody 2F3, 1A11 or 1F11cc with a specific binding site for the 145-164 cTnT. Kits, constructed by using the following antibodies as Antibody 1-4 of the large-size cTnITC detection Kit **4**, were also employed in the experiment: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.
3. Construction of total cardiac troponin ternary complex detection kits

Capture antibody-detection antibody were used in the double antibody sandwich chemiluminescence immunoassay method to construct detection kits for detecting the total ternary troponin complex cTnITC in a sample.
Total cTnITC Detection Kit **1**:
   Capture antibody: Antibody **2-1**: 7E7 (capable of specifically binding to the 223-242 fragment of cTnT); Antibody **2-3**: 329cc (capable of specifically binding to the 119-138 fragment of cTnT).
   Detection antibody: Antibody **2-4**: 20C6cc (capable of specifically binding to the epitope of cTnIC complex).
Total cTnITC Detection Kit **2**:
   Capture antibody: Antibody **2-1**: 7E7 (capable of specifically binding to the 223-242 fragment of cTnT); Antibody **2-3**: 329cc (capable of specifically binding to the 119-138 fragment of cTnT).
   Detection antibody: Antibody **2-5**: 19C7cc (capable of specifically binding to the 41-49 fragment of cTnI).
Total cTnITC Detection Kit **3**:
   Capture antibody: Antibody **2-1**: 7E7 (capable of specifically binding to the 223-242 fragment of cTnT); Antibody **2-3**: 329cc (capable of specifically binding to the 119-138 fragment of cTnT).
   Detection antibody: Antibody **2-2**: 7B9cc (capable of specifically binding to TnC); Antibody **2-4**: 20C6cc (capable of specifically binding to the epitope of cTnIC complex);
Total cTnITC Detection Kit **4**:
   Capture antibody: Antibody **2-1**: 7E7 (capable of specifically binding to the 223-242 fragment of cTnT); Antibody **2-3**: 329cc (capable of specifically binding to the 119-138 fragment of cTnT).
   Detection antibody: Antibody **2-2**: 7B9cc (capable of specifically binding to TnC); Antibody **2-5**: 19C7cc (capable of specifically binding to the 41-49 fragment of cTnI).
Total cTnITC Detection Kit **5**:
   Capture antibody: Antibody **2-1**: 7E7 (capable of specifically binding to the 223-242 fragment of cTnT); Antibody **2-3**: 329cc (capable of specifically binding to the 119-138 fragment of cTnT).
   Detection antibody: Antibody **2-2**: 7B9cc (capable of specifically binding to TnC).
Total cTnITC Detection Kit **6**:
   Capture antibody: Antibody **2-1**: 7E7 (capable of specifically binding to the 223-242 fragment of cTnT).
   Detection antibody: Antibody **2-2**: 7B9cc (capable of specifically binding to TnC).
Total cTnITC Detection Kit **7**:
   Capture antibody: Antibody **2-1**: 155 (capable of specifically binding to the 262-281 fragment of cTnT).
   Detection antibody: Antibody **2-2**: 7B9cc (capable of specifically binding to TnC); Antibody **2-4**: Tcom8 (capable of specifically binding to the epitope of cTnIC complex).
Total cTnITC Detection Kit **8**:
   Capture antibody: Antibody **2-1**: 7E7 (capable of specifically binding to the 223-242 fragment of cTnT); Antibody **2-3**: 406cc (capable of specifically binding to the 132-151 fragment of cTnT).
   Detection antibody: Antibody **2-2**: 7B9cc (capable of specifically binding to TnC); Antibody **2-4**: 20C6cc (capable of specifically binding to the epitope of cTnIC complex);
Total cTnITC Detection Kit **9**:
   Capture antibody: Antibody **2-1**: 7E7 (capable of specifically binding to the 223-242 fragment of cTnT); Antibody **2-3**: 300cc (capable of specifically binding to the 119-138 fragment of cTnT).
   Detection antibody: Antibody **2-2**: 7B9cc (capable of specifically binding to TnC); Antibody **2-4**: 20C6cc (capable of specifically binding to the epitope of cTnIC complex);
Total cTnITC Detection Kit **10**:
   Capture antibody: Antibody **2-1**: 155 (capable of specifically binding to the 262-281 fragment of cTnT); Antibody **2-3**: 406cc (capable of specifically binding to the 132-151 fragment of cTnT).
   Detection antibody: Antibody **2-2**: 7B9cc (capable of specifically binding to TnC); Antibody **2-4**: 20C6cc (capable of specifically binding to the epitope of cTnIC complex);
Total cTnITC Detection Kit **11**:
   Capture antibody: Antibody **2-1**: 7E7 (capable of specifically binding to the 223-242 fragment of cTnT); Antibody **2-3**: 7G7 (capable of specifically binding to the 67-86 fragment of cTnT).
   Detection antibody: Antibody **2-2**: 7B9cc (capable of specifically binding to TnC); Antibody **2-4**: 20C6cc (capable of specifically binding to the epitope of cTnIC complex);
Total cTnITC Detection Kit **12**:
   Capture antibody: Antibody **2-1**: 7E7 (capable of specifically binding to the 223-242 fragment of cTnT); Antibody **2-3**: 1C11cc (capable of specifically binding to the 171-190 fragment of cTnT).
   Detection antibody: Antibody **2-2**: 7B9cc (capable of specifically binding to TnC); Antibody **2-4**: Tcom8 (capable of specifically binding to the epitope of cTnIC complex).

In addition to the above kits, kits, constructed by using the following antibodies as Antibody **2-3** of the Total cTnITC Detection Kits **3** to **5** and **8** to **12**, were also employed in the experiment: Antibody 7F4 with a specific binding site for the 67-86 fragment of cTnT, Antibody 2F3, 1A11 or 1F11cc with a specific binding site for the 145-164 fragment of cTnT. Kits, constructed by using the following antibodies as Antibody **2-5** of the Total cTnITC Detection Kit **4** , were also employed in the experiment: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

Each detection kit comprised:
A. magnetic bead coating working solution, used for capturing myocardial injury markers in a sample. The solution comprised a mixture of superparamagnetic microparticles coated with capture antibodies.
B. enzyme marker working solution, used for detecting the myocardial injury markers captured by the superparamagnetic microparticles. The solution comprised a detection antibody labeled with alkaline phosphatase.

### 4. Detection method for myocardial injury markers

The detection method was as follows:
Step I: the sample, the magnetic bead coating working solution, and the enzyme marker working solution were added to a reaction tube. The mixture was incubated to allow the target protein in the sample to bind to the antibody coated on the magnetic beads, and to allow the antibody-alkaline phosphatase marker to bind to the target protein in the sample; after the reaction was completed, the solid phase was placed in a magnetic field, the magnetic field attracted the magnetic beads, the substances bound to the solid phase were retained, and the unbound substances were washed away;
Step II: the chemiluminescent substrate was added to the reaction tube, in which the luminescent substrate (3-(2-spiroadamantane)-4-methoxy-4-(3-phosphoryloxy)-phenyl-1,2-dioxetane, AMPPD) was decomposed by alkaline phosphatase, losing a phosphate group to generate an unstable intermediate product; the intermediate product underwent intramolecular electron transfer to generate methyl m-oxybenzoate anion, chemiluminescence was emitted when the excited anion returned to the ground state, and the number of photons was measured by a photomultiplier tube; the photon count was proportional to the target protein concentration in the sample; and the analyte amount in the sample was determined by the calibration curve.

The above detection kit was compatible with Mindray's fully automatic chemiluminescence analyzers including models CL2000i, CL6000i, CL8000i, and others.

### II. Analysis of signal-to-noise ratio of detection kits

### 1. Analysis of signal-to-noise ratio of large-size cTnITC detection kits

Samples containing antigens at different concentrations were prepared, including two high-concentration samples (high-value samples) and two low-concentration samples (low-value samples), in which the antigen was recombinant cardiac troponin ternary complex (Hytest, 8ITCR). The samples were analyzed using the Large-size cTnITC Detection Kits **1** to **8**, respectively. At the same time, the signal of the blank sample without antigen was recorded and the signal-to-noise ratio was calculated.

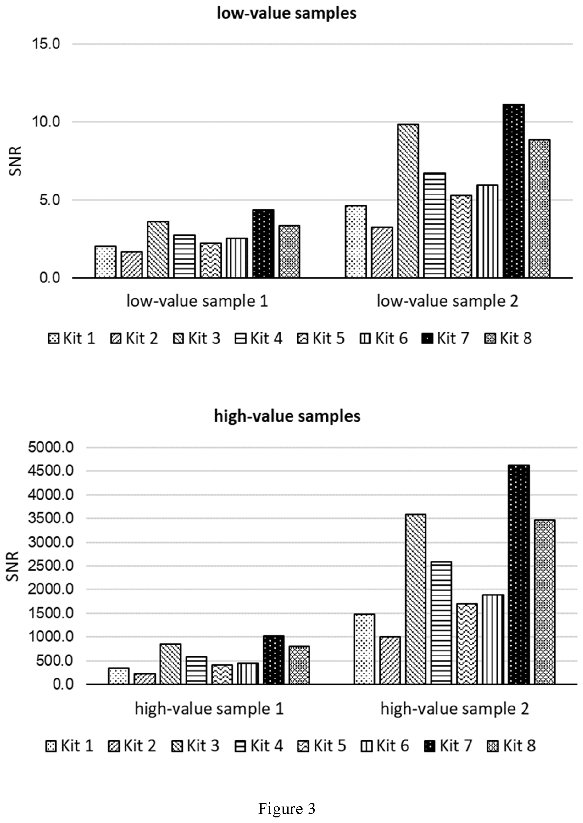

The test results were shown in Figure 3. As shown in Figure 3, all Large-size cTnITC Detection Kits **1** to **8** exhibited good signal-to-noise ratios, meeting clinical requirements. Among them, the signal-to-noise ratio of Large-size cTnITC Detection Kit **3** was significantly higher than those of Large-size cTnITC Detection Kit **1** and Large-size cTnITC Detection Kit **5**, and the signal-to-noise ratio of Large-size cTnITC Detection Kit **4** was significantly higher than those of Large-size cTnITC Detection Kit **2** and Large-size cTnITC Detection Kit **5**, indicating that the use of Antibody **1-2** capable of specifically binding to TnC in combination with Antibody **1-3** or Antibody **1-4** can significantly improve the signal-to-noise ratio. Large-size cTnITC Detection Kits **3**, **7**, and **8** showed high signal-to-noise ratios, suggesting that Antibody **1-1** with specific binding site for amino acids 119-138 and amino acids 132-151 of cTnT was significantly helpful in improving the signal-to-noise ratio.

In addition, the signal differences among samples could be effectively reflected when Antibody **1-1** used in Large-size cTnITC Detection Kits **3** to **8** was replaced with the following antibodies: Antibody 7F4 or 7G7 with a specific binding site for the 67-86 fragment of cTnT, Antibody 2F3, 1A11 or 1F11cc with a specific binding site for the 145-164 fragment of cTnT, and Antibody **1-4** used in Large-size cTnITC Detection Kit **4** was replaced with the following antibodies: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

### 2. Analysis of signal-to-noise ratio of total cTnITC detection kits

The signal-to-noise ratios of Total cTnITC Detection Kits **1** to **12** were analyzed with reference to the signal-to-noise ratio analysis method of large-size cTnITC assay kits.

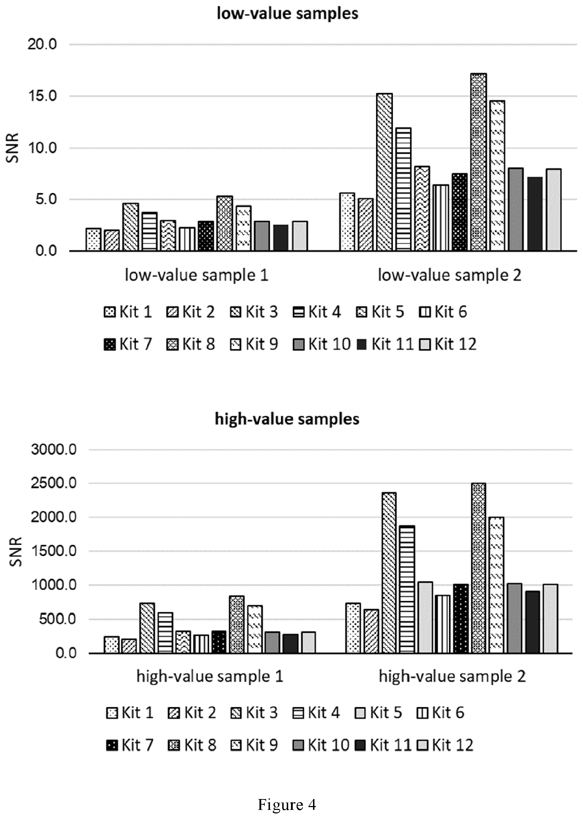

The test results were shown in Figure 4. As shown in Figure 4, all Total cTnITC Detection Kits **1** to **12** exhibited good signal-to-noise ratios, meeting the clinical requirements. Among them, the signal-to-noise ratio of Total cTnITC Detection Kit **5** was higher than that of Total cTnITC Detection Kit **6**, indicating that the addition of Antibody **2-3** capable of specifically binding to the 67-222 fragment of cTnT improved the signal-to-noise ratio. The signal-to-noise ratio of Total cTnITC Detection Kit **3** was significantly higher than those of Total cTnITC Detection Kit **1** and Total cTnITC Detection Kit **5**, and the signal-to-noise ratio of Total cTnITC Detection Kit **4** was significantly higher than those of Total cTnITC Detection Kit **2** and Total cTnITC Detection Kit **5**, indicating that the use of Antibody **2-2** capable of specifically binding to TnC in combination with Antibody **2-4** or Antibody **2-5** could significantly improve the signal-to-noise ratio. The signal-to-noise ratios of Total cTnITC Detection Kits **3**, **8**, and **9** were similar, indicating that when the antibodies capable of specifically binding to different fragments of amino acids 67-222 of cTnT were selected as Antibody **2-3**, the resulting kits had comparable signal-to-noise ratios. The signal-to-noise ratio of Total cTnITC Detection Kit **8** was significantly better than that of Total cTnITC Detection Kit **10**, indicating that using antibodies capable of specifically binding to amino acids 223-242 of cTnT as Antibody **2-1** had a better signal-to-noise ratio than using antibodies capable of specifically binding to amino acids 262-281 of cTnT. The signal-to-noise ratio of Total cTnITC Detection Kit **3** was significantly higher than those of Total cTnITC Detection kits **11** and **12**, indicating that using antibodies capable of specifically binding to amino acids 119-138 of cTnT as Antibody **2-3** had more advantageous than using antibodies capable of specifically binding to amino acids 67-86 or 171-190 of cTnT.

In addition, the signal differences among samples could be effectively reflected when Antibody **2-3** used in Total cTnITC Detection Kits **3** to **5**, and **8** to **12** was replaced with the following antibodies: Antibody 7F4 with a specific binding site for the 67-86 fragment of cTnT, Antibody 2F3, 1A11 or 1F11cc with a specific binding site for the 145-164 fragment of cTnT, and the Antibody **2-5** used in Total cTnITC Detection Kit **4** was replaced with the following antibodies: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

### III. Specificity analysis of detection kits

### 1. Specificity analysis of large-size cTnITC detection kits

Different antigens at the same concentration were added to serum from healthy individuals, and Large-size cTnITC Detection Kits **1** to **8** were used to analyze them by chemiluminescence immunoassay. The antigens analyzed included: cTnT (Hytest, 8RTT5), cTnI (Hytest, 8RT17), cTnIC (Hytest, 8ICR3), cTnITC (Hytest, 8ITCR).

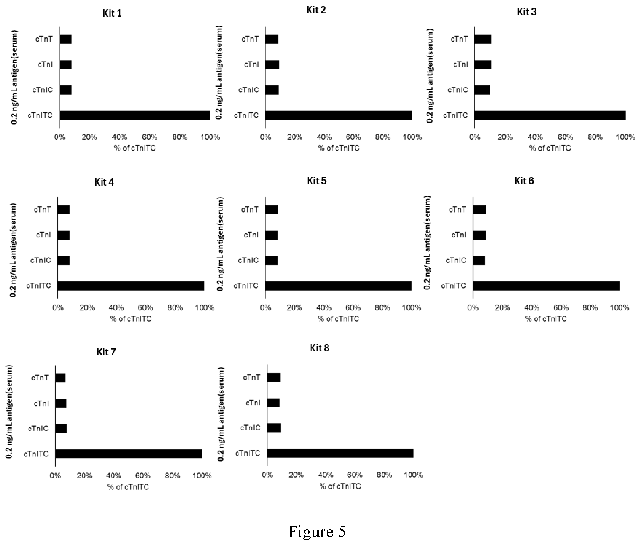

The experimental results were shown in Figure 5. Large-size cTnITC Detection Kits **1** to **8** exhibited exclusive reactivity to the cTnITC antigen, without cross-reactivity against cTnT, cTnI, or binary cTnIC. In addition, the cTnITC antigen could also be effectively recognized when the Antibody **1-1** used in Large-size cTnITC Detection Kits **3** to **8** was replaced with the following antibodies: Antibody 7F4 or 7G7 with a specific binding site for the 67-86 fragment of cTnT, Antibody 2F3, 1A11 or 1F11cc with a specific binding site for the 145-164 fragment of cTnT, and when the Antibody **1-4** used in Large-size cTnITC Detection Kit **4** was replaced with the following antibodies: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

### 2. Specificity analysis of total cTnITC detection kits

The specificity of Total cTnITC Detection Kits **1** to **9** was analyzed with reference to the specificity analysis method for large-size cTnITC detection kits.

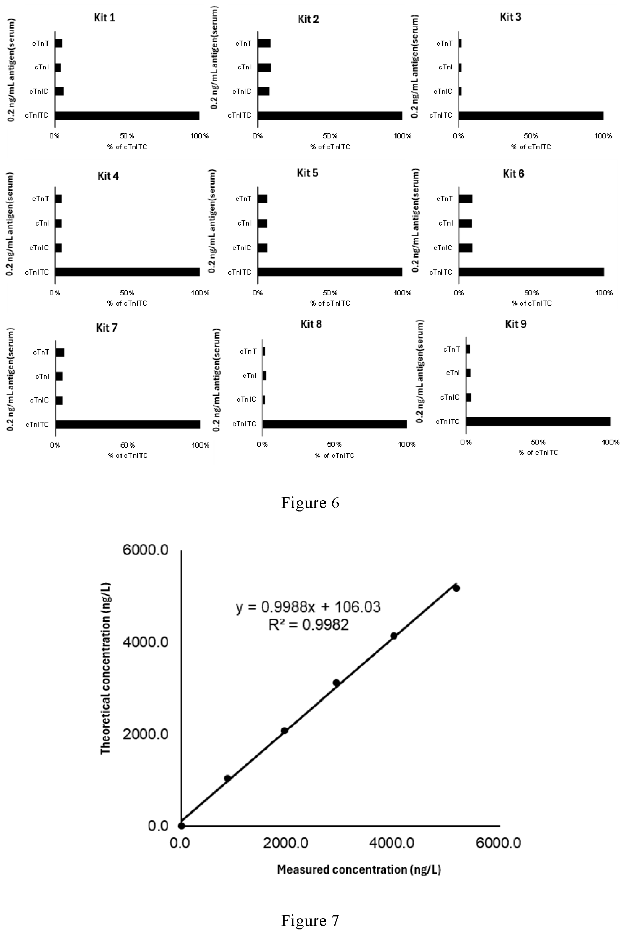

The experimental results were shown in Figure 6. Total cTnITC Detection Kits **1** to **9** exhibited exclusive reactivity to the cTnITC antigen, without cross-reactivity against cTnT, cTnI, or binary cTnIC. In addition, the cTnITC antigen could also be effectively recognized when the Antibody **2-3** used in Total cTnITC Detection Kits **3** to **5** and **8** to **12** was replaced with the following antibodies: Antibody 7F4 with a specific binding site for the 67-86 fragment of cTnT, Antibody 2F3, 1A11 or 1F11cc with a specific binding site for the 145-164 fragment of cTnT, and when the Antibody **2-5** used in Total cTnITC Detection Kit **4** was replaced with the following antibodies: Antibody M18cc with a specific binding site for the 18-28 fragment of cTnI, Antibody 16A11cc, 16A12cc or 8E10cc with a specific binding site of the 86-90 fragment of cTnI, Antibody M46 with a specific binding site for the 130-145 fragment of cTnI, and Antibody MF4cc with a specific binding site for the 190-196 fragment of cTnI.

### IV. Establishment of limit of blank and limit of detection of detection kits

The limit of blank (LoB) and limit of detection (LoD) were established according to the recommendations of the Clinical and Laboratory Standards Institute (CLSI) (EP-17A2 Protocols for Determination of Limits of Detection and Limits of Quantitation).

The LoB test results were obtained from 5 blank samples, which were run for 4 days, with 4 replicates per assay. The general formula was LoB = mean + 1.65*SD.

The LoD test results were obtained from 5 low-concentration samples, which were run for 4 days, with 4 replicates per assay. The general formula was LoD = LoB + 1.65*SD.

The test results were shown in Table 1-1 and Table 1-2.

**Table 1-1: LoB and LoD of large-size cTnITC detection kits**

| Large-size cTnITC Detection Kit | 1 | 2 | 3 | 4 | 5 | 6 | 7 | 8 |
|---|---|---|---|---|---|---|---|---|
| Limit of blank (LoB) (ng/L) | 2.93 | 3.72 | 0.20 | 1.88 | 2.31 | 2.04 | 0.13 | 1.20 |
| Limit of detection (LoB) (ng/L) | 5.12 | 6.02 | 0.34 | 3.85 | 4.37 | 4.03 | 0.31 | 2.83 |

**Table 1-2: LoB and LoD of total cTnITC detection kits**

| Total cTnITC Detection Kit | 1 | 2 | 3 | 4 | 5 | 6 | 7 | 8 | 9 |
|---|---|---|---|---|---|---|---|---|---|
| Limit of blank (LoB) (ng/L) | 4.8 | 6.1 | 0.9 | 2.3 | 3.2 | 4.2 | 3.4 | 0.8 | 1.8 |
| Limit of detection (LoB) (ng/L) | 8.6 | 11.2 | 1.5 | 3.8 | 5.8 | 7.2 | 5.9 | 1.4 | 3.1 |

The blank and detection limits of Large-size cTnITC Detection Kits **1** to **8** met the clinical requirements. Among them, the blank and detection limits of Large-size cTnITC Detection Kit **3** were significantly lower than those of Large-size cTnITC Detection Kit **1** and Large-size cTnITC Detection Kit **5**, and the blank and detection limits of Large-size cTnITC Detection Kit **4** were significantly lower than those of Large-size cTnITC Detection Kit **2** and Large-size cTnITC Detection Kit **5**, indicating that the addition of Antibody **1-2** capable of specifically binding to TnC and use thereof together with Antibody **1-3** or Antibody **1-4** could significantly improve the sensitivity of the detection kits. In addition, the LoB and LoD values were also relatively low when the Antibody **1-1** used in Large-size cTnITC Detection Kits **3** to **8** was replaced with the following antibodies: Antibody 7F4 or 7G7 with a specific binding site for the 67-86 fragment of cTnT, Antibody 2F3, 1A11 or 1F11cc with a specific binding site for the 145-164 fragment of cTnT, and when the Antibody **1-4** used in Large-size cTnITC Detection Kit **4** was replaced with the following antibodies: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

The blank and detection limits of Total cTnITC Detection Kits **1** to **9** all met the clinical requirements. Among them, the blank and detection limits of Total cTnITC Detection Kit **5** were lower than those of Total cTnITC Detection Kit **6**, indicating that the addition of Antibody **2-3** capable of specifically binding to the 119-138 fragment of cTnT improved the sensitivity. The blank and detection limits of Total cTnITC Detection Kit **3** were significantly lower than those of Total cTnITC Detection Kit **1** and Total cTnITC Detection Kit **5**, and the blank and detection limits of Total cTnITC Detection Kit **4** were significantly lower than those of Total cTnITC Detection Kit **2** and Total cTnITC Detection Kit **5**, indicating that the addition of Antibody **2-2** capable of specifically binding to TnC and use thereof together with Antibody **2-4** or Antibody **2-5** significantly improved the sensitivity of the kits. In addition, the LoB and LoD values were also relatively low when the Antibody **2-3** used in Total cTnITC Detection Kits **3** to **5** and **8** to **12** was replaced with the following antibodies: Antibody 7F4 with a specific binding site for the 67-86 fragment of cTnT, Antibody 2F3, 1A11 or 1F11cc with a specific binding site for the 145-164 fragment of cTnT, and when the Antibody **2-5** used in Total cTnITC Detection Kit **4** was replaced with the following antibodies: Antibody M18cc with a specific binding site for the 18-28 fragment of cTnI, Antibody 16A11cc, 16A12cc or 8E10cc with a specific binding site for the 86-90 fragment of cTnI, Antibody M46 with a specific binding site for the 130-145 fragment of cTnI, and Antibody MF4cc with a specific binding site for the 190-196 fragment of cTnI.

### V. Linearity analysis of detection kits

### 1. Linearity analysis of large-size cTnITC detection kits

Large-size cTnITC Detection Kit **3** and Large-size cTnITC Detection Kit **4** were used for linearity analysis.

Clinical serum samples were selected as high-concentration samples, and the high-concentration samples were diluted in a certain ratio to obtain a series of diluted samples, the concentration range of the series of diluted samples was 0 to 6000 ng/L.

The samples were analyzed by chemiluminescent immunoassay using Large-size cTnITC Detection Kit **3**. The average values of the measured concentrations and the theoretical concentrations were linearly fitted, and the correlation coefficient within the linear range was calculated.

The experimental results were shown in Figure 7. The measured concentrations of the diluted samples exhibited a linear relationship with the theoretical concentrations. The R2 value within the linear range (0 to 6000 ng/L) was 0.9982.

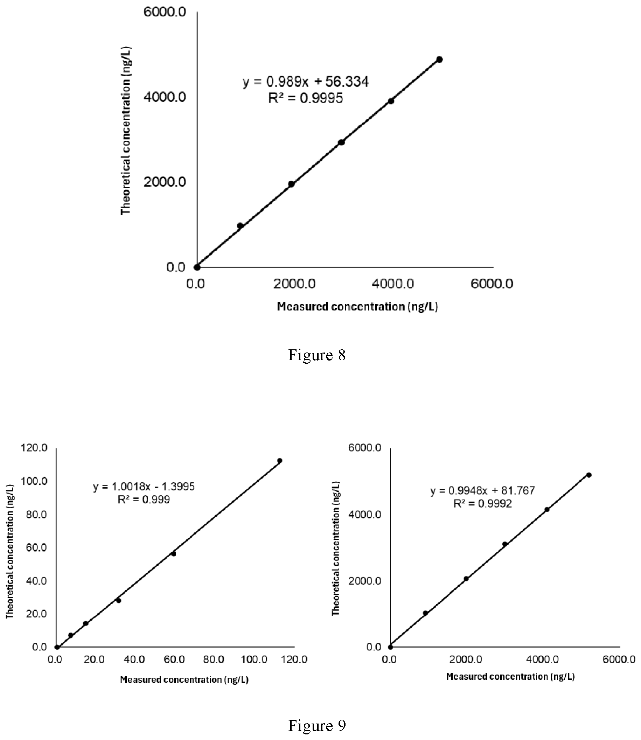

The samples were analyzed by chemiluminescent immunoassay using Large-size cTnITC Detection Kit **4**. The average values of the measured concentration and the theoretical concentrations were linearly fitted, and the correlation coefficient within the linear range was calculated. The experimental results were shown in Figure 8. The measured concentrations of the diluted samples exhibited a linear relationship with the theoretical concentrations. The R2 value within the linear range (0 to 6000 ng/L) was 0.9995.

### 2. Linearity analysis of total cTnITC detection kits

Detection Kits **3** and **4** were used for linearity analysis.

Two clinical serum samples with different concentrations were selected as high-concentration samples, and the high-concentration samples were diluted in a certain ratio to obtain a series of diluted samples. The concentration range of the series samples was 0 to 120 ng/L and 0 to 6000 ng/L. The samples were analyzed by chemiluminescence immunoassay using Detection Kit **3**. The average values of the measured concentrations and the theoretical concentrations were linearly fitted, and the correlation coefficient within the linear range was calculated. The experimental results were shown in Figure 9. The measured concentrations of the diluted samples exhibited a linear relationship with the theoretical concentrations. Within the linear range, the R2 value of the low concentration range (0 to 120 ng/L) was 0.9990, and the R2 value of the high concentration range (0 to 6000 ng/L) was 0.9992.

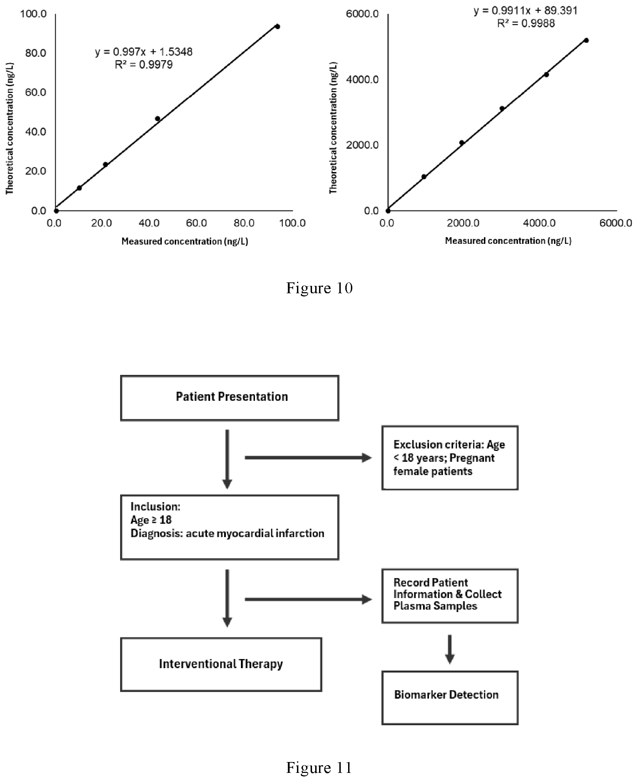

The samples were analyzed by chemiluminescence immunoassay using Detection Kit **4**. The average values of the measured concentrations and the theoretical concentrations were linearly fitted, and the correlation coefficient within the linear range was calculated. The experimental results were shown in Figure 10. The measured concentrations of the diluted samples exhibited a linear relationship with the theoretical concentrations. In the linear range, the R2 value of the low concentration range (0 to 120 ng/L) was 0.9979, and the R2 value of the high concentration range (0 to 6000 ng/L) was 0.9988.

In the following examples, the above-mentioned cTnI detection kit, cTnT detection kit, total cardiac troponin complex detection kit, Large-size cTnITC Detection Kit **7** and Total cTnITC Detection Kit **8** were used to detect the concentration of each marker in samples.

Example 2. Diagnosis of early myocardial infarction (staging of myocardial infarction in patients)

### 1. Patient enrollment

Patients with confirmed admission diagnosis and final diagnosis of Type 1 acute myocardial infarction were enrolled, and all were aged 18 years or older. All enrolled patients were diagnosed with myocardial infarction and subsequently received interventional treatment. Acute myocardial infarction was independently diagnosed by hospital cardiologists in accordance with its definition. The diagnosis of acute myocardial infarction was established based on clinical examinations, including physical examination, echocardiography, electrocardiography, high-sensitivity troponin I detection, high-sensitivity troponin T detection and coronary angiography. Patients with a final diagnosis other than acute myocardial infarction and those with incomplete diagnostic information were excluded. Patients younger than 18 years of age and pregnant female patients were also excluded. Lithium heparin plasma samples were collected prior to interventional treatment for the analysis of troponin complex and fragment composition. The patient enrollment process was shown in Figure 11.

Patient information was recorded, including age, gender, symptoms (chest pain, chest tightness, dyspnea, etc.) and time of onset, past medical history, hypertension, diabetes mellitus, smoking status, creatinine level, and glomerular filtration rate. Samples that met the enrollment criteria were processed for detection of relevant markers. A total of 61 patients with acute myocardial infarction were enrolled, 49 of whom were male, accounting for 80%. The patient information was shown in Table 2-1.

**Table 2-1: Enrollment information of patients with acute myocardial infarction**

| Total number of patients | 61 |
|---|---|
| Age | 59 (50.0-70.5) |
| Male | 49 (80%) |
| Recent smoking status | 17 (28%) |
| Hypertension | 36 (59%) |
| Diabetes mellitus | 14 (23%) |
| Creatinine, mol/L | 88.0 (76.5-105.4) |
| Glomerular filtration rate, eGFR (mL/min/1.73 m²) | 81.0 (63.5-100.0) |
| Time of symptom onset at blood sampling (hours) | 112 (21.0-241.0) |
| | ≤10 hours, 9 (15%) |
| | 10 hours<, ≤30 hours, 9 (15%) |
| | 30 hours<, ≤72hours, 6 (10%) |
| | > 72 hours, 37 (60%) |

| | |
|---|---|
| Note: Continuous variables were presented as medians (25%-75% quartiles); categorical variables were presented as counts (percentage). | |

### 2. Analysis of relationship between concentration of troponin markers and duration of chest pain in patients with acute myocardial infarction

After sample enrollment, the troponin markers in patient samples were measured using a Mindray chemiluminescence analyzer, including total complex, large-size cTnITC, total cTnITC, cTnT and cTnI. The patients were stratified into different subgroups according to the duration of chest pain (≤10 hours, 10-30 hours, 30-72 hours and > 72 hours). The concentrations of total complex, large-size cTnITC, total cTnITC, cTnT and cTnI in patient blood were shown in Table 2-2. Among them, the concentrations of total complex, large-size cTnITC, total cTnITC, cTnT and cTnI in patients with chest pain duration of 10 to 72 hours were significantly higher than those in patients with chest pain duration > 72 hours. The measured values obtained from the cTnI kit were comparable to those of the total complex, indicating a certain degree of clinical equivalence between the two.

**Table 2-2: Concentrations of total complex, large-size cTnITC, total cTnITC, cTnT and cTnI in patients with different durations of chest pain**

| Chest pain duration | ≤10 hours | 10 hours<, ≤30 hours | 30 hours<, ≤72 hours | >72 hours |
|---|---|---|---|---|
| Total complex (10⁻² pmol/L) | 63.3 (22.5-7167.7) | 5797.3 (1421.0-45608.2) | 1326.3 (555.9-5337.8) | 248.8 (38.4-2088.1) |
| Large-size cTnITC (10⁻² pmol/L) | 11.0 (2.7-2348.2) | 578.1 (39.1-2855.3) | 73.3 (4.2-295.5) | 3.0 (0.7-18.4) |
| Total cTnITC (10⁻² pmol/L) | 29.0 (10.5-3591.5) | 1725.2 (1725.2 76.2-30012.2) | 147.0 (3.3-957.7) | 37.8 (2.5-329.8) |
| cTnT (10⁻² pmol/L) | 417.3 (186.7-10079.7) | 7410.4 (1609.4-35288.9) | 2866.4 (1292.3-6713.3) | 1529.9 (232.0-9349.1) |
| cTnI (10⁻² pmol/L) | 82.3 (21.8-8249.1) | 7536.5 (1945.4-48530.8) | 1631.2 (703.2-4804.1) | 281.2 (40.4-2255.2) |

| | | | | |
|---|---|---|---|---|
| Note: Values were shown as medians (25%-75% interquartile range). | | | | |

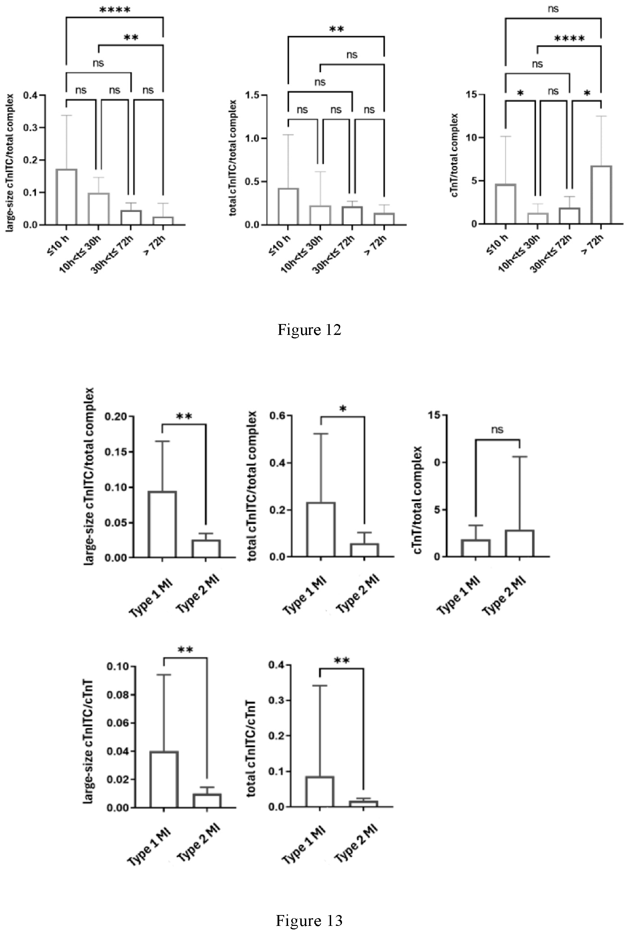

Figure 12 showed the proportional relationships between the complexes and fragments of troponin in patients with different chest pain durations, including the ratio of large-size cTnITC concentration to total complex concentration, the ratio of total cTnITC concentration to total complex concentration, and the ratio of cTnT concentration to total complex concentration. Among them, the proportion of ternary cTnITC (including large-size cTnITC and total cTnITC) was higher in patients with early acute myocardial infarction with shorter chest pain duration, whereas the proportion of cTnT was higher in patients with chest pain duration less than 10 hours or more than 72 hours. The above results indicated that in patients diagnosed with acute myocardial infarction, the complexes and fragment composition of cardiac troponin were highly correlated with the time from symptom onset to blood sampling. In patients with early acute myocardial infarction with a shorter chest pain duration, the proportion of large-size cTnITC or total cTnITC was higher; in patients with chest pain duration of less than 10 hours or more than 72 hours, the proportion of cTnT was higher. The correlation between the complexes and fragment composition of troponin and the duration of chest pain in patients with acute myocardial infarction indicated that it is difficult to accurately judge the stage of disease occurrence using a single marker alone, such as cTnT, especially in patients with less obvious chest pain (e.g., in cases of taking painkillers). Specific identification of different troponin fragments could be used for the diagnosis of acute myocardial infarction and the determination of disease status. In particular, the continuous downward trend of the ternary complexes (including large-size cTnITC and total cTnITC) was more obvious and could be more suitable for early diagnosis.

### 3. Diagnostic performance of concentration and concentration ratio as characteristic parameters for predicting early myocardial infarction

Table 2-3 showed the diagnostic performance of concentration and concentration ratio as characteristic parameters. In this example, the patients with chest pain onset time within 72 hours were selected as patients with early myocardial infarction, among them, the total complex concentration, large-size cTnITC concentration, total cTnITC concentration, cTnI concentration, the concentration ratio of large-size cTnITC to total complex, the concentration ratio of total cTnITC to total complex, the concentration ratio of cTnT to total complex, the concentration ratio of large-size cTnITC to cTnT, the concentration ratio of total cTnITC to cTnT, the concentration ratio of large-size cTnITC to cTnI all had *P* values of less than 0.05, indicating that the variables showed statistically significant differences. SPSS software was used to generate an ROC curve to obtain the area under curve (AUC). The area under curve (AUC) of the large-size cTnITC concentration was 0.833, the AUC of the concentration ratio of large-size cTnITC to total complex was 0.883, and the AUC of the concentration ratio of large-size cTnITC to cTnT was 0.923, indicating favorable diagnostic performance. The measured values obtained from cTnI kit were comparable to those of total complex, indicating clinical equivalence between the two. The results showed that compared with using the concentration of total complex, cTnT or cTnI alone as a characteristic parameter, using the concentration of large-size cTnITC or total cTnITC alone, or using the ratio of large-size cTnITC concentration or total cTnITC concentration to total complex concentration, cTnT concentration or cTnI concentration as a characteristic parameter could more effectively predict early myocardial infarction.

**Table 2-3: Concentration and concentration ratio as characteristic parameters for diagnosis of early myocardial infarction**

| Characteristic parameter | ROC_AUC | Standard error | *P* | 95% CI lower limit | 95% CI upper limit |
|---|---|---|---|---|---|
| Total complex concentration | 0.659 | 0.073 | 0.037 | 0.515 | 0.803 |
| Large-size cTnITC concentration | 0.833 | 0.053 | 0.000 | 0.729 | 0.937 |
| Total cTnITC concentration | 0.663 | 0.072 | 0.032 | 0.522 | 0.805 |
| cTnT concentration | 0.570 | 0.074 | 0.360 | 0.425 | 0.715 |
| cTnI concentration | 0.657 | 0.074 | 0.040 | 0.511 | 0.802 |
| Large-size cTnITC concentration/total complex concentration | 0.883 | 0.047 | 0.000 | 0.790 | 0.975 |
| Total cTnITC concentration/total complex concentration | 0.736 | 0.075 | 0.002 | 0.590 | 0.883 |
| cTnT concentration/total complex concentration | 0.806 | 0.064 | 0.000 | 0.680 | 0.931 |
| Large-size cTnITC concentration/cTnT concentration | 0.923 | 0.034 | 0.000 | 0.856 | 0.990 |
| Total cTnITC concentration/cTnT concentration | 0.775 | 0.072 | 0.000 | 0.634 | 0.916 |
| Large-size cTnITC concentration/cTnI concentration | 0.880 | 0.048 | 0.000 | 0.785 | 0.974 |

The total complex concentration, large-size cTnITC concentration, total cTnITC concentration, cTnI concentration, concentration ratio of large-size cTnITC to total complex, concentration ratio of total cTnITC to total complex, concentration ratio of cTnT to total complex, concentration ratio of large-size cTnITC to cTnT, concentration ratio of total cTnITC to cTnT, and concentration ratio of large-size cTnITC to cTnI were used as the sensitivity and specificity corresponding to different cutoff values of characteristic parameters to calculate Youden indexes, and the optimal diagnostic CUTOFF values were determined according to the maximum values of the Youden indexes, as shown in Table 2-4.

**Table 2-4: Early myocardial infarction diagnosis cutoff values and corresponding sensitivity and specificity using concentration and concentration ratio characteristic parameters**

| Characteristic parameter | CUTOFF | Sensitivity | Specificity | Maximum of Youden index |
|---|---|---|---|---|
| Total complex concentration | 250.0 | 0.792 | 0.514 | 0.305 |
| Large-size cTnITC concentration | 41.2 | 0.625 | 0.919 | 0.544 |
| Total cTnITC concentration | 1671.2 | 0.333 | 0.973 | 0.306 |
| cTnI concentration | 282.3 | 0.792 | 0.514 | 0.305 |
| Large-size cTnITC concentration/total complex concentration | 0.0450 | 0.833 | 0.919 | 0.752 |
| Total cTnITC concentration/total complex concentration | 0.1450 | 0.833 | 0.622 | 0.455 |
| cTnT concentration/total complex concentration | 3.4000 | 0.784 | 0.792 | 0.575 |
| Large-size cTnITC concentration/cTnT concentration | 0.0175 | 0.833 | 0.892 | 0.725 |
| Total cTnITC concentration/cTnT concentration | 0.0365 | 0.833 | 0.730 | 0.563 |
| Large-size cTnITC concentration/cTnI concentration | 0.0345 | 0.875 | 0.865 | 0.740 |

### 4. Diagnostic performance of the combination of multiple concentration characteristic parameters for predicting early myocardial infarction

The characteristic parameter of marker concentration comprised total complex concentration, large-size cTnITC concentration, total cTnITC concentration, cTnT concentration, and cTnI concentration. In this example, patients with chest pain onset within 72 hours were selected as early myocardial infarction patients.

The large-size cTnITC concentration and cTnT concentration were selected as the characteristic variables for conjoint analysis, and the characteristic parameter was constructed using the logistic regression algorithm. The coefficient of each characteristic variable was estimated by logistic regression, and the calculation formula for the characteristic parameter was derived as follows: Logit(P) = 0.0661*(large-size cTnITC concentration) - 0.0012*(cTnT concentration) - 0.4725. The calculation method of prediction probability (Characteristic Parameter **1**) was: P=1/(1+e^{-logit})*100%.

The large-size cTnITC concentration and total complex concentration were selected as the characteristic variables for conjoint analysis, and the characteristic parameter was constructed using the logistic regression algorithm. The coefficient of each characteristic variable was estimated by logistic regression, and the calculation formula for the characteristic parameter was derived as follows: Logit(P) = 0.0313*(large-size cTnITC concentration) - 0.0009*(total complex concentration) - 0.8964. The calculation method of prediction probability (Characteristic Parameter **2**) was: P=1/(1+e^{-logit})*100%.

The large-size cTnITC concentration and total cTnITC concentration were selected as characteristic variables for conjoint analysis. The characteristic parameter was constructed using the logistic regression algorithm. The coefficient of each characteristic variable was estimated by logistic regression, and the calculation formula for the characteristic parameter was derived as follows: Logit(P) = 0.0224*(large-size cTnITC concentration) - 0.0013*(total cTnITC concentration) - 1.2990. The calculation method of the predicted probability (Characteristic Parameter **3**) was: P=1/(1+e^{-logit})*100%.

The large-size cTnITC concentration, total cTnITC concentration, total complex concentration, and cTnT concentration were selected as characteristic variables for conjoint analysis. The characteristic parameter was constructed using the logistic regression algorithm. The coefficient of each characteristic variable was estimated by logistic regression, and the calculation formula of the characteristic parameter was derived as follows: Logit(P) = 0.0615*(large-size cTnITC concentration) - 0.0002*(total cTnITC concentration) + 0.0003*(total complex concentration) - 0.0013*(cTnT concentration)-0.4727. The calculation method of the predicted probability (Characteristic Parameter **4**) was: P=1/(1+e^{-logit})*100%.

Among them, the area under curve (AUC) of Characteristic Parameter **1**, Characteristic Parameter **2**, Characteristic Parameter **3**, and Characteristic Parameter **4** were 0.923, 0.880, 0.858, and 0.920, respectively, indicating good diagnostic performance, as shown in Table 2-5. The optimal CUTOFF values determined based on the maximum values of Youden indexes were 0.3829, 0.2949, 0.2249, and 0.3592, respectively, as shown in Table 2-6.

**Table 2-5: Diagnostic performance of conjoint analysis of multiple concentration characteristic parameters in predicting early myocardial infarction**

| Characteristic parameter | ROC_AUC | Standard error | *P* | 95% CI lower limit | 95% CI upper limit |
|---|---|---|---|---|---|
| Characteristic Parameter 1 | 0.923 | 0.035 | 0.000 | 0.854 | 0.993 |
| Characteristic Parameter 2 | 0.880 | 0.050 | 0.000 | 0.782 | 0.977 |
| Characteristic Parameter 3 | 0.858 | 0.052 | 0.000 | 0.756 | 0.961 |
| Characteristic Parameter 4 | 0.920 | 0.036 | 0.000 | 0.849 | 0.991 |

**Table 2-6: Sensitivity and specificity corresponding to the cutoff values of conjoint analysis of multiple concentration characteristic parameters**

| Characteristic parameter | CUTOFF | Sensitivity | Specificity | Maximum of Youden index |
|---|---|---|---|---|
| Characteristic Parameter 1 | 0.3829 | 0.833 | 0.919 | 0.752 |
| Characteristic Parameter 2 | 0.2949 | 0.875 | 0.838 | 0.713 |
| Characteristic Parameter 3 | 0.2249 | 0.833 | 0.811 | 0.644 |
| Characteristic Parameter 4 | 0.3592 | 0.875 | 0.838 | 0.713 |

In addition, other concentration characteristic parameter combinations included: total cTnITC concentration + total complex concentration, total cTnITC concentration + cTnT concentration, large-size cTnITC concentration + total cTnITC concentration + total complex concentration, large-size cTnITC concentration + total cTnITC concentration + cTnT concentration, large-size cTnITC concentration + total complex concentration + cTnT concentration, total cTnITC concentration + total complex concentration + cTnT concentration, and they all had good diagnostic performance when applied in combination in the above conjoint analysis for the diagnosis of early myocardial infarction. The results showed that the large-size cTnITC concentration and/or total cTnITC concentration in combination with other troponin fragment concentrations had good performance in predicting early myocardial infarction.

### 5. Diagnostic performance of concentration ratio characteristic parameter in predicting early myocardial infarction

The concentration ratio characteristic parameter included the ratio of the concentration of large-size cTnITC or total cTnITC to the concentration of total complex, cTnT or cTnI, that was, large-size cTnITC concentration/total complex concentration, total cTnITC concentration/total complex concentration, cTnT concentration/total complex concentration, large-size cTnITC concentration/cTnT concentration, total cTnITC concentration/cTnT concentration, large-size cTnITC concentration/cTnI concentration, total cTnITC concentration/cTnT concentration. In this example, patients with chest pain onset within 72 hours were selected as early myocardial infarction patients.

The large-size cTnITC concentration/total complex concentration, total cTnITC concentration/total complex concentration, and cTnT concentration/total complex concentration were selected as characteristic variables for conjoint analysis. The characteristic parameter was constructed using the logistic regression algorithm. The coefficient for each characteristic variable was estimated by logistic regression, and the calculation formula of the characteristic parameter was derived as follows: Logit(P) = 27.708*(large-size cTnITC concentration/total complex concentration) + 9.322*(total cTnITC/total complex) - 0.558*(cTnT concentration/total complex concentration) - 1.456. The calculation method of the predicted probability (Characteristic Parameter **5**) was: P=1/(1+e^{-logit})*100%.

The large-size cTnITC concentration/cTnT concentration, and total cTnITC concentration/cTnT concentration were selected as characteristic variables for conjoint analysis. The characteristic parameter was constructed using the logistic regression algorithm. The coefficient for each characteristic variable was estimated by logistic regression, and the calculation formula of the characteristic parameter, i.e., Logit(P) = 78.924*(large-size cTnITC concentration/cTnT concentration) + 21.483*(total cTnITC concentration/cTnT concentration) - 3.004. The calculation method of the predicted probability (Characteristic Parameter **6**) was: P=1/(1+e^{-logit})*100%.

Among them, the area under curve (AUC) values of Characteristic Parameter **5** and Characteristic Parameter **6** were 0.945 and 0.938, respectively, indicating good diagnostic performance, as shown in Table 2-7. The optimal diagnostic CUTOFF values determined by the maximum value of Youden index were 0.2729 and 0.2031, respectively, as shown in Table 2-8.

**Table 2-7: Diagnostic performance of conjoint analysis of multiple concentration ratio characteristic parameters in predicting early myocardial infarction**

| Characteristic parameter | ROC_AUC | Standard error | *P* | 95% CI lower limit | 95% CI upper limit |
|---|---|---|---|---|---|
| Characteristic Parameter **5** | 0.945 | 0.027 | 0.000 | 0.892 | 0.998 |
| Characteristic Parameter **6** | 0.938 | 0.028 | 0.000 | 0.883 | 0.994 |

**Table 2-8: Sensitivity and specificity corresponding to the cutoff values of conjoint analysis of multiple concentration ratio parameters**

| Characteristic parameter | CUTOFF | Sensitivity | Specificity | Maximum of Youden index |
|---|---|---|---|---|
| Characteristic Parameter **5** | 0.2729 | 0.958 | 0.811 | 0.769 |
| Characteristic Parameter **6** | 0.2031 | 0.958 | 0.811 | 0.769 |

In addition, other concentration ratio parameter combinations included large-size cTnITC concentration/total complex concentration + total cTnITC concentration/total complex concentration, large-size cTnITC concentration/total complex concentration + cTnT concentration/total complex concentration, total cTnITC concentration/total complex concentration + cTnT concentration/total complex concentration, large-size cTnITC concentration/total complex concentration + total cTnITC concentration/total complex concentration + large-size cTnITC concentration/cTnT concentration, large-size cTnITC concentration/total complex concentration + total cTnITC concentration/total complex concentration + total cTnITC concentration/cTnT concentration, and large-size cTnITC concentration/total complex concentration + total cTnITC concentration/total complex concentration + total cTnITC concentration/cTnT concentration, and they had good diagnostic performance when used in the above-mentioned conjoint analysis for diagnosis of early myocardial infarction. The results showed that the combination of the ratio between large-size cTnITC and other troponin fragments and the ratio between total cTnITC and other troponin fragments, as well as the ratio between at least one of them and other troponin fragments, had good performance in predicting early myocardial infarction.

The above data showed that the large-size cTnITC concentration and total cTnITC concentration could can be used as characteristic parameter alone to predict early myocardial infarction. The ratio of large-size cTnITC concentration or total cTnITC concentration to total complex concentration, cTnT or cTnI, preferably large-size cTnITC concentration/total complex concentration, large-size cTnITC concentration/cTnT concentration or large-size cTnITC concentration/cTnI concentration, could also be used to predict early myocardial infarction. In addition, the combined use of multiple parameters, such as the combined use of large-size cTnITC concentration and cTnT concentration, the combined use of large-size cTnITC concentration and total complex concentration, the combined use of large-size cTnITC concentration and total cTnITC concentration, or the combined use of large-size cTnITC concentration, total cTnITC concentration, total complex concentration and cTnT concentration, or the combined use of large-size cTnITC concentration/total complex concentration, total cTnITC concentration/total complex concentration and cTnT concentration/total complex concentration, or large-size cTnITC concentration/cTnT concentration and total cTnITC concentration/cTnT concentration, could further improve the diagnostic performance. Although not bound by theory, it was possible that the large-size cTnITC or the characteristic parameter obtained based on large-size cTnITC was more suitable for the diagnosis of early myocardial infarction.

### Example 3. Distinguishing between Type 1 and Type 2 myocardial infarction

### 1. Patient enrollment

Patients with confirmed admission diagnosis and final diagnosis of Type 1 acute myocardial infarction and Type 2 acute myocardial infarction were enrolled, and all were aged 18 years or older. All enrolled patients were diagnosed with Type 1 acute myocardial infarction or Type 2 acute myocardial infarction, and the onset of symptoms was less than 72 hours. Acute myocardial infarction was independently diagnosed by hospital cardiologists in accordance with its definition of acute myocardial infarction. The determination of acute myocardial infarction was established based on clinical examinations, including physical examination, echocardiography, electrocardiography, high-sensitivity troponin I detection, high-sensitivity troponin T detection, and coronary angiography. Patients with a final diagnosis other than Type 1 acute myocardial infarction or Type 2 acute myocardial infarction and those with incomplete diagnostic information were excluded. Patients younger than 18 years of age and pregnant female patients were excluded. Lithium heparin plasma samples were collected prior to patient treatment for the analysis of troponin complexes and fragment composition.

A total of 24 patients with Type 1 acute myocardial infarction and 6 patients with Type 2 acute myocardial infarction were enrolled.

### 2. Analysis of concentrations of myocardial injury markers in patients with Type 1 myocardial infarction and Type 2 myocardial infarction

After the samples were enrolled, the troponin markers in the samples of the enrolled patients, including total complex, large-size cTnITC, total cTnITC, cTnT and cTnI, were detected using a Mindray chemiluminescence instrument. The differences in troponin composition between patients with acute myocardial infarction and patients with cardiomyopathy or chronic heart failure and pneumonia were analyzed. The concentrations of total complex, large-size cTnITC, total cTnITC, cTnT and cTnI in patient blood were shown in Table 3-1. Among them, the concentrations of total complex, large-size cTnITC, total cTnITC and cTnI in patients with Type 1 myocardial infarction were significantly higher than those in patients with Type 2 myocardial infarction. The measured values obtained from the cTnI kit were comparable to those of the total complex, indicating a certain degree of clinical equivalence between the two.

**Table 3-1: Concentrations of total complex, large-size cTnITC, total cTnITC, cTnT, and cTnI in patients with Type 1 myocardial infarction and Type 2 myocardial infarction**

| Myocardial infarction | Type 1 myocardial infarction | Type 2 myocardial infarction | *P* value |
|---|---|---|---|
| Total complex (10⁻² pmol/L) | 1606.3 (257.2-12555.3) | 120.6 (87.6-285.1) | * |
| Large-size cTnITC (10⁻² pmol/L) | 77.2 (8.2-931.4) | 3.9 (1.2-7.0) | ** |
| Total cTnITC (10⁻² pmol/L) | 217.4 (8.5-3733.5) | 6.5 (4.2-17.0) | * |
| cTnT (10⁻² pmol/L) | 2728.0 (811.6-14839.3) | 430.3 (283.6-1404.7) | ns |
| cTnI (10⁻² pmol/L) | 2122.2 (297.3-13537.9) | 136.2 (90.2-343.2) | * |

| | | | |
|---|---|---|---|
| Note: Values were shown as medians (25%-75% interquartile range). *P* values between Type 1 myocardial infarction and Type 2 myocardial infarction were compared using the Mann-Whitney U test. **P*<0.05*, **P*<0.01*, ***P*<0.001*, ****P<*0.0001, ns: no significant difference. | | | |

Figure 13 showed the relationship between the ratios of troponin complexes and fragments in patients with Type 1 myocardial infarction and Type 2 myocardial infarction, including the ratio of large-size cTnITC concentration to total complex concentration, the ratio of total cTnITC concentration to total complex concentration, the ratio of cTnT concentration to total complex concentration, the ratio of large-size cTnITC concentration to cTnT concentration, and the ratio of total cTnITC concentration to cTnT concentration. Among them, the ratio of large-size cTnITC concentration to total complex concentration, the ratio of total cTnITC concentration to total complex concentration, the ratio of large-size cTnITC concentration to cTnT concentration, and the ratio of total cTnITC concentration to cTnT concentration were higher in patients with Type 1 myocardial infarction and lower in patients with Type 2 myocardial infarction.

The above data showed that the large-size cTnITC concentration or total cTnITC concentration could be used to distinguish Type 1 myocardial infarction from Type 2 myocardial infarction. Among them, the large-size cTnITC was preferred.

### Example 4-1. Distinguishing between acute myocardial infarction (Type 1) and chronic cardiac events

### 1. Patient enrollment

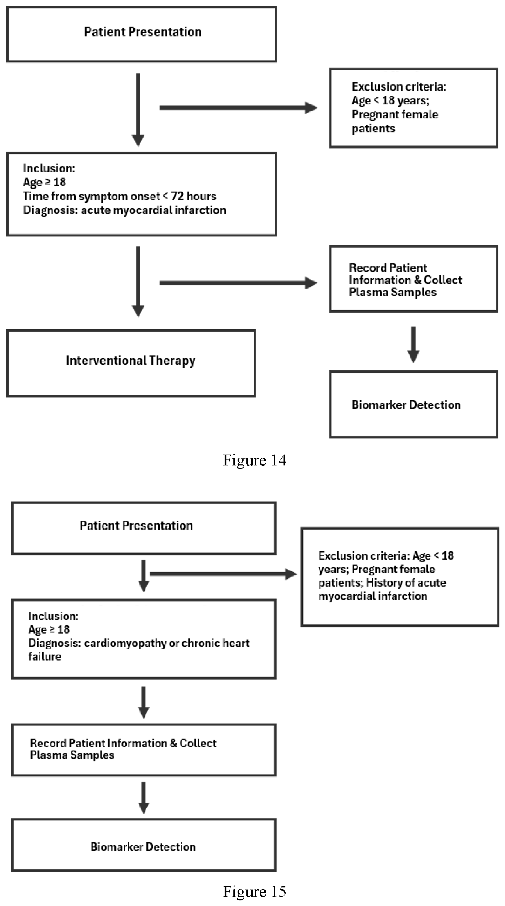

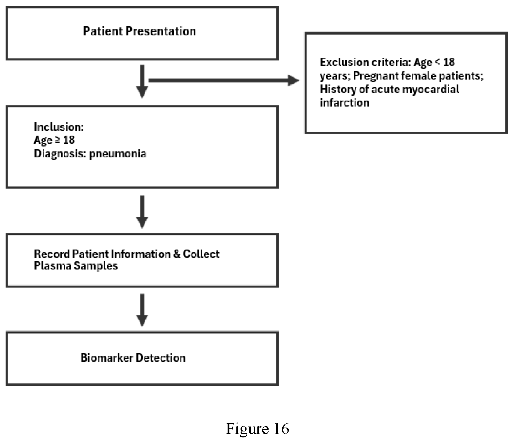

(1) Acute myocardial infarction (Type 1) patient group: Patients with confirmed admission diagnosis and final diagnosis of Type 1 acute myocardial infarction were enrolled. All were aged 18 years or older, and all were diagnosed with acute myocardial infarction with a symptom onset of less than 72 hours. These patients subsequently received interventional treatment. Acute myocardial infarction was independently diagnosed by hospital cardiologists in accordance with its definition. Clinical examinations, including physical examination, echocardiography, electrocardiography, high-sensitivity troponin I detection, high-sensitivity troponin T detection, and coronary angiography and other methods were used to complete the determination of acute myocardial infarction. Patients with a final diagnosis other than acute myocardial infarction and those with incomplete diagnostic information were excluded. Patients under 18 years of age and pregnant female patients were also excluded. Lithium heparin plasma samples were collected prior to interventional treatment and used for the analysis of troponin complexes and fragment composition. The patient enrollment process was shown in Figure 14.
(2) Chronic cardiac event patient group: a) Patients with confirmed admission diagnosis and final diagnosis of chronic heart failure or cardiomyopathy were enrolled, and all were aged 18 years or older. Patients with a final diagnosis other than chronic heart failure or cardiomyopathy and those also diagnosed with acute myocardial infarction concurrently were excluded. Patients under 18 years of age and pregnant female patients were also excluded. Cardiomyopathy and chronic heart failure were independently diagnosed by hospital clinicians. Acute myocardial infarction was independently diagnosed by hospital cardiologists in accordance with its definition. The first lithium heparin plasma samples after admission were collected from these patients and used for the analysis of troponin complexes and fragment composition. The patient enrollment process was shown in Figure 15.
   b) Patients with confirmed admission diagnosis and final diagnosis of pneumonia were enrolled, and all were aged 18 years or older. Patients with a final diagnosis other than pneumonia and those also diagnosed with acute myocardial infarction were excluded. Patients under 18 years of age and pregnant female patients were also excluded. Pneumonia was diagnosed independently by hospital clinicians. Acute myocardial infarction was independently diagnosed by hospital cardiologists in accordance with its definition. The first lithium heparin plasma samples after admission of these patients were collected and used for the analysis of troponin complexes and fragment composition. The patient enrollment process was shown in Figure 16.

The patient information was recorded, including: age, gender, past medical history, hypertension, diabetes mellitus, smoking status, creatinine level, and glomerular filtration rate. Samples that met the enrollment criteria were selected for detection of relevant markers. A total of 24 patients with acute myocardial infarction (Type 1) and 145 patients with chronic cardiac events were enrolled, of whom 94 samples of hospitalized patients with cardiomyopathy or chronic heart failure and 51 samples of patents with pneumonia were enrolled. The patient information was shown in Table 4-1-1.

**Table 4-1-1: Characteristics of enrolled patients.**

| | Acute myocardial infarction (Type 1) | |
|---|---|---|
| Total number of patients | n= 24 | |
| Age | 59 (51.3-68.3) | |
| Male | 22 (92%) | |
| Recent smoking status | 11 (46%) | |
| Hypertension | 13 (54%) | |
| Diabetes mellitus | 6 (25%) | |
| Creatinine, mmol/L | 87.5 (77.3-104.5) | |
| Glomerular filtration rate, eGFR (mL/min/1.73 m²) | 80.5 (65.0-98.3) | |

| Chronic cardiac event | Cardiomyopathy or chronic heart failure | Pneumonia |
|---|---|---|
| Total number of patients | n= 94 | n= 51 |
| Age | 63 (54.8-69.3) | 75 (68.0-81.0) |
| Male | 61 (65%) | 31 (61%) |
| Recent smoking status | 3 (3%) | 1 (2%) |
| Hypertension | 52 (55%) | 28 (55%) |
| Diabetes mellitus | 20 (21%) | 14 (27%) |
| Creatinine, mmol/L | 87.0 (72.0-108.3) | 78.0 (66.0-103.5) |
| Glomerular filtration rate, eGFR (mL/min/1.73 m²) | 75.0 (57.0-99.0) | 77.0 (52.3-85.3) |
| COVID-19 infection | - | 30 (59%) |

Continuous variables were presented as medians (25%-75% quartiles); categorical variables were presented as counts (percentages).

### 2. Analysis of troponin composition in samples of acute myocardial infarction (Type 1) and chronic cardiac events

After the samples were enrolled, the troponin markers in the samples, including troponin total complex, large-size cTnITC, total cTnITC and cTnT, were detected using a Mindray chemiluminescence analyzer and supporting reagents. The differences in troponin composition between patients with acute myocardial infarction (Type 1) and patients with cardiomyopathy or chronic heart failure and pneumonia were analyzed. The concentrations of total troponin complex, large-size cTnITC, total cTnITC and cTnT in patient blood were shown in Table 4-1-2.

**Table 4-1-2: Concentrations of total complex, large-size cTnITC, total cTnITC and cTnT in patients with acute myocardial infarction (Type 1) and chronic cardiac events**

| Cause of myocardial injury | Acute myocardial infarction (Type 1) | Cardiomyopathy or chronic heart failure | Pneumonia |
|---|---|---|---|
| Total troponin complex (10⁻² pmol/L) | 1606.3 (257.2-12555.3) | 19.5 (9.9-48.2) | 9.5 (4.9-45.3) |
| Large-size cTnITC (10⁻² pmol/L) | 77.2 (8.2-931.4) | 0.8 (0.3-1.7) | 0.3 (0.1-0.6) |
| Total cTnITC (10⁻² pmol/L) | 217.4 (8.5-3733.5) | 3.0 (1.2-4.9) | 2.9 (1.2-4.9) |
| cTnT (10⁻² pmol/L) | 2728.0 (811.6-14839.3) | 193.8 (105.9-326.1) | 172.1 (94.6-363.9) |
| cTnI (10⁻² pmol/L) | 2122.2 (297.3-13537.9) | 22.3 (10.2-57.1) | 9.6 (5.3-52.5) |

| | | | |
|---|---|---|---|
| Note: Values were shown as medians (25%-75% interquartile ranges). | | | |

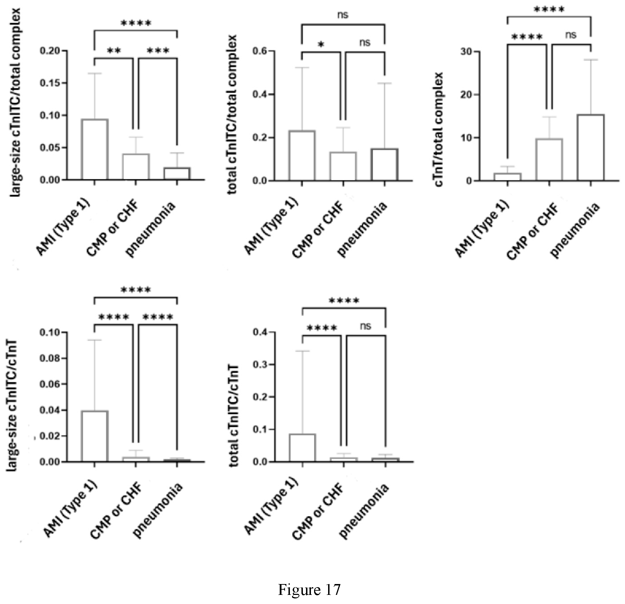

Figure 17 showed the proportional relationship between the troponin complexes and fragments in patients with acute myocardial infarction (Type 1) and patients with chronic cardiac events, including the ratios of large-size cTnITC/total complex, total cTnITC/total complex, cTnT/total complex, large-size cTnITC/cTnT, and total cTnITC/cTnT. The ratios of large-size cTnITC/total complex, large-size cTnITC/cTnT, and total cTnITC/cTnT were lower in patients with chronic cardiac events (including patients with cardiomyopathy or chronic heart failure and pneumonia), and higher in patients with acute myocardial infarction (Type 1); while the ratio of cTnT/total complex was higher in patients with chronic cardiac events, and lower in patients with acute myocardial infarction (Type 1).

The correlation between the composition of troponin complexes and fragments and the disease types indicated that the specific identification of different troponin fragments could be used to diagnose and distinguish acute myocardial infarction (Type 1) from chronic cardiac events.

### 3. Diagnostic performance of concentration characteristic parameters and concentration ratio characteristic parameters in distinguishing acute myocardial infarction (Type 1) from chronic cardiac events

Table 4-1-3 showed the diagnostic performance of the troponin complex and fragment markers used. In this example, the patient with acute myocardial infarction (Type 1) or chronic cardiac events, and the chronic cardiac event patients with cardiomyopathy, chronic heart failure or pneumonia. In order to facilitate the ratio analysis between complexes, the concentration of total complexes in the patient samples enrolled in the analysis was higher than 0.1 pmol/L.

Among them, the total complex concentration, large-size cTnITC concentration, total cTnITC concentration, cTnT concentration, cTnI concentration, ratio of large-size cTnITC/total complex, ratio of total cTnITC/total complex, ratio of cTnT/total complex, ratio of large-size cTnITC/cTnT, ratio of total cTnITC/cTnT, and ratio of large-size cTnITC/cTnI all had *P* values less than 0.05, indicating that the variables showed statistically significant differences. The area under curve (AUC) of the large-size cTnITC concentration was 0.929, the area under curve (AUC) of the total cTnITC concentration was 0.869, and the area under curve (AUC) of the ratio of large-size cTnITC/cTnT was 0.870, indicating good diagnostic performance. The values obtained from the cTnI kit were comparable to those of the total complex, indicating clinical equivalence between the two. The results showed that compared with the concentration of troponin total complex, cTnT or cTnI alone, using the concentration of large-size cTnITC or total cTnITC alone, or using the ratio of large-size cTnITC to cTnT could more effectively distinguish acute myocardial infarction (Type 1) from chronic cardiac events.

**Table 4-1-3: Diagnostic performance of concentration characteristic parameters and concentration ratio characteristic parameters in distinguishing acute myocardial infarction (Type 1) from chronic cardiac events**

| Characteristic parameter | ROC_AUC | Standard error | *P* | 95% CI lower limit | 95% CI upper limit |
|---|---|---|---|---|---|
| Total complex concentration | 0.850 | 0.055 | 0.000 | 0.743 | 0.958 |
| Large-size cTnITC concentration | 0.929 | 0.026 | 0.000 | 0.877 | 0.980 |
| Total cTnITC concentration | 0.869 | 0.048 | 0.000 | 0.776 | 0.962 |
| cTnT concentration | 0.850 | 0.053 | 0.000 | 0.745 | 0.954 |
| cTnI concentration | 0.849 | 0.056 | 0.000 | 0.739 | 0.959 |
| Large-size cTnITC/total complex | 0.782 | 0.060 | 0.000 | 0.664 | 0.899 |
| Total cTnITC/total complex | 0.749 | 0.071 | 0.000 | 0.610 | 0.887 |
| cTnT/total complex | 0.806 | 0.053 | 0.000 | 0.702 | 0.911 |
| Large-size cTnITC/cTnT | 0.870 | 0.043 | 0.000 | 0.785 | 0.955 |
| Total cTnITC/cTnT | 0.837 | 0.071 | 0.000 | 0.698 | 0.976 |
| Large-size cTnITC/cTnI | 0.755 | 0.063 | 0.000 | 0.633 | 0.878 |

Youden indexes was calculated using the sensitivity and specificity corresponding to different cutoff values of the characteristic parameters, and the optimal diagnostic CUTOFF values were determined based on the maximum values of the Youden indexes (Table 4-1-4). The CUTOFF value of total complex concentration was 247.7, the CUTOFF value of large-size cTnITC concentration was 1.9, and the CUTOFF value of total cTnITC concentration was 13.6; the CUTOFF value of cTnT concentration was 754.7; the CUTOFF value of cTnI concentration was 278.8; the CUTOFF value of large-size cTnITC/total complex was 0.0498; the CUTOFF value of total cTnITC/total complex was 0.1779; the CUTOFF value of cTnT/total complex was 3.4450; the CUTOFF value of large-size cTnITC/cTnT was 0.0165; the CUTOFF value of total cTnITC/cTnT was 0.0365; the CUTOFF value of large-size cTnITC/cTnI was 0.0705.

**Table 4-1-4: CUTOFF values and corresponding sensitivity and specificity of concentration and concentration ratio characteristic parameters for distinguishing acute myocardial infarction (Type 1) from chronic cardiac events**

| Characteristic parameter | CUTOFF | Sensitivity | Specificity | Maximum Youden index |
|---|---|---|---|---|
| Total complex concentration | 247.7 | 0.792 | 0.906 | 0.698 |
| Large-size cTnITC concentration | 1.9 | 0.958 | 0.750 | 0.708 |
| Total cTnITC concentration | 13.6 | 0.750 | 0.906 | 0.656 |
| cTnT concentration | 754.7 | 0.792 | 0.896 | 0.688 |
| cTnI concentration | 278.8 | 0.792 | 0.906 | 0.698 |
| Large-size cTnITC/total complex | 0.0498 | 0.833 | 0.740 | 0.573 |
| Total cTnITC/total complex | 0.1779 | 0.792 | 0.719 | 0.510 |
| cTnT/total complex | 3.4450 | 0.802 | 0.792 | 0.594 |
| Large-size cTnITC/cTnT | 0.0165 | 0.833 | 0.854 | 0.688 |
| Total cTnITC/cTnT | 0.0365 | 0.833 | 0.906 | 0.740 |
| Large-size cTnITC/cTnI | 0.0705 | 0.625 | 0.885 | 0.510 |

### 4. Diagnostic performance of multi-parameter conjoint analysis of concentration characteristic parameters in distinguishing acute myocardial infarction (Type 1) from chronic cardiac events

Diagnostic performance of multi-parameter conjoint analysis of concentration characteristic parameters in distinguishing acute myocardial infarction (Type 1) from chronic cardiac events was studied, in which the characteristic parameters included the concentrations of troponin complexes and fragments, namely, total complex concentration, large-size cTnITC concentration, total cTnITC concentration, cTnT concentration, and cTnI concentration. In this example, the patient with acute myocardial infarction (Type 1) or chronic cardiac events, and the chronic cardiac event patient with cardiomyopathy, chronic heart failure, or pneumonia, and the total complex concentrations in the patient samples were higher than 0.1 pmol/L.

The large-size cTnITC concentration + cTnT concentration were selected as the characteristic variables for conjoint analysis, and the combined prediction parameter was constructed using the logistic regression algorithm. The coefficient for each characteristic variable was estimated by logistic regression. The prediction value calculation formula was output. Logit(P) = 0.0484*(large-size cTnITC concentration) - 0.0003*(cTnT concentration) - 2.3441. The calculation method of prediction probability (Prediction Parameter **1**) was: $P = 1 / \left(1+{e}^{- logit}\right) * 100 % .$

The large-size cTnITC concentration + total complex concentration were selected as characteristic variables for conjoint analysis, the combined prediction parameter was constructed using the logistic regression algorithm, and the coefficient for each characteristic variable was estimated by logistic regression. The prediction value calculation formula was output. Logit(P) = 0.0266*(large-size cTnITC concentration) - 0.00004*(total complex concentration) - 2.3382. The calculation method of prediction probability (Prediction Parameter **2**) was: P=1/(1+e^{-logit})*100%.

The large-size cTnITC concentration + total cTnITC concentration were selected as characteristic variables for conjoint analysis, the combined prediction parameter was constructed using the logistic regression algorithm, and the coefficient for each characteristic variable was estimated by logistic regression. The prediction value calculation formula was output. Logit(P) = 0.0315*(large-size cTnITC concentration) - 0.0016*(total cTnITC concentration) - 2.3538. The calculation method of prediction probability (Prediction Parameter **3**) was: P=1/(1+e^{-logit})*100%.

The large-size cTnITC concentration + total cTnITC concentration + total complex concentration + cTnT concentration were selected as characteristic variables for conjoint analysis, and the combined prediction parameter was constructed using the logistic regression algorithm. The coefficient for each characteristic variable was estimated by logistic regression. The prediction value calculation formula was output. Logit(P) = 0.0523*(large-size cTnITC concentration) + 0.0221*(total cTnITC concentration) + 0.0001*(total complex concentration) - 0.0019*(cTnT concentration) - 2.0412. The calculation method of prediction probability (Prediction Parameter **4**) was: P=1/(1+e^{-logit})*100%

Among them, the area under curve (AUC) values of Prediction Parameter **1**, Prediction Parameter **2**, Prediction Parameter **3**, and Prediction Parameter **4** were 0.870, 0.928, 0.845, and 0.960, respectively, indicating good diagnostic performance (Table 4-1-5). The optimal CUTOFF values determined by the maximum values of Youden indexes were 0.1150, 0.0920, 0.1828, and 0.1077, respectively (Table 4-1-6).

**Table 4-1-5: Diagnostic performance of multi-parameter conjoint analysis of troponin concentration characteristic parameters in distinguishing acute myocardial infarction (Type 1) from chronic cardiac events**

| Characteristic parameter | ROC_AUC | Standard error | *P* | 95% CI lower limit | 95% CI upper limit |
|---|---|---|---|---|---|
| Prediction Parameter 1 | 0.870 | 0.056 | 0.000 | 0.761 | 0.980 |
| Prediction parameter 2 | 0.928 | 0.026 | 0.000 | 0.877 | 0.979 |
| Prediction parameter 3 | 0.845 | 0.061 | 0.000 | 0.726 | 0.964 |
| Prediction parameter 4 | 0.960 | 0.017 | 0.000 | 0.926 | 0.993 |

**Table 4-1-6: Sensitivity and specificity corresponding to the cutoff values of multi-parameter conjoint analysis of troponin concentration characteristic parameters**

| Marker parameter | CUTOFF | Sensitivity | Specificity | Maximum Youden index |
|---|---|---|---|---|
| Prediction Parameter 1 | 0.1150 | 0.792 | 0.917 | 0.708 |
| Prediction parameter 2 | 0.0920 | 0.958 | 0.750 | 0.708 |
| Prediction parameter 3 | 0.1828 | 0.708 | 0.958 | 0.667 |
| Prediction parameter 4 | 0.1077 | 0.958 | 0.875 | 0.833 |

In addition, other combinations included: total cTnITC concentration + total complex concentration, total cTnITC concentration + cTnT concentration, large-size cTnITC concentration + total cTnITC concentration + total complex concentration, large-size cTnITC concentration + total cTnITC concentration + cTnT concentration, large-size cTnITC concentration + total complex concentration + cTnT concentration, total cTnITC concentration + total complex concentration + cTnT concentration, and they all had good diagnostic performance when used in conjoint analysis according to the above method in distinguishing acute myocardial infarction (Type 1) from chronic cardiac events. The results showed that the large-size cTnITC concentration or total cTnITC concentration combined with other troponin fragment concentrations had good performance in distinguishing acute myocardial infarction (Type 1) from chronic cardiac events.

### 6. Diagnostic performance of multi-parameter conjoint analysis of concentration ratio characteristic parameters in distinguishing acute myocardial infarction (Type 1) from chronic cardiac events

Diagnostic performance of multi-parameter conjoint analysis of concentration ratio characteristic parameters in distinguishing acute myocardial infarction (Type 1) from chronic cardiac events was studied, in which the concentration ratio characteristic parameters included the ratio of troponin complexes and fragments to troponin total complexes, cTnT or cTnI, that is, large-size cTnITC/total complexes, total cTnITC/total complexes, cTnT/total complexes, large-size cTnITC/cTnT, total cTnITC/cTnT, large-size cTnITC/cTnI, and total cTnITC/cTnI. In this example, the patient with acute myocardial infarction (Type 1) or chronic cardiac events, and the patient with chronic cardiac events with cardiomyopathy, chronic heart failure or pneumonia, and the concentrations of total complex in the patient samples were higher than 0.1 pmol/L.

The large-size cTnITC/total complex + total cTnITC/total complex + cTnT/total complex were selected as characteristic variables for conjoint analysis, and the combined prediction parameter was constructed using the logistic regression algorithm. The coefficient for each characteristic variable was estimated by logistic regression. The prediction value calculation formula was output. Logit(P) = 10.532*(large-size cTnITC/total complex) + 24.346*(total cTnITC/total complex) - 1.076*(cTnT/total complex) - 1.099. The calculation method of prediction probability (Prediction Parameter **5**) was: P=1/(1+e^{-logit})* 100%.

The large-size cTnITC/cTnT and total cTnITC/cTnT were selected as characteristic variables for conjoint analysis, and the combined prediction parameter was constructed using the logistic regression algorithm. The coefficient for each characteristic variable was estimated by logistic regression. The prediction value calculation formula was output. Logit(P) = 16.149*(large-size cTnITC/cTnT) + 62.770*(total cTnITC/cTnT) - 4.041. The calculation method of prediction probability (Prediction Parameter 6) was: P=1/(1+e^{-logit})*100%.

Among them, the area under curve (AUC) values of Prediction Parameter **5** and Prediction Parameter **6** were 0.961 and 0.892, respectively, indicating good diagnostic performance (Table 4-1-7). The optimal CUTOFF values determined based on the maximum values of the Youden indexes were 0.1562 and 0.2286, respectively (Table 4-1-8).

**Table 4-1-7: Diagnostic performance of multi-parameter conjoint analysis of concentration ratios in distinguishing acute myocardial infarction (Type 1) from chronic cardiac events**

| Marker parameter | ROC_AUC | Standard error | *P* | 95% CI lower limit | 95% CI upper limit |
|---|---|---|---|---|---|
| Prediction parameter 5 | 0.961 | 0.018 | 0.000 | 0.927 | 0.996 |
| Prediction parameter 6 | 0.892 | 0.047 | 0.000 | 0.800 | 0.983 |

**Table 4-1-8: Sensitivity and specificity corresponding to the cutoff values of multi-parameter conjoint analysis of troponin complex and fragment ratios**

| Characteristic parameter | CUTOFF | Sensitivity | Specificity | Maximum Youden index |
|---|---|---|---|---|
| Prediction parameter 5 | 0.1562 | 0.917 | 0.854 | 0.771 |
| Prediction parameter 6 | 0.2286 | 0.792 | 0.969 | 0.760 |

In addition, other combinations included large-size cTnITC/total complex + total cTnITC/total complex, large-size cTnITC/total complex + cTnT/total complex, total cTnITC/total complex + cTnT/total complex, large-size cTnITC/total complex + total cTnITC/total complex + large-size cTnITC/cTnT, large-size cTnITC/total complex + total cTnITC/total complex + total cTnITC/cTnT, and they all had good diagnostic performance when used in conjoint analysis according to the above method in distinguishing acute myocardial infarction (Type 1) from chronic cardiac events. The results showed that the conjoint analysis of the combination of the ratio of large-size cTnITC to other troponin fragments and the ratio of total cTnITC to other troponin fragments, as well as the combination of the ratio between at least one of the two to other troponin fragments, had good performance in distinguishing acute myocardial infarction (Type 1) from chronic cardiac events.

The above data showed that the troponin large-size cTnITC complex and total cTnITC complex alone could be used to distinguish acute myocardial infarction (Type 1) from chronic cardiac events. The ratio of large-size cTnITC complex or total cTnITC complex to total complex, and the ratio of large-size cTnITC concentration or total cTnITC concentration to cTnT concentration could also be used to distinguish acute myocardial infarction (Type 1) from chronic cardiac events. The combined use of multiple marker concentrations, such as troponin large-size cTnITC complex concentration + total complex concentration, large-size cTnITC concentration + cTnT concentration, large-size cTnITC concentration + total cTnITC concentration, or large-size cTnITC concentration + total cTnITC concentration + total complex concentration + cTnT concentration, or the combined use of multiple marker ratios, such as large-size cTnITC/total complex, total cTnITC/total complex, cTnT/total complex, or the combined use of large-size cTnITC concentration/cTnT concentration, total cTnITC concentration/cTnT concentration, could further improve the diagnostic performance.

### Example 4-2. Distinction between acute myocardial infarction (Type 1) and chronic cardiac events (patients under observation)

### 1. Patient enrollment

Patients who had their first blood collected at admission time and whose troponin I values were between the 99^{th} percentile and 5 times the 99^{th} percentile were enrolled. According to the high-sensitivity cardiac troponin 0-3 h rapid diagnostic process, these patients could not be enrolled or excluded, and were called patients under observation. A total of 84 patients were enrolled, including 9 patients with Type 1 acute myocardial infarction who had symptom onset time less than 72 hours, and 75 patients with other chronic cardiac events.

### Patient information was shown in Table 4-2-1.

**Table 4-2-1: Characteristics of enrolled patients.**

| Total number of patients | 84 |
|---|---|
| Age | 64 (56 - 73) |
| Male | 51 (61%) |
| Creatinine, mmol/L | 91.5 (74.8 - 111.0) |
| Glomerular filtration rate, eGFR (mL/min/1.73 m²) | 70.5 (49.8 - 93.0) |
| Acute myocardial infarction (Type 1) | 9 (11%) |
| Chronic cardiac events | 75 (89 %) |

| | |
|---|---|
| Note: Continuous variables were presented as medians (25%-75% quartiles); categorical variables were presented as counts (percentages). | |

After the samples were enrolled, the troponin markers in the samples, including troponin total complex, large-size cTnITC, total cTnITC, and cTnT, were detected using a Mindray chemiluminescence analyzer and supporting reagents. The differences in troponin composition between patients with acute myocardial infarction (Type 1) and patients with chronic cardiac events were analyzed. The concentrations of total complex, large-size cTnITC, total cTnITC and cTnT in patient blood were shown in Table 4-2-2.

**Table 4-2-2: Values of concentration or concentration ratio characteristic parameters in patients with acute myocardial infarction (Type 1) and chronic cardiac events**

| Marker | Acute myocardial infarction (Type 1) | Chronic cardiac events |
|---|---|---|
| Total complex (10-2 pmol/L) | 63.5 (32.0 - 91.2) | 47.5 (32.8 - 60.3) |
| Large-size cTnITC (10-2 pmol/L) | 5.4 (2.7 - 11.9) | 1.2 (0.7 - 1.8) |
| Total cTnITC (10-2 pmol/L) | 8.3 (4.4 - 12.4) | 4.5 (1.7 - 6.7) |
| cTnT (10-2 pmol/L) | 197.3 (150.8 - 288.8) | 301.7 (202.3 - 483.1) |
| Large-size cTnITC/total complex (%) | 10.9 (4.6 - 21.2) | 2.7 (1.5 - 4.4) |
| Total cTnITC/total complex (%) | 12.3 (9.7 - 21.5) | 9.8 (4.9 - 16.4) |
| cTnT/total complex (%) | 350.6 (259.2 - 484.1) | 718.5 (437.7 - 1119.8) |
| Large-size cTnITC/cTnT (%) | 2.8 (1.3 - 6.2) | 0.3 (0.2 - 0.8) |
| Large-size cTnITC/cTnT (%) | 3.2 (2.3 - 6.0) | 1.2 (0.6 - 2.4) |

| | | |
|---|---|---|
| Note: Values were shown as medians (25%-75% interquartile ranges). | | |

Among them, the ratio of large-size cTnITC concentration to total complex concentration, the ratio of large-size cTnITC concentration to cTnT concentration, and the ratio of total cTnITC concentration to cTnT concentration were lower in patients with chronic cardiac events and higher in patients with acute myocardial infarction (Type 1); while the ratio of cTnT/total complex was higher in patients with chronic cardiac events and lower in patients with acute myocardial infarction (Type 1). The correlation between troponin complex and fragment composition and disease type suggested that specific identification of different troponin fragments could be used to distinguish acute myocardial infarction (Type 1) from chronic cardiac events in patients with troponin I values between the 99^{th} percentile and 5 times the 99^{th} percentile.

Table 4-2-3 showed the diagnostic performance of the concentration and concentration ratio characteristic parameters used in distinguishing acute myocardial infarction (Type 1) from chronic cardiac events in patients with troponin I values between the 99^{th} percentile and 5 times the 99^{th} percentile.

**Table 4-2-3: Diagnostic performance of concentration and concentration ratio characteristic parameters in distinguishing acute myocardial infarction (Type 1) from chronic cardiac events**

| Characteristic parameter | ROC_AUC | Standard error | *P* | 95% CI lower limit | 95% CI upper limit |
|---|---|---|---|---|---|
| Total complex concentration | 0.623 | 0.116 | 0.232 | 0.395 | 0.850 |
| Large-size cTnITC concentration | 0.842 | 0.086 | 0.001 | 0.674 | 1.000 |
| Total cTnITC concentration | 0.714 | 0.085 | 0.037 | 0.547 | 0.881 |
| cTnT concentration | 0.702 | 0.069 | 0.048 | 0.566 | 0.838 |
| Large-size cTnITC concentration/total complex concentration | 0.852 | 0.065 | 0.001 | 0.726 | 0.979 |
| Total cTnITC concentration/total complex concentration | 0.653 | 0.085 | 0.136 | 0.486 | 0.820 |
| cTnT concentration/total complex concentration | 0.799 | 0.064 | 0.004 | 0.673 | 0.924 |
| Large-size cTnITC concentration/cTnT concentration | 0.875 | 0.046 | 0.000 | 0.785 | 0.965 |
| Total cTnITC concentration/cTnT concentration | 0.842 | 0.059 | 0.001 | 0.726 | 0.957 |

Among them, the large-size cTnITC concentration, total cTnITC concentration, cTnT concentration, the ratio of large-size cTnITC concentration to total complex concentration, the ratio of cTnT concentration to total complex concentration, the ratio of large-size cTnITC concentration to cTnT concentration, and the ratio of total cTnITC concentration to cTnT concentration all had *P* values of less than 0.05, indicating that the variables showed statistically significant differences. Among them, the area under curve (AUC) of large-size cTnITC concentration was 0.842, the AUC of the ratio of large-size cTnITC concentration to total complex concentration was 0.852, the AUC of the ratio of large-size cTnITC concentration to cTnT concentration was 0.875, and the AUC of the ratio of total cTnITC concentration to cTnT concentration was 0.842, indicating good diagnostic performance. The results showed that the use of large-size cTnITC concentration or the use of the ratio of large-size cTnITC concentration to total complex concentration, the ratio of large-size cTnITC concentration to cTnT concentration, and the ratio of total cTnITC concentration to cTnT concentration as characteristic parameters could more effectively distinguish acute myocardial infarction (Type 1) from chronic cardiac events.

The Youden indexes were calculated using the sensitivity and specificity corresponding to different cutoff values of the characteristic parameters, and the optimal diagnostic CUTOFF values were determined based on the maximum values of the Youden indexes, as shown in Table 4-2-4.

**Table 4-2-4: CUTOFF values and corresponding sensitivity and specificity of concentration and concentration ratio characteristic parameters for distinguishing acute myocardial infarction (Type 1) from chronic cardiac events**

| Characteristic parameter | CUTOFF | Sensitivity | Specificity | Maximum Youden index |
|---|---|---|---|---|
| Total complex concentration | 62.7900 | 0.667 | 0.795 | 0.461 |
| Large-size cTnITC concentration | 1.9350 | 0.889 | 0.795 | 0.683 |
| Total cTnITC concentration | 5.6600 | 0.778 | 0.658 | 0.435 |
| cTnT concentration | 295.0900 | 0.533 | 0.889 | 0.422 |
| Large-size cTnITC concentration/total complex concentration | 0.0505 | 0.778 | 0.836 | 0.613 |
| Total cTnITC concentration/total complex concentration | 0.1066 | 0.778 | 0.575 | 0.353 |
| cTnT concentration/total complex concentration | 5.1106 | 0.733 | 0.889 | 0.622 |
| Large-size cTnITC concentration/cTnT concentration | 0.0102 | 0.889 | 0.836 | 0.725 |
| Total cTnITC concentration/cTnT concentration | 0.029 | 0.778 | 0.836 | 0.613 |

The characteristic parameters obtained by combining the concentration characteristic parameters of multiple myocardial injury markers were analyzed to distinguish acute myocardial infarction (Type 1) from chronic cardiac events in patients with troponin I values between the 99^{th} percentile and 5 times the 99^{th} percentile. The concentration characteristic parameters of myocardial injury markers included the concentrations of troponin complexes and fragments, namely, total complex concentration, large-size cTnITC concentration, total cTnITC concentration, cTnT concentration, and cTnI concentration.

The large-size cTnITC concentration and cTnT concentration were selected as the characteristic variables for conjoint analysis, and the characteristic parameter was constructed using the logistic regression algorithm. The coefficient for each characteristic variable was estimated by logistic regression, and the calculation formula of the characteristic parameter was derived as follows: Logit(P) = 0.1593*(large-size cTnITC concentration) - 0.0052*(cTnT concentration) - 1.3890. The calculation method of the predicted probability (Characteristic Parameter **1**) was: P=1/(1+e^{-logit})*100%.

The large-size cTnITC concentration and total complex concentration were selected as the characteristic variables for conjoint analysis, and the characteristic parameter was constructed using the logistic regression algorithm. The coefficient for each characteristic variable was estimated by logistic regression, and the calculation formula of the characteristic parameter was derived as follows: Logit(P) = 0.1722*(large-size cTnITC concentration) + 0.0066*(total complex concentration) - 3.2247. The calculation method of the predicted probability (Characteristic Parameter **2**) was: P=1/(1+e^{-logit})*100%.

The large-size cTnITC concentration and total cTnITC concentration were selected as the characteristic variables for conjoint analysis, and the characteristic parameter was constructed using the logistic regression algorithm. The coefficient for each characteristic variable was estimated by logistic regression, and the calculation formula of the characteristic parameter was derived as follows: Logit(P) = 0.1761*(large-size cTnITC concentration) + 0.0608*(total cTnITC concentration) - 3.2829. The calculation method of the predicted probability (Characteristic Parameter **3**) was: P=1/(1+e^{-logit})*100%.

The large-size cTnITC concentration, total cTnITC concentration, total complex and cTnT concentration were selected as characteristic variables for conjoint analysis. The characteristic parameter was constructed using the logistic regression algorithm. The coefficient for each characteristic variable was estimated by logistic regression, and the calculation formula of the characteristic parameter was obtained was derived as follows: Logit(P) = 0.1450*(large-size cTnITC concentration) + 0.2588*(total cTnITC concentration) + 0.0086*(total complex concentration) - 0.0160*(cTnT concentration) -1.048. The calculation method of the predicted probability (Characteristic Parameter **4**) was: P=1/(1+e^{-logit})*100%.

Among them, the area under curve (AUC) values of Characteristic Parameter **1**, Characteristic Parameter **2**, Characteristic Parameter **3**, and Characteristic Parameter **4** were 0.880, 0.809, 0.803, and 0.899, respectively, indicating good diagnostic performance, as shown in Table 4-2-5. The optimal CUTOFF values for diagnosis were determined according to the maximum values of Youden Indexes, as shown in Table 4-2-6.

**Table 4-2-5: Diagnostic performance of conjoint analysis of multiple concentration characteristic parameters in distinguishing acute myocardial infarction (Type 1) from chronic cardiac events**

| Characteristic parameter | ROC_AUC | Standard error | *P* | 95% CI lower limit | 95% CI upper limit |
|---|---|---|---|---|---|
| Characteristic Parameter 1 | 0.880 | 0.043 | 0.000 | 0.796 | 0.964 |
| Characteristic Parameter 2 | 0.809 | 0.097 | 0.003 | 0.619 | 0.999 |
| Characteristic Parameter 3 | 0.803 | 0.091 | 0.003 | 0.625 | 0.981 |
| Characteristic Parameter 4 | 0.899 | 0.041 | 0.000 | 0.820 | 0.979 |

**Table 4-2-6: Sensitivity and specificity corresponding to the cutoff values of conjoint analysis of multiple concentration characteristic parameters**

| Characteristic parameter | CUTOFF | Sensitivity | Specificity | Maximum Youden index |
|---|---|---|---|---|
| Characteristic Parameter 1 | 0.1165 | 0.889 | 0.800 | 0.689 |
| Characteristic Parameter 2 | 0.082 | 0.889 | 0.760 | 0.649 |
| Characteristic Parameter 3 | 0.094 | 0.778 | 0.840 | 0.618 |
| Characteristic Parameter 4 | 0.071 | 1.000 | 0.733 | 0.733 |

In addition, other concentration parameter combinations included: total cTnITC concentration + total complex concentration, total cTnITC concentration + cTnT concentration, large-size cTnITC concentration + total cTnITC concentration + total complex concentration, large-size cTnITC concentration + total cTnITC concentration + cTnT concentration, large-size cTnITC concentration + total complex concentration + cTnT concentration, total cTnITC concentration + total complex concentration + cTnT concentration, and they all had good diagnostic performance when used in conjoint analysis according to the above method to distinguish acute myocardial infarction (Type 1) from chronic cardiac events. The results showed that the combination of large-size cTnITC concentration and total cTnITC concentration, or the combination of at least one of them with other troponin fragment concentrations, had good performance in distinguishing acute myocardial infarction (Type 1) from chronic cardiac events.

The diagnostic performance of using the characteristic parameter obtained by combining the ratio parameters of multiple myocardial injury markers to distinguish acute myocardial infarction (Type 1) from chronic cardiac events in patients with troponin I values between the 99^{th} percentile and 5 times the 99^{th} percentile was analyzed. The ratio parameters of myocardial injury markers included the ratio of large-size cTnITC concentration or total cTnITC concentration to total complex concentration, cTnT concentration or cTnI concentration, namely, large-size cTnITC concentration/total complex concentration, total cTnITC concentration/total complex concentration, cTnT concentration/total complex concentration, large-size cTnITC concentration/cTnT concentration, total cTnITC concentration/cTnT concentration, large-size cTnITC concentration/cTnI concentration, and total cTnITC concentration/cTnI concentration.

The large-size cTnITC concentration/total complex concentration, total cTnITC concentration/total complex concentration, and cTnT concentration/total complex concentration were selected as characteristic variables for conjoint analysis. The characteristic parameter was constructed using the logistic regression algorithm. The coefficient for each characteristic variable was estimated by logistic regression, and the calculation formula of the characteristic parameter was derived as follows: Logit(P) = 6.880*(large-size cTnITC concentration/total complex concentration) + 13.985*(total cTnITC concentration/total complex concentration) - 0.619*(cTnT concentration/total complex concentration) - 1.201. The calculation method of the predicted probability (Characteristic Parameter **5**) was: P=1/(1+e^{-logit})*100%.

The large-size cTnITC concentration/cTnT concentration and total cTnITC concentration/cTn concentration were selected as characteristic variables for conjoint analysis. The characteristic parameter was constructed using the logistic regression algorithm. The coefficient for each characteristic variable was estimated by logistic regression, and the prediction value calculation formula was derived as follows: Logit(P) = 30.283*(large-size cTnITC concentration/cTnT concentration) + 68.804*(total cTnITC concentration/cTnT concentration) - 4.519. The calculation method for the prediction probability (Characteristic Parameter **6**) was: P=1/(1+e^{-logit})*100%.

Among them, the area under curve (AUC) values of Characteristic Parameter **5** and Characteristic Parameter **6** were 0.875 and 0.854, respectively, indicating good diagnostic performance, as shown in Table 4-2-7. The optimal diagnostic CUTOFF values were determined according to the maximum values of the Youden indexes, as shown in Table 4-2-8.

**Table 4-2-7: Diagnostic performance of conjoint analysis of multiple ratio parameters in distinguishing acute myocardial infarction (Type 1) from chronic cardiac events**

| Characteristic parameter | ROC_AUC | Standard error | *P* | 95% CI lower limit | 95% CI upper limit |
|---|---|---|---|---|---|
| Characteristic Parameter 5 | 0.875 | 0.068 | 0.000 | 0.742 | 1.000 |
| Characteristic Parameter 6 | 0.854 | 0.065 | 0.001 | 0.727 | 0.981 |

**Table 4-2-8: Sensitivity and specificity corresponding to the cutoff values of the conjoint analysis of multiple ratio parameters**

| Characteristic parameter | CUTOFF | Sensitivity | Specificity | Maximum Youden index |
|---|---|---|---|---|
| Characteristic Parameter 5 | 0.1286 | 0.889 | 0.808 | 0.697 |
| Characteristic Parameter 6 | 0.1109 | 0.778 | 0.822 | 0.600 |

In addition, other ratio parameter combinations included: large-size cTnITC concentration/total complex concentration + total cTnITC concentration/total complex concentration, large-size cTnITC concentration/total complex concentration + cTnT concentration/total complex concentration, total cTnITC concentration/total complex concentration + cTnT concentration/total complex concentration, large-size cTnITC concentration/total complex concentration + total cTnITC concentration/total complex concentration + large-size cTnITC concentration/cTnT concentration, large-size cTnITC concentration/total complex concentration + total cTnITC concentration/total complex concentration + total cTnITC concentration/cTnT concentration, and large-size cTnITC concentration/total complex concentration + total cTnITC concentration/total complex concentration + total cTnITC concentration/cTnT concentration, and they all had good diagnostic performance when used in the conjoint analysis according to the above method to distinguish acute myocardial infarction (Type 1) from chronic cardiac events. The results showed that the combination of the ratio of large-size cTnITC to other troponin fragments and the ratio of total cTnITC to other troponin fragments, or the combination of at least one of the two with the ratio of other troponin fragments had good performance in distinguishing acute myocardial infarction (Type 1) from chronic cardiac events.

The above data showed that the large-size cTnITC concentration or total cTnITC concentration, especially large-size cTnITC concentration, could be used alone to distinguish acute myocardial infarction (Type 1) from chronic cardiac events in patients with troponin I values between the 99^{th} percentile and 5 times the 99^{th} percentile. In addition, the ratio of large-size cTnITC concentration or total cTnITC concentration to total complex concentration, the ratio of large-size cTnITC concentration or total cTnITC concentration to cTnT concentration could also be used to distinguish acute myocardial infarction (Type 1) from chronic cardiac events, especially the ratio of large-size cTnITC concentration or total cTnITC concentration to cTnT concentration or the ratio of large-size cTnITC concentration to total complex concentration had better diagnostic performance. The combined use of multiple parameters, such as the combined use of large-size cTnITC concentration + total complex concentration, large-size cTnITC concentration + cTnT concentration, large-size cTnITC concentration + total cTnITC concentration, or large-size cTnITC concentration + total cTnITC concentration + total complex concentration + cTnT concentration, or the combined use of large-size cTnITC concentration/total complex concentration, total cTnITC concentration/total complex concentration, cTnT concentration/total complex concentration, or the combined use of large-size cTnITC concentration/cTnT concentration, total cTnITC concentration/cTnT concentration, could further improve the diagnostic performance.

In view of the combination of Examples 4-1 and 4-2, Example 4-2 is a secondary analysis of patients with mildly elevated troponin among the patients enrolled in Example 4-1. In Example 4-2, the study found that for patients with mildly elevated troponin, the characteristic parameters obtained based on large-size cTnITC concentration and/or total cTnITC concentration had better diagnostic performance for acute myocardial infarction (Type 1) and chronic cardiac events. Preferably, the large-size cTnITC concentration or characteristic parameters obtained based thereon had better diagnostic and therapeutic performance. In addition, the ratio of total cTnITC concentration to cTnT concentration, or characteristic parameters obtained based thereon, also had good diagnostic and therapeutic performance.

### Example 5. Distinguishing myocardial injury due to invasive procedure from chronic cardiac events

### 1. Patient enrollment

### (1) Invasive procedure patient group:

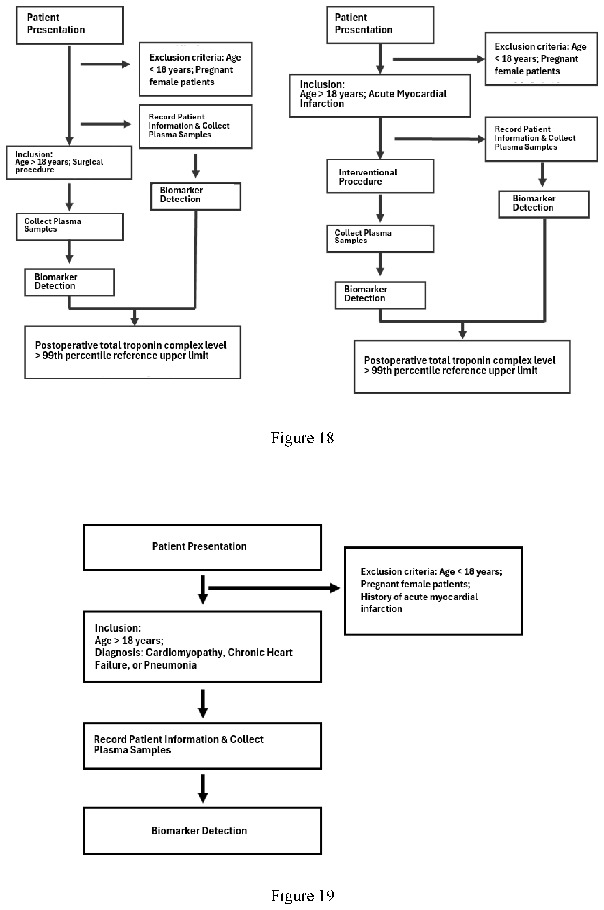

Patients who received invasive procedure were enrolled, and all were aged 18 years or older. Those who underwent coronary artery bypass grafting (CABG) or heart valve replacement were enrolled in the surgical group, and those who diagnosed with myocardial infarction and underwent percutaneous coronary intervention (PCI) were enrolled in the interventional surgery group. Patients under the age of 18 years and pregnant female patients were excluded. Lithium heparin plasma samples prior to invasive procedure and the first lithium heparin plasma samples after invasive procedure were collected for analysis. Patients with total complex levels elevated after surgery and higher than the sex-specific 99^{th} percentile upper reference limit (URL) were enrolled for the subsequent analysis. The patient enrollment process was shown in Figure 18.

### (2) Chronic cardiac event patient group:

Patients with confirmed admission diagnosis and final diagnosis of chronic heart failure, cardiomyopathy or pneumonia were enrolled, and all were aged 18 years or older. Patients with final diagnosis other than chronic heart failure, cardiomyopathy or pneumonia were excluded, as well as those also diagnosed with acute myocardial infarction. Patients under the age of 18 years and pregnant female patients were excluded. Cardiomyopathy, chronic heart failure, and pneumonia were independently diagnosed by hospital clinicians. Acute myocardial infarction was independently diagnosed by hospital cardiologists in accordance with its definition. The first lithium heparin plasma samples after admission of these patients were collected for the analysis of troponin complexes and fragment composition. The patient enrollment process was shown in Figure 19.

The patient information was recorded, including age, gender, past medical history, hypertension, diabetes mellitus, smoking status, creatinine level, and glomerular filtration rate. Samples that met the enrollment criteria were selected for detection of relevant markers. A total of 82 patients who had undergone invasive procedure were enrolled, including 48 patients who had undergone surgical operations and 34 patients who had undergone interventional treatment; and 145 patients with chronic cardiac events were enrolled. The patient information was shown in Table 5-1.

**Table 5-1: Characteristics of enrolled patients**

| Invasive treatment | Surgical procedure | Interventional procedure |
|---|---|---|
| Total number of patients | n= 48 | n= 34 |
| Age | 60 (53.3-66.0) | 62 (50.0-69.3) |
| Male | 32 (48%) | 27 (79%) |
| Recent smoking status | 7 (15%) | 9 (26%) |
| Hypertension | 28 (58%) | 21 (62%) |
| Diabetes mellitus | 7 (15%) | 11 (32%) |
| Creatinine, mmol/L | 81.5 (67.0-107.0) | 90.0 (77.8-1034.3) |
| Glomerular filtration rate, eGFR (mL/min/1.73 m²) | 82.0 (59.3-103.0) | 80.5 (66.3-93.0) |
| Invasive treatment method | CABG: 25 (52%) | PCI: 34 (100%) |
| Cardiac valve replacement: 23 | | |
| (48%) | | |
| Chronic cardiac events | Cardiomyopathy, chronic heart failure or pneumonia | |
| Total number of patients | n= 145 | |
| Age | 67 (57.0-75.0) | |
| Male | 92 (63%) | |
| Recent smoking status | 4 (3%) | |
| Hypertension | 80 (55%) | |
| Diabetes mellitus | 34 (23%) | |
| Creatinine, mmol/L | 86.0 (70.3-108.0) | |
| Glomerular filtration rate, eGFR (mL/min/1.73 m²) | 77.0 (56.8-95.3) | |

| | | |
|---|---|---|
| Note: Continuous variables were presented as medians (25%-75% quartiles); categorical variables were presented as counts (percentages) | | |

### 2. Analysis of troponin fragments and complexes in patients with myocardial injury due to invasive procedures and chronic cardiac events

After the samples were enrolled, the troponin markers in the samples, including total troponin complex, large-size cTnITC, total cTnITC and cTnT were detected using a Mindray chemiluminescence analyzer and supporting reagents. The differences in troponin composition between patients with myocardial injury due to invasive procedures and patients with chronic cardiac events were analyzed. The concentrations of total complex, large-size cTnITC, total cTnITC, cTnT and cTnI in patient blood were shown in Table 5-2. The measured values obtained from the cTnI kit were comparable to those of the total complex, indicating a certain degree of clinical equivalence between the two.

**Table 5-2: Concentrations of total complex, large-size cTnITC, total cTnITC, cTnT and cTnI in patients with myocardial injury due to invasive procedures and chronic cardiac events**

| Causes of myocardial injury | Surgical procedure | Interventional procedure | Chronic cardiac events |
|---|---|---|---|
| Total complex (10⁻² pmol/L) | 3466.3 747.1-9631.4) | 4801.3 (647.1-29650.8) | 17.8 (7.4-47.3) |
| Large-size cTnITC (10⁻² pmol/L) | 361.0 (67.1-9631.4) | 364.4 (15.1-5522.9) | 0.6 (0.2-1.3) |
| Total cTnITC (10⁻² pmol/L) | 1557.6 (270.6-4246.2) | 1086.7 (26.6-14518.6) | 2.9 (1.2-4.9) |
| cTnT (10⁻² pmol/L) | 4602.5 (1311.7-9188.2) | 6042.0 (954.7-36662.3) | 189.2 (102.2-339.7) |
| cTnI (10⁻² pmol/L) | 3570.3 (846.7-9450.8) | 4551.3 (637.3-33360.1) | 19.5 (8.0-55.3) |

| | | | |
|---|---|---|---|
| Note: Values were shown as medians (25%-75% interquartile ranges). | | | |

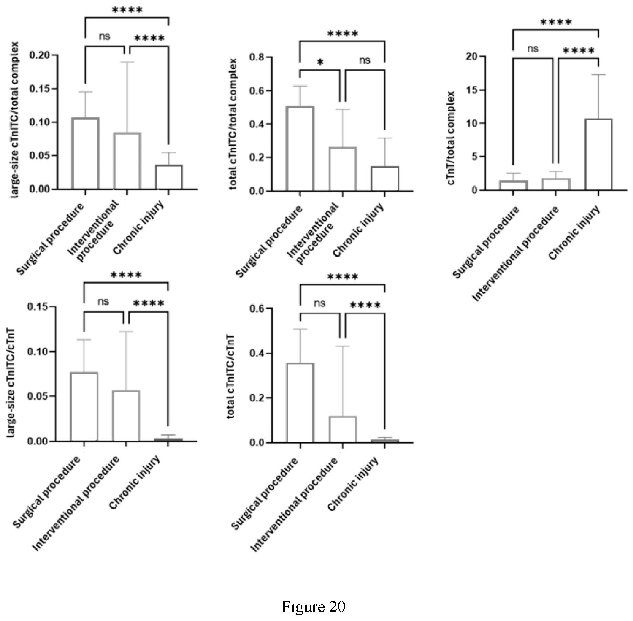

Figure 20 showed the relationship between ratios of troponin complexes and fragments in patients with myocardial injury due to invasive procedure and patients with chronic cardiac events, including the ratio of large-size cTnITC concentration to total complex concentration, the ratio of total cTnITC concentration to total complex concentration, the ratio of cTnT concentration to total complex concentration, the ratio of large-size cTnITC concentration to cTnT concentration, and the ratio of total cTnITC concentration to cTnT concentration. Among them, the ratio of large-size cTnITC concentration to total complex concentration, the ratio of total cTnITC concentration to total complex concentration, the ratio of large-size cTnITC concentration to cTnT concentration, and the ratio of total cTnITC concentration to cTnT concentration were higher in patients with myocardial injury due to invasive procedure, and lower in patients with chronic cardiac events; the ratio of cTnT concentration to total complex concentration was higher in patients with chronic cardiac events, and lower in patients with myocardial injury due to invasive procedure.

The concentrations of troponin complexes and fragments, and the ratio between them were related to the type of injury. Specific detection of different troponin complexes helped to distinguish and diagnose chronic cardiac events and myocardial injury due to invasive procedure.

### 3. Diagnostic performance of myocardial injury markers in distinguishing myocardial injury due to invasive procedures from chronic cardiac events

Table 5-3 showed the diagnostic performance of the myocardial injury markers used. In this example, patients had experienced myocardial injury due to invasive procedure, or had a chronic cardiac event, and those with chronic cardiac events suffered from cardiomyopathy, chronic heart failure or pneumonia, and the concentrations of troponin complex in the patents' samples were higher than the 99^{th} percentile upper reference limit.

Among them, the total complex concentration, large-size cTnITC concentration, total cTnITC concentration, cTnT concentration, cTnI concentration, the ratio of large-size cTnITC concentration to total complex concentration, the ratio of total cTnITC concentration to total complex concentration, the ratio of cTnT concentration to total complex concentration, the ratio of large-size cTnITC concentration to cTnT concentration, the ratio of total cTnITC concentration to cTnT concentration, and the ratio of large-size cTnITC concentration to cTnI concentration all had *P* values of less than 0.05, indicating that the variables showed statistically significant differences. The area under curve (AUC) of large-size cTnITC concentration was 0.952, the area under curve (AUC) of total cTnITC concentration was 0.940, and the AUC of the ratio of total cTnITC concentration to cTnT concentration was 0.913, which indicated that they had good diagnostic performance. The results showed that compared with the concentration of total complex, cTnT or cTnI alone, the characteristic parameter obtained based on the concentration of large-size cTnITC or total cTnITC could more effectively distinguish myocardial injury due to invasive procedures from chronic cardiac events, among which the concentration of large-size cTnITC or total cTnITC had good diagnostic and therapeutic efficacy.

**Table 5-3: Diagnostic performance of concentration and concentration ratio characteristic parameters in distinguishing myocardial injury due to invasive procedures from chronic cardiac events**

| Characteristic parameter | ROC_AUC | Standard error | *P* | 95% CI lower limit | 95% CI upper limit |
|---|---|---|---|---|---|
| Total complex concentration | 0.928 | 0.022 | 0.000 | 0.885 | 0.971 |
| Large-size cTnITC concentration | 0.952 | 0.015 | 0.000 | 0.922 | 0.982 |
| Total cTnITC concentration | 0.940 | 0.021 | 0.000 | 0.898 | 0.981 |
| cTnT concentration | 0.907 | 0.027 | 0.000 | 0.854 | 0.961 |
| cTnI concentration | 0.929 | 0.022 | 0.000 | 0.886 | 0.971 |
| Large-size cTnITC concentration/total complex concentration | 0.841 | 0.034 | 0.000 | 0.774 | 0.907 |
| Total cTnITC concentration/total complex concentration | 0.854 | 0.032 | 0.000 | 0.791 | 0.917 |
| cTnT concentration/total complex concentration | 0.828 | 0.038 | 0.000 | 0.753 | 0.903 |
| Large-size cTnITC concentration/cTnT concentration | 0.892 | 0.028 | 0.000 | 0.837 | 0.947 |
| Total cTnITC concentration/cTnT concentration | 0.913 | 0.025 | 0.000 | 0.864 | 0.962 |
| Large-size cTnITC concentration/cTnI concentration | 0.836 | 0.035 | 0.000 | 0.768 | 0.904 |

The Youden indexes were calculated using the sensitivity and specificity corresponding to different cutoff values of the characteristic parameters, and the optimal diagnostic CUTOFF values were determined based on the maximum values of the Youden indexes, as shown in Table 5-4.

**Table 5-4: Cutoff values and corresponding sensitivity and specificity of concentration and concentration ratio characteristic parameters for distinguishing myocardial injury due to invasive procedures from chronic cardiac events**

| Characteristic parameters | CUTOFF | Sensitivity | Specificity | Maximum Youden index |
|---|---|---|---|---|
| Total complex concentration | 256.2 | 0.914 | 0.855 | 0.768 |
| Large-size cTnITC concentration | 22.5 | 0.852 | 0.919 | 0.771 |
| Total cTnITC concentration | 35.4 | 0.877 | 0.968 | 0.844 |
| cTnT concentration | 951.1 | 0.827 | 0.903 | 0.730 |
| cTnI concentration | 292.6 | 0.914 | 0.855 | 0.768 |
| Large-size cTnITC concentration/total complex concentration | 0.0485 | 0.765 | 0.823 | 0.588 |
| Total cTnITC concentration/total complex concentration | 0.2145 | 0.765 | 0.919 | 0.685 |
| cTnT concentration/total complex concentration | 2.8950 | 0.774 | 0.841 | 0.616 |
| Large-size cTnITC concentration/cTnT concentration | 0.0245 | 0.815 | 0.871 | 0.686 |
| Total cTnITC concentration/cTnT concentration | 0.0455 | 0.827 | 0.968 | 0.795 |
| Large-size cTnITC concentration/cTnI concentration | 0.0505 | 0.741 | 0.855 | 0.596 |

### 4. Diagnostic performance of combination of concentration parameters of multiple myocardial injury markers in distinguishing the myocardial injury due to invasive procedures from chronic cardiac events

The concentration parameters of myocardial injury markers included the concentrations of troponin complexes and fragments, namely, total complex concentration, large-size cTnITC concentration, total cTnITC concentration, cTnT concentration, and cTnI concentration. In this example, the patients experienced myocardial injury due to invasive procedure, or suffered from chronic cardiac events. The patients with chronic cardiac event suffered from cardiomyopathy, chronic heart failure, or pneumonia, and the troponin complex concentrations of the patients' samples were higher than the 99^{th} percentile upper reference limit.

The large-size cTnITC concentration and cTnT concentration were selected as characteristic variables for conjoint analysis, and the characteristic parameter was constructed using the logistic regression algorithm. The coefficient for each characteristic variable was estimated by logistic regression, and the calculation formula of the characteristic parameter was derived as follows: Logit(P) = 0.0417*(large-size cTnITC concentration) - 0.0001*(cTnT concentration) - 1.346. The calculation method of the predicted probability (Characteristic Parameter **1**) was: $P = 1 / \left(1+{e}^{- logit}\right) * 100 % .$

The large-size cTnITC concentration and the total complex concentration were selected as the characteristic variables for conjoint analysis, and the characteristic parameter was constructed using the logistic regression algorithm. The coefficient for each characteristic variable was estimated by logistic regression, and the calculation formula of the characteristic parameter was derived as follows: Logit(P) = 0.0522*(large-size cTnITC concentration) - 0.0009*(total complex concentration) - 1.2682. The calculation method of the predicted probability (Characteristic Parameter **2**) was: P=1/(1+e^{-logit})*100%.

The large-size cTnITC concentration and total cTnITC concentration were selected as characteristic variables for conjoint analysis. The characteristic parameter was constructed using the logistic regression algorithm. The coefficient for each characteristic variable was estimated by logistic regression, and the calculation formula of the characteristic parameter was derived as follows: Logit(P) = 0.0217*(large-size cTnITC concentration) + 0.0040*(total cTnITC concentration) - 1.3428. The calculation method of the predicted probability (Characteristic Parameter **3**) was: P=1/(1+e^{-logit})*100%.

The large-size cTnITC concentration and total cTnITC concentration + total complex concentration + cTnT concentration were selected as the characteristic variables for conjoint analysis. The characteristic parameter was constructed using the logistic regression algorithm. The coefficient for each characteristic variable was estimated by logistic regression, and the calculation formula of the characteristic parameter was obtained was derived as follows: Logit(P) = 0.0375*(large-size cTnITC concentration) + 0.0053*(total cTnITC concentration) - 0.0007*(total complex concentration) - 0.0001*(cTnT concentration) - 1.3256. The calculation method of the predicted probability (Characteristic Parameter **4**) was: P=1/(1+e^{-logit})*100%.

Among them, the area under curve (AUC) of Characteristic Parameter **1**, Characteristic Parameter **2**, Characteristic Parameter **3**, and Characteristic Parameter **4** were 0.940, 0.921, 0.955, and 0.937, respectively, indicating good diagnostic performance, as shown in Table 5-5. The optimal CUTOFF values determined based on the maximum values of the Youden indexes were 0.4163, 0.3081, 0.3169, and 0.2827, respectively, as shown in Table 5-6.

**Table 5-5: Diagnostic performance of conjoint analysis of multiple concentration parameters in distinguishing myocardial injury due to invasive procedures from chronic cardiac events**

| Characteristic parameter | ROC_AUC | Standard error | *P* | 95%CI lower limit | 95% CI upper limit |
|---|---|---|---|---|---|
| Characteristic Parameter 1 | 0.940 | 0.018 | 0.000 | 0.905 | 0.975 |
| Characteristic Parameter 2 | 0.921 | 0.024 | 0.000 | 0.874 | 0.968 |
| Characteristic Parameter 3 | 0.955 | 0.016 | 0.000 | 0.923 | 0.986 |
| Characteristic Parameter 4 | 0.937 | 0.021 | 0.000 | 0.896 | 0.979 |

**Table 5-6: Sensitivity and specificity corresponding to the cutoff values of conjoint analysis of multiple concentration parameters**

| Characteristic parameter | CUTOFF | Sensitivity | Specificity | Maximum Youden index |
|---|---|---|---|---|
| Characteristic Parameter 1 | 0.4163 | 0.817 | 0.952 | 0.769 |
| Characteristic Parameter 2 | 0.3081 | 0.866 | 0.903 | 0.769 |
| Characteristic Parameter 3 | 0.3169 | 0.878 | 0.935 | 0.814 |
| Characteristic Parameter 4 | 0.2827 | 0.890 | 0.903 | 0.793 |

In addition, other concentration parameter combinations included: total cTnITC concentration + total complex concentration, total cTnITC concentration + cTnT concentration, large-size cTnITC concentration + total cTnITC concentration + total complex concentration, large-size cTnITC concentration + total cTnITC concentration +cTnT concentration, large-size cTnITC concentration + total complex concentration + cTnT concentration, total cTnITC concentration + total complex concentration + cTnT concentration, and they all had good diagnostic performance when used in the conjoint analysis according to the above method to distinguish myocardial injury due to invasive procedures from chronic cardiac events. The results showed that the large-size cTnITC concentration or total cTnITC concentration combined with other troponin fragment concentrations had good performance in distinguishing myocardial injury due to invasive procedures from chronic cardiac events.

### 5. Diagnostic performance of combination of ratio parameters of multiple myocardial injury markers in distinguishing myocardial injury due to invasive procedures from chronic cardiac events

The ratio parameters of myocardial injury markers included the ratio of large-size cTnITC concentration to total complex, the ratio of total cTnITC concentration to total complex concentration, the ratio of cTnT concentration to total complex concentration, the ratio of large-size cTnITC concentration to cTnT concentration, the ratio of total cTnITC concentration to cTnT concentration, the ratio of large-size cTnITC concentration to cTnI concentration, and the ratio of total cTnITC concentration to cTnI concentration. In this example, the patients experienced myocardial injury due to invasive procedure, or suffered from chronic cardiac events. The patients with chronic cardiac events suffered from cardiomyopathy, chronic heart failure or pneumonia, and the concentrations of troponin complex in the patients' sample were higher than the 99^{th} percentile upper reference limit.

The large-size cTnITC concentration/total complex concentration, total cTnITC concentration/total complex concentration, and cTnT concentration/total complex concentration were selected as characteristic variables for conjoint analysis. The characteristic parameter was constructed using the logistic regression algorithm. The coefficient for each characteristic variable was estimated by logistic regression, and the calculation formula of the characteristic parameter was derived as follows: Logit(P) = 4.775*(large-size cTnITC concentration/total complex concentration) + 10.015*(total cTnITC concentration/total complex concentration) - 0.413*(cTnT concentration/total complex concentration) - 0.251. The calculation method of the prediction probability (Characteristic Parameter **5**) was: P=1/(1+e^{-logit})*100%.

The large-size cTnITC concentration/cTnT concentration and total cTnITC concentration/cTnT concentration were selected as characteristic variables for conjoint analysis. The characteristic parameter was constructed using the logistic regression algorithm. The coefficient for each characteristic variable was estimated by logistic regression, and the calculation formula of the characteristic parameter was derived as follows: Logit(P) = 9.933*(large-size cTnITC concentration/cTnT concentration) + 40.395*(total cTnITC concentration/cTnT concentration) - 2.094. The calculation method of the predicted probability (Characteristic Parameter **6**) was: P=1/(1+e^{-logit})*100%.

Among them, the area under curve (AUC) of Characteristic Parameter **5** and Characteristic Parameter **6** were 0.926 and 0.921, respectively, indicating good diagnostic performance, as shown in Table 5-7. The optimal CUTOFF values determined by the maximum values of the Youden indexes were 0.6594 and 0.6053, respectively, as shown in Table 5-8.

**Table 5-7: Diagnostic performance of conjoint analysis of multiple ratio parameters in distinguishing myocardial injury due to invasive procedures from chronic cardiac events**

| Characteristic parameter | ROC_AUC | Standard error | *P* | 95% CI lower limit | 95% CI upper limit |
|---|---|---|---|---|---|
| Characteristic Parameter 5 | 0.926 | 0.023 | 0.000 | 0.882 | 0.971 |
| Characteristic Parameter 6 | 0.921 | 0.023 | 0.000 | 0.876 | 0.966 |

**Table 5-8: Sensitivity and specificity corresponding to the cutoff values of conjoint analysis of multiple ratio parameters**

| Characteristic parameter | CUTOFF | Sensitivity | Specificity | Maximum Youden index |
|---|---|---|---|---|
| Characteristic Parameter 5 | 0.6594 | 0.790 | 1.000 | 0.790 |
| Characteristic Parameter 6 | 0.6053 | 0.815 | 0.968 | 0.783 |

In addition, other ratio parameter combinations included: large-size cTnITC concentration/total complex concentration + total cTnITC concentration/total complex concentration, large-size cTnITC concentration/total complex concentration + cTnT concentration/total complex concentration, total cTnITC concentration/total complex concentration + cTnT concentration/total complex concentration, large-size cTnITC concentration/total complex concentration + total cTnITC concentration/total complex concentration + large-size cTnITC concentration/cTnT concentration, large-size cTnITC concentration/total complex concentration + total cTnITC concentration/total complex concentration + total cTnITC concentration/cTnT concentration, and they all had good diagnostic performance when used in conjoint analysis according to the above method to distinguish myocardial injury due to invasive procedures from chronic cardiac events. The results showed that the combination of the ratio of large-size cTnITC to other troponin fragments and the ratio of total cTnITC to other troponin fragments, as well as the combination of at least one of the two and the ratio between other troponin fragments, had good performance in distinguishing myocardial injury due to invasive procedures from chronic cardiac events.

The above data showed that the large-size cTnITC concentration or total cTnITC concentration can be used alone to distinguish myocardial injury due to invasive procedures from chronic cardiac events. The ratio of large-size cTnITC concentration or total cTnITC concentration to total complex concentration, the ratio of large-size cTnITC concentration or total cTnITC concentration to cTnT concentration, and the ratio of large-size cTnITC concentration or total cTnITC concentration to cTnI concentration could also be used to distinguish myocardial injury due to invasive procedures from chronic cardiac events. The combined use of multiple parameters, such as large-size cTnITC concentration + total complex concentration, large-size cTnITC concentration + cTnT concentration, large-size cTnITC concentration + total cTnITC concentration, or large-size cTnITC concentration + total cTnITC concentration + total complex concentration + cTnT concentration, or the combined use of large-size cTnITC concentration/total complex concentration, total cTnITC concentration/total complex concentration, cTnT concentration/total complex concentration, or the combined use of large-size cTnITC concentration/cTnT concentration, total cTnITC concentration/cTnT concentration, could also be used to distinguish myocardial injury due to invasive procedures from chronic cardiac events. Although not wishing to be bound by theory, it is possible that large-size cTnITC concentration or characteristic parameters obtained based thereon are more suitable for distinguishing myocardial injury due to invasive procedures from chronic cardiac events.

### Example 6. Assessment of prognosis of acute myocardial injury

This example was divided into the prognosis of acute myocardial injury in patients undergoing cardiac surgery and the prognosis of acute myocardial injury in patients with myocardial infarction. These two sections illustrate the application of the markers of the present application in the prognosis of acute myocardial injury.

### (I) Section of surgery

### 1. Patient enrollment and follow-up method

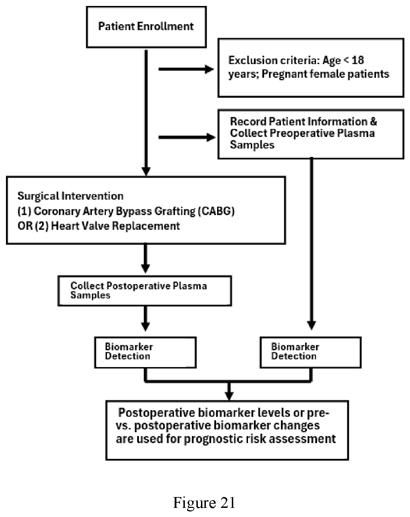

Patients who underwent cardiac surgery were enrolled, and all were aged 18 years or older. Cardiac surgery included (1) coronary artery bypass grafting (CABG), or (2) cardiac valve replacement. Patients under the age of 18 and pregnant female patients were excluded. Lithium heparin plasma samples prior to invasive procedure and lithium heparin plasma samples after invasive procedure (within 48 hours) were collected for analysis. The patient enrollment process was shown in Figure 21.

The patient information was recorded, including: age, gender, medical history, glomerular filtration rate, etc. The samples that met the enrollment criteria were collected for detection of relevant markers. A total of 311 patients who underwent cardiac surgery were enrolled in this study, including 133 patients who underwent coronary artery bypass grafting and 178 patients who underwent cardiac valve replacement. The patient information was shown in Table 6-1.

**Table 6-1: Characteristics of enrolled patients**

| Acute myocardial injury | Patients underwent surgery |
|---|---|
| Total number of patients | n= 311 |
| Age | 61 (55 - 68) |
| Male | 186 (60%) |
| Creatinine, mmol/L | 83 (66 - 104) |
| Glomerular filtration rate, eGFR (mL/min/1.73 m²) | 83 (63 - 94) |

### Continuous variables were presented as medians (25%-75% quartiles); categorical variables were presented as counts (percentages)

Patients were followed up for 3 months or 1 year in this study, and clinic visits, telephone follow-up and/or collection of electronic medical records may be performed to record information about clinical events. Major clinical events included composite events of all-cause mortality, myocardial infarction, and unplanned coronary revascularization. Minor clinical events included cardiovascular death, components of major clinical events, stroke, hospitalization for heart failure or emergency observation for 24 hours or more, cardiac arrest or malignant arrhythmia and other hospitalization events due to cardiovascular disease, as well as different combination composite endpoints of the above events.

### 3. Evaluation of the predictive ability of troponin fragments and complexes for prognostic risk in patients

In this study, a total of 311 patients who underwent cardiac surgery were enrolled, 133 of whom (43%) underwent coronary artery bypass grafting. Within 3 months after surgery, the total mortality rate was 0.6% (2/311), and the incidence of the composite endpoint of death and adverse cardiovascular events was 5.1% (16/311). Within 1 year after surgery, the total mortality rate was 1.0% (3/311), and the incidence of composite endpoint of death and adverse cardiovascular events was 7.7% (24/311).

The ROC curve analysis was used to evaluate the efficacy of troponin complex and fragment markers in predicting patients' prognostic risk (Table 6-2). The predicted event was the composite endpoint event of death and adverse cardiovascular events within 1 year after surgery. The variables included troponin total complex concentration, large-size cTnITC concentration, total cTnITC concentration, and cTnT concentration within 24 hours after surgery.

Among them, the total complex concentration, large-size cTnITC concentration, total cTnITC concentration, and cTnT concentration all had *P* values of less than 0.05, indicating that the variables showed statistically significant differences. Among them, the area under curve (AUC) of total complex concentration and large-size cTnITC absolute concentration was 0.765, and the area under curve (AUC) of total cTnITC absolute concentration was 0.771, indicating that they exhibited a certain predictive ability for adverse cardiovascular events and mortality within one year. The results showed that troponin total complex concentration, large-size cTnITC concentration, total cTnITC concentration, and cTnT concentration could assess the prognostic risk of patients.

**Table 6-2: Evaluation of the predictive ability of troponin complex and fragment markers for prognosis risk in patients**

| Marker parameter | ROC_AUC | Standard error | *P* | 95% CI lower limit | 95% CI upper limit |
|---|---|---|---|---|---|
| Total complex concentration | 0.765 | 0.040 | 0.000 | 0.687 | 0.843 |
| Large-size cTnITC concentration | 0.765 | 0.042 | 0.000 | 0.682 | 0.848 |
| Total cTnITC concentration | 0.771 | 0.041 | 0.000 | 0.691 | 0.852 |
| cTnT concentration | 0.776 | 0.041 | 0.000 | 0.696 | 0.856 |

The sensitivity and specificity corresponding to different CUTOFF values using the total troponin complex concentration, large-size cTnITC concentration, total cTnITC concentration, and cTnT concentration as markers were used to calculate the Youden indexes, so as to ensure high sensitivity and specificity. The optimal CUTOFF values were determined based on the maximum Youden indexes (Table 6-3). When the total complex concentration was higher than 6848.6 ng/L, the predicted sensitivity was 66.7% and the specificity was 69.3%; when the large-size cTnITC concentration was higher than 1001.0 ng/L, the predicted sensitivity was 71.4% and the specificity was 78.7%; when the total cTnITC concentration was higher than 1897.1 ng/L, the predicted sensitivity was 81.0% and the specificity was 65.5%; when the cTnT concentration was higher than 738.9 ng/L, the predicted sensitivity was 66.7% and the specificity was 76.0%.

**Table 6-3: Sensitivity and specificity corresponding to the cutoff values of troponin complex and fragment markers for predicting prognosis risk of patients**

| | ≥ CUTOFF (ng/L) | Sensitivity | Specificity | Youden index |
|---|---|---|---|---|
| Total complex concentration | 6848.6 | 0.667 | 0.693 | 0.360 |
| Large-size cTnITC concentration | 1001.0 | 0.714 | 0.787 | 0.502 |
| Total cTnITC concentration | 1897.1 | 0.810 | 0.655 | 0.465 |
| cTnT concentration | 738.9 | 0.667 | 0.760 | 0.426 |

In addition, the differences (postoperative concentration - preoperative concentration) and fold changes (postoperative concentration/preoperative concentration) in troponin fragments and complexes, comparing postoperative values with preoperative values, were calculated to predict patient's prognostic risk (Table 6-4). The variables included troponin total complex concentration (postoperative - preoperative), large-size cTnITC concentration (postoperative - preoperative), total cTnITC concentration (postoperative - preoperative), cTnT concentration (postoperative - preoperative), and total complex (postoperative/preoperative), large-size cTnITC (postoperative/preoperative), total cTnITC (postoperative/preoperative), and cTnT (postoperative/preoperative).

Among them, the postoperative-preoperative concentration differences in total complex, large-size cTnITC, total cTnITC, and cTnT, as well as the postoperative/preoperative concentration ratios in large-size cTnITC, total cTnITC, and cTnT all had the *P* values of less than 0.05, indicating that the variables showed statistically significant differences. Among them, the area under curve (AUC) of large-size cTnITC (postoperative-preoperative) was 0.759, the area under curve (AUC) of total cTnITC (postoperative-preoperative) was 0.774, the area under curve (AUC) of large-size cTnITC (postoperative/preoperative) was 0.663, and the area under curve (AUC) of total cTnITC (postoperative/preoperative) was 0.660, indicating that they had the ability to predict adverse cardiovascular events and morality within one year. The results showed that the postoperative-preoperative differences or fold changes in total complex, large-size cTnITC, total cTnITC, and cTnT could assess the prognostic risk in patients and predict the risk of myocardial injury in patients undergoing cardiac surgery.

**Table 6-4: Evaluation of the predictive ability of postoperative-preoperative concentration differences and fold changes of troponin complexes and fragments for prognostic risk of patients**

| Marker parameter | ROC_A UC | Standard error | *P* | 95% CI lower limit | 95% CI upper limit |
|---|---|---|---|---|---|
| Total complex (postoperative-preoperative) | 0.770 | 0.042 | 0.000 | 0.688 | 0.851 |
| Large-size cTnITC (postoperative-preoperative) | 0.759 | 0.046 | 0.000 | 0.668 | 0.850 |
| Total cTnITC (postoperative-preoperative) | 0.774 | 0.044 | 0.000 | 0.688 | 0.860 |
| cTnT (postoperative-preoperative) | 0.777 | 0.044 | 0.000 | 0.690 | 0.863 |
| Total complex (postoperative/preoperative) | 0.623 | 0.049 | 0.073 | 0.528 | 0.719 |
| Large-size cTnITC (postoperative/preoperative) | 0.663 | 0.054 | 0.018 | 0.557 | 0.768 |
| Total cTnITC (postoperative/preoperative) | 0.660 | 0.058 | 0.020 | 0.547 | 0.773 |
| cTnT (postoperative-preoperative) | 0.641 | 0.057 | 0.040 | 0.529 | 0.753 |

The sensitivity and specificity corresponding to different CUTOFF values using postoperative-preoperative concentration differences and fold changes of large-size cTnITC, total cTnITC and cTnT as parameters were used to calculate Youden indexes, so as to ensure higher sensitivity and specificity, and the optimal CUTOFF values were determined according to the maximum values of Youden indexes (Table 6-5). When the postoperative large-size cTnITC concentration increased by more than 1008.0 ng/L compared with the preoperative concentration, the predicted sensitivity was 68.4% and the specificity was 77.8%; when the total cTnITC concentration increased by more than 1896.6 ng/L, the predicted sensitivity was 84.2% and the specificity was 65.4%; when the postoperative large-size cTnITC concentration increased by more than 3320 times compared with the preoperative concentration, the predicted sensitivity was 63.2% and the specificity was 67.3%; when the total cTnITC concentration increased by more than 970 times, the predicted sensitivity was 63.2% and the specificity was 55.3%.

**Table 6-5: Sensitivity and specificity corresponding to the cutoff values of postoperative-preoperative concentration differences and fold changes of troponin complex and fragment for predicting the prognostic risk of patients**

| | ≥ CUTOFF | Sensitivity | Specificity | Youden Index |
|---|---|---|---|---|
| Total complex (postoperative-preoperative) | 6841.2 ng/L | 0.684 | 0.693 | 0.377 |
| Large-size cTnITC (postoperative-preoperative) | 1008.0 ng/L | 0.684 | 0.778 | 0.462 |
| Total cTnITC (postoperative-preoperative) | 1896.6 ng/L | 0.842 | 0.654 | 0.496 |
| cTnT (postoperative-preoperative) | 779.5 ng/L | 0.684 | 0.763 | 0.447 |
| Total complex (postoperative/preoperative) | 522 | 0.684 | 0.514 | 0.198 |
| Large-size cTnITC (postoperative/preoperative) | 3320 | 0.632 | 0.673 | 0.305 |
| Total cTnITC (postoperative/preoperative) | 970 | 0.632 | 0.553 | 0.184 |
| cTnT (postoperative/preoperative) | 35 | 0.632 | 0.514 | 0.145 |

### 4. Evaluation of predictive ability of conjoint analysis of troponin fragment concentrations for prognostic risk of patients

The conjoint analysis using multiple parameters of markers was used to predict prognostic risk of patients. The parameters included postoperative-preoperative concentration differences (postoperative concentration-preoperative concentration) and ratios (postoperative concentration/preoperative concentration) of troponin complexes and fragments.

Taking the large-size cTnITC concentration + cTnT concentration as an example, conjoint analysis was performed, and the combined prediction parameter was constructed using the logistic regression algorithm. The coefficient for each characteristic variable was estimated by logistic regression. The prediction value calculation formula was output. Logit(P) = 0.0002*(large-size cTnITC concentration) + 0.0002*(cTnT concentration) - 3.103. The calculation method of prediction probability (Prediction Parameter **1**) was: P=1/(1+e^{-logit})*100%.

Through ROC curve analysis, the *P* value of Prediction Parameter **1** was less than 0.05, indicating that the variable was significant. The area under curve (AUC) was 0.789, and the diagnostic performance was slightly improved compared with the use of a single marker (Table 6-6).

**Table 6-6: Conjoint analysis of multiple parameters of troponin absolute concentrations for predicting patient prognosis risk**

| Marker parameter | ROC_AUC | Standard error | *P* | 95% CI lower limit | 95% CI upper limit |
|---|---|---|---|---|---|
| Prediction Parameter 1 | 0.789 | 0.041 | 0.000 | 0.709 | 0.869 |

In addition, other combinations including large-size cTnITC + total complex, large-size cTnITC + total cTnITC, total cTnITC + total complex, total cTnITC + cTnT, large-size cTnITC + total cTnITC + total complex, large-size cTnITC + total cTnITC + cTnT, large-size cTnITC + total complex + cTnT, total cTnITC + total complex + cTnT, as well as postoperative-preoperative concentration differences or ratios were also used to evaluate prognosis risk of patients. The results showed that large-size cTnITC combined with other troponin fragments could be used to predict the prognosis risk of patients.

### 5. Risk of composite endpoint events in different troponin fragment concentration groups

Taking the postoperative troponin concentration value as an example, patients were stratified into different subgroups according to the postoperative troponin concentration: Group 1 "< median concentration", Group 2 "median concentration - 75^{th} percentile concentration", Group 3 "> 75^{th} percentile concentration". The corresponding subgroup concentrations for each marker were shown in Table 6-7.

**Table 6-7: Corresponding concentration ranges for different troponin fragment and complex concentration groups**

| Marker parameter | Group 1 | Group 2 | Group 3 |
|---|---|---|---|
| Total complex (ng/L) | ≤ 2964 | 2964 - 9605 | > 9605 |
| Large-size cTnITC (ng/L) | ≤ 371 | 371 - 997 | > 997 |
| Total cTnITC (ng/L) | ≤ 956 | 956 - 4220 | > 4220 |
| cTnT (ng/L) | ≤ 354 | 354 - 809 | > 809 |

Binary logistic regression analysis was used to analyze the risk of composite endpoint events in patients of different troponin fragment and complex concentration groups (Table 6-8). Binary logistic regression analysis was used to analyze the correlation between marker parameters and the occurrence of primary endpoint events. *P* value <0.05 was considered significant.

The analysis found that for the total complex group 2 and group 3, *P* values were less than 0.05, and OR values were 10.0 and 11.5, respectively; for the large-size cTnITC group 3, *P* value was less than 0.05, and OR value was 11.6; for the total cTnITC group 2 and group 3, *P* values were less than 0.05, and OR values were 8.8 and 12.1, respectively; for the cTnT group 2 and group 3, *P* values were less than 0.05, and OR values were 4.2 and 8.9, respectively; the above results showed that the total complex concentration higher than 2964 ng/L, the large-size cTnITC concentration higher than 997 ng/L, the total cTnITC concentration higher than 956 ng/L, and the cTnT concentration higher than 354 ng/L were risk factors for the occurrence of composite endpoint events in patients.

**Table 6-8: Risk of occurrence of composite endpoint events in different troponin fragment concentration groups**

| Marker parameter | Concentration group | P value | OR | 95% CI lower limit | 95% CI upper limit |
|---|---|---|---|---|---|
| Total complex | Group 1 | - | 1 | | |
| | Group 2 | 0.004 | 10.043 | 2.114 | 47.712 |
| | Group 3 | 0.002 | 11.493 | 2.451 | 53.879 |
| Large-size cTnITC | Group 1 | - | 1 | | |
| | Group 2 | 0.389 | 2.041 | 0.402 | 10.355 |
| | Group 3 | 0.000 | 11.615 | 3.251 | 41.495 |
| Total cTnITC | Group 1 | - | 1 | | |
| | Group 2 | 0.007 | 8.812 | 1.823 | 42.583 |
| | Group 3 | 0.001 | 12.116 | 2.615 | 56.137 |
| cTnT | Group 1 | - | 1 | | |
| | Group 2 | 0.045 | 4.254 | 1.034 | 17.497 |
| | Group 3 | 0.001 | 8.882 | 2.428 | 32.499 |

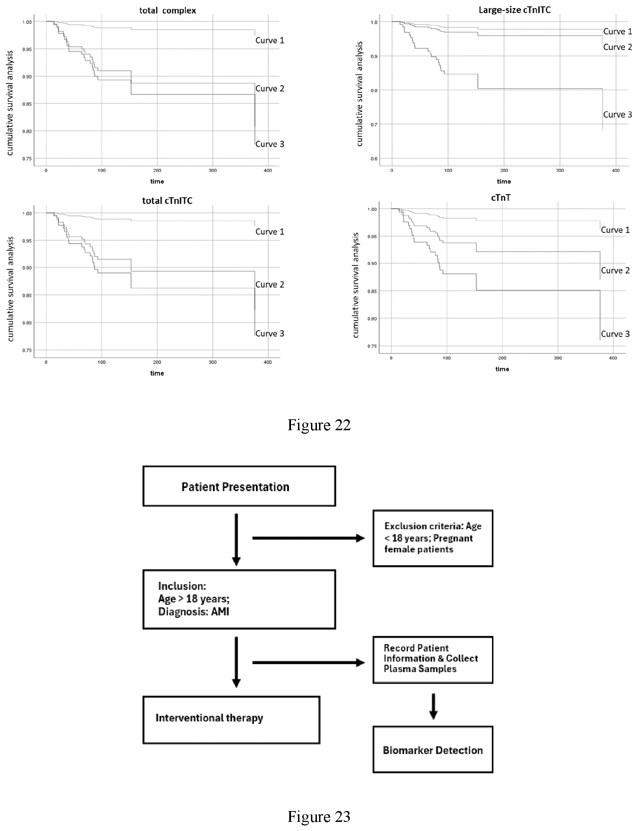

COX regression was used to analyze the risk of composite endpoint events in patients of different troponin fragment and complex concentration groups (Table 6-9), and the survival curves of different patient groups were plotted (Figure 22). Cox proportional hazard model was used to determine the relationship between the marker parameters or combination thereof and the ending of the studied subjects. The hazard ratio (HR) was calculated based on the Cox proportional hazard model to analyze the risk level of different risk groups of marker parameter values, i.e., the fold increases in the risk of endpoint events relative to the baseline group. *P* value <0.05 was considered significant.

The analysis found that for the total complex group 2 (median concentration - 75^{th} percentile concentration, 2964-9605 ng/L) and group 3 (> 75^{th} percentile concentration 9605 ng/L), P values were less than 0.05, and HR values were 8.0 and 9.63, respectively; for the large-size cTnITC group 3 (> 75^{th} percentile concentration 997 ng/L), *P* value was less than 0.05, and HR value was 9.9; for the total cTnITC group 2 (median concentration - 75^{th} percentile concentration, 956-4220 ng/L) and group 3 (> 75^{th} percentile concentration 4220 ng/L), *P* values were less than 0.05, and HR values were 7.8 and 10.1, respectively; for the cTnT group 3 (> 75^{th} percentile concentration 809 ng/L), *P* value was less than 0.05, and **HR** value was 7.2. The above results showed that total complex concentration higher than 2964 ng/L, large-size cTnITC concentration higher than 997 ng/L, total cTnITC concentration higher than 956 ng/L, and cTnT concentration higher than 809 ng/L are risk factors for patients to have composite endpoint events.

**Table 6-9: Risk of occurrence of composite endpoint events in different troponin fragment concentration groups (COX regression analysis)**

| Marker parameter | Concentration group | P value | HR | 95% CI lower limit | 95% CI upper limit |
|---|---|---|---|---|---|
| Total complex | Group 1 | - | 1 | | |
| | Group 2 | 0.008 | 8.050 | 1.722 | 37.634 |
| | Group 3 | 0.004 | 9.638 | 2.093 | 44.391 |
| Large-size cTnITC | Group 1 | - | 1 | | |
| | Group 2 | 0.447 | 1.862 | 0.375 | 9.244 |
| | Group 3 | 0.000 | 9.856 | 2.818 | 34.472 |
| Total cTnITC | Group 1 | - | 1 | | |
| | Group 2 | 0.010 | 7.768 | 1.641 | 36.787 |
| | Group 3 | 0.003 | 10.143 | 2.227 | 46.195 |
| cTnT | Group 1 | - | 1 | | |
| | Group 2 | 0.068 | 3.650 | 0.908 | 14.667 |
| | Group 3 | 0.002 | 7.190 | 2.009 | 25.739 |

The above data showed that large-size cTnITC or total cTnITC, preferably large-size cTnITC, could be used for the prognosis of postoperative myocardial injury in patients undergoing cardiac surgery, and the grouping data also showed that the higher the concentration of this marker, the higher the risk and the worse the prognosis.

### (II) Section of myocardial infarction

### 1. Patient enrollment and follow-up method

Acute myocardial infarction (Type 1) patients were enrolled, and patients with admission diagnosis and final diagnosis of Type 1 acute myocardial infarction were enrolled. All were aged 18 years or older. Acute myocardial infarction was independently diagnosed by hospital cardiologists in accordance with its definition. The determination of acute myocardial infarction was established based on clinical examinations, including physical examination, echocardiography, electrocardiography, high-sensitivity troponin I detection, high-sensitivity troponin T detection and coronary angiography. Patients with a final diagnosis other than acute myocardial infarction and those with incomplete diagnostic information were excluded. Patients under 18 years of age and pregnant female patients were excluded. The first lithium heparin plasma samples of these patients at admission time and prior to interventional treatment were collected for the analysis of troponin complexes and fragment composition. The patient enrollment process was shown in Figure 23.

The patient information was recorded, including: age, gender, past medical history, glomerular filtration rate, etc. The samples that met the enrollment criteria were selected for detection of relevant markers. A total of 324 patients with acute myocardial infarction were enrolled. The patient information was shown in Tables 6-10.

**Table 6-10: Characteristics of enrolled patients**

| Acute myocardial injury | Acute myocardial infarction |
|---|---|
| Total number of patients | n= 324 |
| Age | 60 (53 - 69) |
| Male | 255 (79%) |
| Creatinine, mmol/L | 85.0 (72.8-102.2) |
| Glomerular filtration rate, eGFR (mL/min/1.73 m2) | 80.9 (64.3-93.1) |

### Among them, continuous variables were presented as medians (25%-75% quartiles); and categorical variables were presented as counts (percentages).

Patients were followed up for 3 months or 1 year in this study, and clinic visits, telephone follow-up, and/or collection of electronic medical records may be performed to record information about clinical events. Major clinical events included composite events of all-cause mortality, myocardial infarction, and unplanned coronary revascularization. Minor clinical events included: cardiovascular death, various components of major clinical events, stroke, hospitalization for heart failure or emergency observation for 24 hours or more, cardiac arrest or malignant arrhythmia and other hospitalization events due to cardiovascular disease, as well as different combination composite endpoints of the above events.

### 2. Evaluation of the predictive ability of troponin fragments and complexes for prognostic risk of patients

In this study, a total of 324 patients with acute myocardial infarction were enrolled. Within 3 months after hospital discharge, the total mortality rate was 0.6% (2/324), and the incidence of the composite endpoint of death and adverse cardiovascular events was 6.5% (21/324). Within 1 year after discharge, the total mortality rate was 1.2% (4/324), and the incidence of the composite endpoint of death and adverse cardiovascular events was 9.6% (31/324).

The patients were stratified into different subgroups according to the median or 25^{th} percentile of troponin concentrations. The subgroup concentrations corresponding to troponin fragments or complexes were shown in Tables 6-11.

**Table 6-11: Concentration ranges corresponding to different troponin fragment and complex concentration groups**

| Marker parameter | Group 1 | Group 2 |
|---|---|---|
| Total complex (ng/L) | ≤ 43.5 | > 43.5 |
| Large-size cTnITC (ng/L) | ≤ 1.1 | > 1.1 |
| Total cTnITC (ng/L) | ≤ 38.8 | > 38.8 |
| cTnT (ng/L) | ≤ 136.8 | > 136.8 |

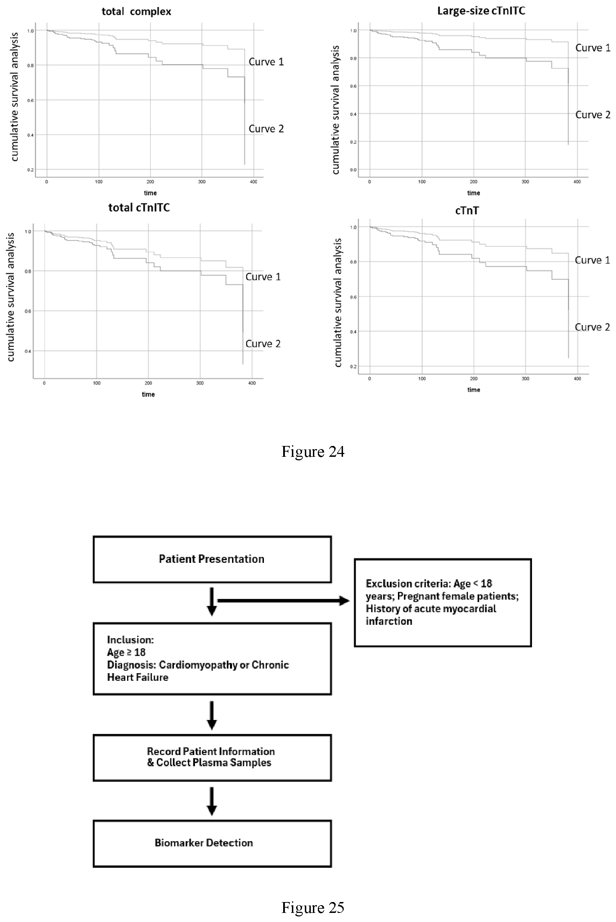

COX regression was used to analyze the risk of composite endpoint events in patients stratified by different concentration groups of troponin fragment and complex (Table 6-12), and survival curves were plotted for the respective patient groups (Figure 24). The analysis found that for the large-size cTnITC concentration > 25^{th} percentile concentration (1.1 ng/L), *P* value was less than 0.05 with a HR value of 3.6; for the cTnT concentration > median concentration (136.8 ng/L), P value was less than 0.05 with a HR value of 2.2. The above results showed that elevated large-size cTnITC and cTnT concentrations were risk factors for composite endpoint events in patients.

**Table 6-12: Risk of occurrence of composite endpoint events in different troponin fragment concentration groups (COX regression analysis)**

| Marker parameter | Concentration group | P value | HR | 95% CI lower limit | 95% CI upper limit |
|---|---|---|---|---|---|
| Total complex | Group 1 | - | 1 | | |
| | Group 2 | 0.075 | 2.721 | 0.906 | 8.175 |
| Large-size cTnITC | Group 1 | - | 1 | | |
| | Group 2 | 0.046 | 3.581 | 1.024 | 12.525 |
| Total cTnITC | Group 1 | - | 1 | | |
| | Group 2 | 0.252 | 1.555 | 0.731 | 3.306 |
| cTnT | Group 1 | - | 1 | | |
| | Group 2 | 0.049 | 2.163 | 0.998 | 4.685 |

The above data showed that the large-size cTnITC could be used for the prognosis of myocardial injury in patients with myocardial infarction, and the grouping data also showed that the higher the concentration of the marker, the higher the risk and the worse the prognosis.

### Example 7. Evaluation of prognosis of patients with chronic myocardial injury

This example illustrated the application of the markers of the present application in the prognosis of myocardial injury in patients with chronic myocardial injury, such as chronic heart failure or cardiomyopathy.

### 1. Patient enrollment and follow-up method

For the enrollment of patients with chronic cardiac events, patients with confirmed admission diagnosis and final diagnosis of chronic heart failure or cardiomyopathy were selected, and all were aged 18 years or older. Patients with final diagnosis other than chronic heart failure or cardiomyopathy and those also diagnosed with acute myocardial infarction were excluded. Patients under 18 years old and pregnant female patients were also excluded. Cardiomyopathy and chronic heart failure were independently diagnosed by hospital clinicians. Acute myocardial infarction was independently diagnosed by hospital cardiologists in accordance with its definition. The first lithium heparin plasma samples after admission of these patients were collected for analysis of troponin complex and fragment composition. The patient enrollment process was shown in Figure 25.

The patient information was recorded, including: age, gender, past medical history, glomerular filtration rate, etc. Samples that met the enrollment criteria were selected for detection of relevant markers. A total of 179 patients with chronic cardiac events were enrolled, of whom 29 were diagnosed with chronic heart failure, 62 had cardiomyopathy, and 88 had both chronic heart failure and cardiomyopathy. The patient information was shown in Table 7-1.

**Table 7-1: Characteristics of enrolled patients**

| Chronic cardiac event | Patients with chronic cardiac event |
|---|---|
| Total number of patients | n= 179 |
| Age | 60 (49-68) |
| Male | 127 (71%) |
| Creatinine, mmol/L | 82.0 (70.3-104.8) |
| Glomerular filtration rate, eGFR (mL/min/1.73 m2) | 67.7 (32.8-92.6) |

### Among them, continuous variables were presented as medians (25%-75% quartiles); categorical variables were presented as counts (percentages)

In this study, the patients were followed-up for 3 months or 1 year, and clinic visits, telephone follow-up, and/or collection of electronic medical records may be performed to record information about clinical events. Major clinical events included composite events of all-cause mortality, myocardial infarction, and unplanned coronary revascularization. Minor clinical events included cardiovascular death, components of major clinical event, stroke, hospitalization for heart failure or emergency observation for 24 hours or more, cardiac arrest or malignant arrhythmia and other hospitalization events due to cardiovascular disease, as well as different combination composite endpoints of the above events.

### 2. Evaluation of the predictive ability of troponin fragments and complexes for prognostic risk of patients

In this study, a total of 179 patients with chronic cardiac events were enrolled, of whom 29 (16%) had chronic heart failure, 62 (35%) had cardiomyopathy, and 88 (49%) had chronic heart failure and cardiomyopathy. Within 3 months after hospital discharge, the total mortality rate was 0.6% (1/179), and the incidence of the composite endpoint of death and adverse cardiovascular events was 12.8% (23/179). Within 1 year after hospital discharge, the total mortality rate was 1.1% (2/179), and the incidence of the composite endpoint of death and adverse cardiovascular events was 24.0% (43/179).

The ROC curve analysis was used to evaluate the efficacy of troponin complex and fragment markers in predicting the prognosis risk of patients (Table 7-2). In this example, patients with chronic cardiac events suffered from cardiomyopathy or chronic heart failure. The predicted event was a composite endpoint event of death and adverse cardiovascular events within 1 year after hospital discharge. The variables included troponin total complex concentration, large-size cTnITC concentration, total cTnITC concentration, and cTnT concentration.

Among them, for the total complex concentration, large-size cTnITC concentration, and cTnT concentration, *P* values were all less than 0.05, indicating that the variables showed statistically significant differences. Among them, the area under curve (AUC) of the absolute concentration of large-size cTnITC was 0.614, indicating that it exhibited certain predictive ability for adverse cardiovascular events and death events of the patient within one year. The results showed that the troponin total complex concentration, large-size cTnITC concentration, and cTnT concentration could be used to evaluate the prognosis risk of patients.

**Table 7-2: Evaluation of ability of troponin complex and fragment markers to predict prognosis risk of patients**

| Marker parameter | ROC_AUC | Standard error | *P* | 95% CI lower limit | 95% CI upper limit |
|---|---|---|---|---|---|
| Total complex concentration | 0.650 | 0.046 | 0.003 | 0.560 | 0.740 |
| Large-size cTnITC concentration | 0.614 | 0.047 | 0.027 | 0.522 | 0.705 |
| Total cTnITC concentration | 0.572 | 0.049 | 0.159 | 0.476 | 0.668 |
| cTnT concentration | 0.757 | 0.040 | 0.000 | 0.678 | 0.836 |

The sensitivity and specificity corresponding to different CUTOFF values using the total troponin complex concentration, large-size cTnITC concentration, and cTnT concentration as markers were used to calculate Youden indexes, and the optimal CUTOFF values were determined based on the maximum values of the Youden indexes (Table 7-3). When the total complex concentration was higher than 11.7 ng/L, the predicted sensitivity was 86% and the specificity was 42%; when the large-size cTnITC concentration was higher than 0.3 ng/L (about twice the LoD), the predicted sensitivity was 93% and the specificity was 30%; when the cTnT concentration was higher than 24.3 ng/L, the predicted sensitivity was 83% and the specificity was 63%.

**Table 7-3: Sensitivity and specificity corresponding to the cutoff values of troponin complex and fragment markers for predicting prognosis risk of patients**

| Marker parameter | ≥ CUTOFF | Sensitivity | Specificity | Youden index |
|---|---|---|---|---|
| Total complex concentration | 11.7330 | 0.857 | 0.421 | 0.278 |
| Large-size cTnITC concentration | 0.2925 | 0.929 | 0.308 | 0.237 |
| cTnT concentration | 24.2795 | 0.833 | 0.632 | 0.465 |

### 3. Risk of occurrence of composite endpoint events in different troponin fragment concentration groups

Patients were stratified into different subgroups according to troponin concentration, including group 1 "< 25^{th} percentile concentration", group 2 "25^{th} percentile concentration - median concentration", group 3 "> median concentration". The subgroup concentrations corresponding to markers were shown in Table 7-4.

**Table 7-4: Corresponding concentration ranges for different troponin fragment and complex concentration groups**

| Marker parameter | Group 1 | Group 2 | Group 3 |
|---|---|---|---|
| Total complex (ng/L) | ≤ 8.1 | 8.1 - 19.5 | > 19.5 |
| Large-size cTnITC (ng/L) | ≤ 0.3 | 0.3 - 0.7 | > 0.7 |
| Total cTnITC (ng/L) | ≤ 1.4 | 1.4 - 2.7 | > 2.7 |
| cTnT (ng/L) | ≤ 13.7 | 13.7 - 23.8 | > 23.8 |

Binary logistic regression analysis was used to analyze the risk of occurrence of composite endpoint events in patients of different troponin fragment and complex concentration groups (Table 7-5). The analysis found that for the total complex group 3 (> median concentration 19.5 ng/L), *P* value was less than 0.05 with an OR value of 4.3; for the large-size cTnITC group 2 (25^{th} percentile concentration - median concentration, 0.3 - 0.7 ng/L) and group 3 (> median concentration 0.7 ng/L), *P* values were less than 0.05 with an OR values of 4.0 and 3.1, respectively; for the cTnT group 3 (> median concentration 23.8 ng/L), *P* value was less than 0.05 with an OR value of 13.8. The above results showed that total complex concentration higher than 19.5 ng/L, large-size cTnITC concentration higher than 0.3 ng/L, and cTnT concentration higher than 23.8 ng/L are risk factors for occurrence of composite endpoint events in patients.

**Table 7-5: Risk of occurrence of composite endpoint events in different troponin fragment concentration groups**

| Marker parameter | Concentration group | P value | OR | 95% CI lower limit | 95% CI upper limit |
|---|---|---|---|---|---|
| Total complex | Group 1 | - | 1 | | |
| | Group 2 | 0.117 | 2.714 | 0.780 | 9.447 |
| | Group 3 | 0.010 | 4.373 | 1.427 | 13.406 |
| Large-size cTnITC | Group 1 | - | 1 | | |
| | Group 2 | 0.015 | 4.055 | 1.316 | 12.496 |
| | Group 3 | 0.033 | 3.102 | 1.098 | 8.764 |
| Total cTnITC | Group 1 | - | 1 | | |
| | Group 2 | 0.054 | 2.860 | 0.983 | 8.318 |
| | Group 3 | 0.121 | 2.174 | 0.814 | 5.809 |
| cTnT | Group 1 | - | 1 | | |
| | Group 2 | 0.265 | 2.628 | 0.481 | 14.350 |
| | Group 3 | 0.001 | 13.798 | 3.133 | 60.771 |

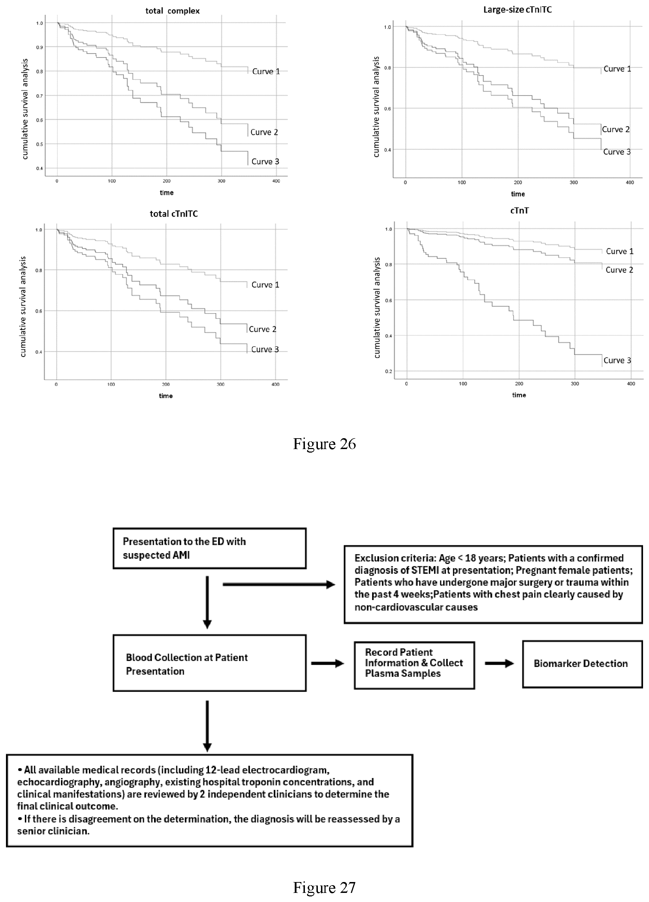

COX regression was used to analyze the risk of occurrence of composite endpoint events in patients stratified by different concentration groups of troponin fragment and complex (Table 7-6), and survival curves were plotted for respective patient groups (Figure 26). The analysis found that for the total complex group 3 (> median concentration 19.5 ng/L), *P* value was less than 0.05 with a HR value of 3.8; for the large-size cTnITC group 2 (25^{th} percentile concentration - median concentration, 0.3 - 0.7 ng/L) and group 3 (> median concentration 0.7 ng/L), *P* values were less than 0.05 with a HR values of 3.4 and 2.8, respectively; for the total cTnITC group 2 (25^{th} percentile concentration - median concentration, 1.4 - 2.7 ng/L), *P* value was less than 0.05 with a HR value of 2.8; for the cTnT group 3 (> median concentration 23.8 ng/L), *P* value was less than 0.05 with a HR value of 9.9. The above results showed that total complex concentration higher than 19.5 ng/L, large-size cTnITC concentration higher than 0.3 ng/L, total cTnITC concentration between 1.4 - 2.7 ng/L, and cTnT concentration higher than 23.8 ng/L were risk factors for occurrence of composite endpoint events in patients.

**Table 7-6: Risk of occurrence of composite endpoint events in different troponin fragment concentration groups COX regression analysis)**

| Marker parameter | Concentration group | P value | HR | 95% CI lower limit | 95% CI upper limit |
|---|---|---|---|---|---|
| Total complex | Group 1 | - | 1 | | |
| | Group 2 | 0.095 | 2.689 | 0.840 | 8.606 |
| | Group 3 | 0.013 | 3.762 | 1.316 | 10.750 |
| Large-size cTnITC | Group 1 | - | 1 | | |
| | Group 2 | 0.017 | 3.472 | 1.245 | 9.681 |
| | Group 3 | 0.034 | 2.840 | 1.081 | 7.466 |
| Total cTnITC | Group 1 | - | 1 | | |
| | Group 2 | 0.040 | 2.772 | 1.048 | 7.333 |
| | Group 3 | 0.125 | 2.091 | 0.816 | 5.359 |
| cTnT | Group 1 | - | 1 | | |
| | Group 2 | 0.523 | 1.720 | 0.326 | 9.062 |
| | Group 3 | 0.002 | 9.872 | 2.320 | 42.014 |

The above data showed that the large-size cTnITC could be used for the prognosis of myocardial injury in patients with chronic cardiac events, such as chronic heart failure or cardiomyopathy, and the grouping data also showed that the higher the concentration of this marker, the higher the risk and the worse the prognosis.

### Example 8. Excluding chest pain subjects without myocardial injury events

### 1. Patient enrollment and diagnosis

Patients with acute chest pain suspected of having coronary syndrome who continuously hospitalized were enrolled from the emergency department to establish the threshold and process for the rapid exclusion of patients with non-ST-segment elevation myocardial infarction (NSTEMI), and evaluate the safety and effectiveness thereof. Enrollment criteria: (1) Chinese population aged 18 years or older, (2) patients seeking medical attention due to symptoms or signs of suspected acute myocardial infarction (possible cardiac symptoms included: acute chest, upper abdomen, neck, jaw or arm pain or discomfort or pressure), (3) blood sampling at admission; Exclusion criteria: patients clearly diagnosed with STEMI at admission; pregnant female patients; patients who had undergone major surgery and trauma within four weeks; and patients with chest pain clearly caused by non-cardiovascular reasons.

Acute myocardial infarction was independently diagnosed by hospital cardiologists in accordance with its definition. Acute myocardial infarction was diagnosed through clinical examinations, including physical examination, echocardiography, electrocardiography, high-sensitivity troponin I detection, high-sensitivity troponin T detection and coronary angiography. Lithium heparin plasma samples were collected prior to the patient received treatment for analysis of troponin complex and fragment composition. The patient enrollment process was shown in Figure 27.

The patient information was recorded, including age, gender, past medical history, hypertension, diabetes mellitus, smoking status, creatinine level, and glomerular filtration rate. Samples that met the enrollment criteria were selected for detection of relevant markers. A total of 1,210 patients with suspected acute myocardial infarction were enrolled, including 138 diagnosed with NSTEMI and 1,072 without myocardial infarction. The key patient information was shown in Table 8-1.

**Table 8-1: Characteristics of enrolled patients**

| Information of patients with suspected acute myocardial infarction | | | |
|---|---|---|---|
| | Total | NSTEMI | non-MI |
| Number of patients | 1210 | 138 (11%) | 1072 (89%) |
| Age | 61 (51 - 70) | 66 (59 - 74) | 60 (50 - 69) |
| Male | 689 (57%) | 94 (68%) | 595 (55%) |
| Glomerular filtration rate, eGFR (mL/min/1.73 m2) | 180 (15%) | 84 (68 - 102) | 88 (71 - 99) |

Among them, continuous variables were presented as medians (25%-75% quartiles); categorical variables were presented as counts (percentages).

### 2. Evaluation of efficacy of troponin fragments and complex for excluding NSTEMI

In this example, all enrolled patients with suspected acute coronary syndrome symptoms were selected for analysis, and a total of 1210 suspected NSTEMI patients were enrolled, including 138 NSTEMI patients. Table 8-2 showed the diagnostic performance of the troponin complex and fragment markers used.

Among them, the absolute concentration of total complex, the absolute concentration of large-size cTnITC, the absolute concentration of total cTnITC, the absolute concentration of cTnT, and the absolute concentration of cTnI all had P values less than 0.05, indicating that the variables showed statistically significant differences. The area under curve (AUC) of the absolute concentration of large-size cTnITC was 0.959, indicating good diagnostic performance. The measured values obtained from the cTnI kit were comparable to those of the total complex, indicating clinical equivalence between the two. The results showed that the absolute concentration of large-size cTnITC or the absolute concentration of total cTnITC alone could more effectively predict NSTEMI. Among them, the large-size cTnITC was preferred.

**Table 8-2: Analysis of AUC values of troponin complex and fragment markers for diagnosis of NSTEMI**

| Marker parameter | ROC_AUC | Standard error | P | 95% CI lower limit | 95% CI upper limit |
|---|---|---|---|---|---|
| Total complex absolute concentration | 0.953 | 0.009 | 0.000 | 0.935 | 0.971 |
| Large-size cTnITC absolute concentration | 0.959 | 0.008 | 0.000 | 0.943 | 0.975 |
| Total cTnITC absolute concentration | 0.889 | 0.016 | 0.000 | 0.858 | 0.921 |
| cTnT absolute concentration | 0.927 | 0.011 | 0.000 | 0.904 | 0.949 |
| cTnI absolute concentration | 0.950 | 0.010 | 0.000 | 0.930 | 0.969 |

In patients with suspected myocardial infarction, using a very low troponin threshold could achieve early and safe exclusion of patients without myocardial infarction at admission. The exclusion thresholds for troponin complexes and fragments were established based on the myocardial infarction exclusion efficiency criteria recommended by the guidelines, which required a diagnostic sensitivity of not less than 99% and a tested negative predictive value NPV of not less than 99.5%. According to the exclusion threshold, the sensitivity and NPV for excluding NSTEMI patients were calculated, along with the proportion of excluded patients. The calculation method was shown in Table 8-3. The established thresholds and diagnostic efficiency data were shown in Table 8-4. These data further showed that the absolute concentrations of large-size cTnITC and total cTnITC could assist in the diagnosis of myocardial infarction and be used to exclude patients with non-myocardial injury. Among these, the measured value of large-size cTnITC could safely exclude a higher proportion of patients without myocardial infarction, and its efficiency was similar to that of total complex, and superior to that of cTnT.

**Table 8-3: Analysis method of diagnostic sensitivity and negative predictive value (NPV)**

| | Clinical judgment result | | |
|---|---|---|---|
| Test judgment result | Confirmed | Excluded | Total |
| Positive | a | b | a+b |
| Negative | c | d | c+d |
| Total | a+c | b+d | n(a+b+c+d) |

$Sensitivity = a / \left(a+c\right) \times 100 % , NPV = d / \left(c+d\right) \times 100 %$

**Table 8-4: Threshold and diagnostic performance of troponin complex and fragment markers for excluding NSTEMI**

| Marker parameter | Exclusion threshold | Sensitivity | NPV | Ratio of excluded patients |
|---|---|---|---|---|
| Total complex absolute concentration | 2.70 | 99.3% | 99.8% | 54% |
| Large-size cTnITC absolute concentration | 0.14 | 99.3% | 99.8% | 52% |
| Total cTnITC absolute concentration | 0.34 | 99.3% | 99.5% | 16% |
| cTnT absolute concentration | 5.70 | 99.3% | 99.8% | 38% |

| | | | | |
|---|---|---|---|---|
| Note: Ratio of excluded patients = number of excluded patients (with measured marker values below the set exclusion threshold)/total number of patients | | | | |

### 3. Evaluation of efficacy of troponin fragments and complexes for excluding NSTEMI (early chest pain patients)

In this example, patients with chest pain onset within 24 hours were selected as early chest pain patients for analysis, and a total of 764 suspected NSTEMI patients were enrolled, including 88 NSTEMI patients. Table 8-5 showed the diagnostic performance of the troponin complex and fragment markers used. Among these, the absolute concentration of total complex, the absolute concentration of large-size cTnITC, the absolute concentration of total cTnITC, and the absolute concentration of cTnT all had P values of less than 0.05, indicating that the variables showed statistically significant differences. Among these, the area under curve (AUC) of the absolute concentration of large-size cTnITC was 0.976, which indicated it had good diagnostic performance.

**Table 8-5: Analysis of AUC values of troponin complex and fragment markers for diagnosis of NSTEMI (early chest pain patients, chest pain duration ≤ 24 hours)**

| Marker parameter | ROC_AUC | Standard error | *P* | 95% CI lower limit | 95% CI upper limit |
|---|---|---|---|---|---|
| Total complex absolute concentration | 0.960 | 0.012 | 0.000 | 0.936 | 0.984 |
| Large-size cTnITC absolute concentration | 0.976 | 0.006 | 0.000 | 0.964 | 0.988 |
| Total cTnITC absolute concentration | 0.896 | 0.019 | 0.000 | 0.859 | 0.933 |
| cTnT absolute concentration | 0.935 | 0.014 | 0.000 | 0.907 | 0.962 |

In patients with early chest pain suspected of having myocardial infarction (chest pain duration ≤ 24 hours), using a very low troponin threshold could achieve early and safe exclusion of patients without myocardial infarction at admission. The exclusion thresholds for troponin complexes and fragments were established based on the myocardial infarction exclusion efficiency criteria recommended by the guidelines, which required a diagnostic sensitivity of not less than 99% and a tested negative predictive value NPV of not less than 99.5%. According to the exclusion thresholds, the sensitivity and NPV for excluding NSTEMI patients were calculated, along with the proportion of the excluded patients. The established thresholds and diagnostic efficiency data were shown in Table 8-6.

**Table 8-6: Thresholds and diagnostic performance of troponin complex and fragment markers for excluding NSTEMI (early chest pain patients, chest pain duration ≤ 24 hours)**

| Marker parameter | Exclusion threshold | Sensitivity | NPV | Ratio of excluded patient |
|---|---|---|---|---|
| Total complex absolute concentration | 3.30 | 98.9% | 99.8% | 59% |
| Large-size cTnITC absolute concentration | 0.25 | 98.9% | 99.8% | 67% |
| Total cTnITC absolute concentration | 0.39 | 98.9% | 99.3% | 20% |
| cTnT absolute concentration | 5.70 | 98.9% | 99.7% | 39% |

| | | | | |
|---|---|---|---|---|
| Note: Ratio of excluded patients = number of excluded patients (with measured marker values lower than the set exclusion threshold)/total number of patients | | | | |

Patients with chest pain onset within 12 hours were selected as early chest pain patients for analysis in this example, a total of 617 patients with suspected NSTEMI were enrolled, including 59 NSTEMI patients. Table 8-7 showed the diagnostic performance of the troponin complex and fragment markers used. Among them, the absolute concentration of total complex, the absolute concentration of large-size cTnITC, the absolute concentration of total cTnITC, and the absolute concentration of cTnT all had *P* values of less than 0.05, indicating that the variables showed statistically significant differences. The area under curve (AUC) of the absolute concentration of large-size cTnITC was 0.972, which indicated that it had good diagnostic performance.

**Table 8-7: Analysis of AUC values of troponin complex and fragment markers for diagnosis of NSTEMI (early chest pain patients, chest pain duration ≤ 12 hours)**

| Marker parameter | ROC_AUC | Standard error | *P* | 95% CI lower limit | 95% CI upper limit |
|---|---|---|---|---|---|
| Total complex absolute concentration | 0.960 | 0.010 | 0.000 | 0.940 | 0.979 |
| Large-size cTnITC absolute concentration | 0.972 | 0.007 | 0.000 | 0.958 | 0.987 |
| Total cTnITC absolute concentration | 0.889 | 0.023 | 0.000 | 0.844 | 0.933 |
| cTnT absolute concentration | 0.932 | 0.014 | 0.000 | 0.904 | 0.960 |

In patients with early chest pain suspected of having myocardial infarction (chest pain duration ≤ 12 hours), using a very low troponin threshold could achieve early and safe exclusion of patients without myocardial infarction at admission. The exclusion thresholds for troponin complexes and fragments were established based on the myocardial infarction exclusion efficiency criteria recommended by the guidelines, which required a diagnostic sensitivity of not less than 99% and a tested negative predictive value NPV of not less than 99.5%. According to the exclusion thresholds, the sensitivity and NPV of excluding NSTEMI patients were calculated, along with the proportion of the excluded patients. The established thresholds and diagnostic efficiency data were shown in Table 8-8.

**Table 8-8: Thresholds and diagnostic performance of troponin complex and fragment markers for excluding NSTEMI (early chest pain patients, chest pain duration ≤ 12 hours)**

| Marker parameter | Exclusion threshold | Sensitivity | NPV | Ratio of excluded patient |
|---|---|---|---|---|
| Total complex absolute concentration | 3.50 | 98.3% | 99.7% | 61% |
| Large-size cTnITC absolute concentration | 0.30 | 98.3% | 99.8% | 72% |
| Total cTnITC absolute concentration | 1.35 | 98.3% | 99.6% | 43% |
| cTnT absolute concentration | 9.60 | 98.3% | 99.7% | 64% |

The above data showed that the large-size cTnITC complex of troponin and the total cTnITC complex could be used alone to assist in the diagnosis of myocardial infarction in the cohort of chest pain patients suspected of having myocardial infarction, as well as to exclude patients without myocardial injury. This enables patients to leave the emergency room early, thereby reducing their observation time and allowing them to receive diagnosis and treatment of other diseases, which could also accelerate the turnover of the emergency room.

In addition, in patients with early chest pain (chest pain time ≤ 12 hours or 24 hours), the measured value of large-size cTnITC could safely exclude a higher proportion of patients without myocardial infarction. Compared with the total complex and cTnT, the number of excluded patients accounted for a higher proportion of the total patient population, with superior diagnostic efficiency. By comparing the data of all chest pain patients within 24 hours of chest pain and those within 12 hours of chest pain, it showed that the larger-size cTnITC had a higher proportion of safe exclusion in patients with shorter chest pain time, and had more advantages than the total complex.

### 3. Evaluation of efficacy of combined detection of troponin fragment concentrations for excluding NSTEMI

Combined detection of multiple troponin fragment concentrations was used to rapidly exclude NSTEMI.

The detection markers included total complex concentration, large-size cTnITC concentration, total cTnITC concentration, and cTnT concentration. Large-size cTnITC concentration + total complex concentration were selected as characteristic variables for combined detection, and thresholds were established respectively. Patients with both large-size cTnITC concentration and total complex concentration lower than the threshold conditions were excluded to achieve maximum safety. The established thresholds and diagnostic performance data were shown in Tables 8-9. The above data showed that combined detection of multiple troponin fragment concentrations could be used to rapidly exclude patients with myocardial infarction and assist in the diagnosis of myocardial infarction. Compared with the use of a single marker, the combined detection of multiple troponin fragments could reduce the number of patients with missed diagnosis of myocardial infarction to 0, thereby achieving 100% sensitivity and 100% negative predictive value.

**Table 8-9: Thresholds and diagnostic performance of troponin complex and fragment markers in excluding NSTEMI (combined use)**

| Conditions for exclusion of patients | Missed diagnosis of NSTEMI | Sensitivity | NPV | Ratio of excluded patients |
|---|---|---|---|---|
| Total complex absolute concentration <4.20 ng/L, and large-size cTnITC concentration <0.18 ng/L | 1 | 99.3% | 99.8% | 52% |
| Total complex absolute concentration <2.70 ng/L, and large-size cTnITC concentration <0.18 ng/L | 0 | 100% | 100% | 46% |
| Total complex absolute concentration <4.20 ng/L, and large-size cTnITC concentration <0.14 ng/L | 0 | 100% | 100% | 46% |
| Total complex absolute concentration <2.70 ng/L | 1 | 99.3% | 99.8% | 54% |
| Large-size cTnITC concentration <0.14 ng/L | 1 | 99.3% | 99.8% | 52% |

The data in Tables 8-9 showed that a single marker could not effectively solve the problem of missed diagnosis, but combined detection could achieve this, and the exclusion ratio did not decrease significantly. Thus, it was the preferred solution to resolve the contradiction between missed diagnosis and the proportion of excluded patients.

This example showed that the large-size cTnITC complex of troponin and the total cTnITC complex could exclude patients without myocardial injury from patients with clinical chest pain. The large-size cTnITC complex was preferred, and its diagnostic effect was superior to that of cTnT; it is particularly more advantageous in patients with early chest pain. Furthermore, its combination with the total complex could avoid missed diagnosis while maintaining a favorable proportion of excluded patients, showing good diagnostic performance in excluding myocardial injury events in subjects with chest pain.

In addition to those described herein, various modifications of the present invention will be apparent to those skilled in the art based on the foregoing description. Such modifications are also intended to fall within the scope of the appended claims. The full scope of the present invention is given by the appended claims and any equivalents thereof.

## Claims

1. A method for assessment of myocardial injury in a subject *in vitro*, comprising:
detecting the concentration of one or more myocardial injury markers in a sample from the subject;
obtaining a characteristic parameter for assessment of myocardial injury based on the concentration of the one or more myocardial injury markers;
comparing the characteristic parameter with a reference value of the characteristic parameter;
assessing myocardial injury of the subject based on the result of the comparison;
wherein the one or more myocardial injury markers comprise a large-size cardiac troponin ternary complex and/or a total cardiac troponin ternary complex;
the large-size cardiac troponin ternary complex is a complex formed by full-length troponin C or any fragment thereof, full-length cardiac troponin I or any fragment thereof, one or more fragments of amino acid residues 223-287 of cardiac troponin T and one or more fragments of amino acid residues 1-222 of cardiac troponin T;
the total cardiac troponin ternary complex is a complex formed by full-length troponin C or any fragment thereof, full-length cardiac troponin I or any fragment thereof, one or more fragments of amino acid residues 223-287 of cardiac troponin T, and optionally one or more fragments of amino acid residues 1-222 of cardiac troponin T.

2. The method according to claim 1, wherein the myocardial injury marker further comprises one or more of the following:
a cTnI, comprising full-length cardiac troponin I or any fragment thereof;
a cTnT, comprising full-length cardiac troponin T or any fragment thereof;
a TnC, comprising full-length troponin C or any fragment thereof;
a cardiac troponin binary complex, comprising a binary complex consisting of full-length troponin C or any fragment thereof and full-length cardiac troponin I or any fragment thereof; and
a total cardiac troponin complex, comprising the total cardiac troponin ternary complex, a binary complex consisting of full-length troponin C or any fragment thereof and full-length cardiac troponin I or any fragment thereof;
preferably, the one or more myocardial injury markers further comprise at least one of cTnI, cTnT and total cardiac troponin complex.

3. The method according to claim 1 or 2, wherein the step of obtaining the characteristic parameter for assessment of myocardial injury based on the concentration of the one or more myocardial injury markers comprises:
determining the concentration of the large-size cardiac troponin ternary complex or total cardiac troponin ternary complex as the characteristic parameter for assessment of myocardial injury; or
obtaining the characteristic parameter for assessment of myocardial injury based on the concentration of the large-size cardiac troponin ternary complex and the concentration of the total cardiac troponin ternary complex.

4. The method according to any one of claims 1 to 3, wherein the step of obtaining the characteristic parameter for assessment of myocardial injury based on the concentration of the one or more myocardial injury markers comprises: inputting the concentration of the one or more myocardial injury markers into a preset function model to obtain an output of the preset function model as the characteristic parameter for assessment of myocardial injury.

5. The method according to any one of claims 1 to 4, wherein the step of obtaining the characteristic parameter for assessing myocardial injury based on the concentration of the one or more myocardial injury markers comprises: determining one of the following ratio parameters as the characteristic parameter for assessment of myocardial injury, or obtaining the characteristic parameter for assessment of myocardial injury based on at least two of the following ratio parameters, or obtaining the characteristic parameter for assessment of myocardial injury based on at least one of the following ratio parameters and the ratio of the concentration of cTnT to the concentration of total cardiac troponin complex;
the ratio parameters comprise: the ratio of the concentration of large-size cardiac troponin ternary complex to the concentration of cTnI, the ratio of the concentration of large-size cardiac troponin ternary complex to the concentration of cTnT, the ratio of the concentration of large-size cardiac troponin ternary complex to the concentration of total cardiac troponin complex, the ratio of the concentration of total cardiac troponin ternary complex to the concentration of cTnI, the ratio of the concentration of total cardiac troponin ternary complex to the concentration of cTnT, the ratio of the concentration of total cardiac troponin ternary complex to the concentration of total cardiac troponin complex, and the ratio of the concentration of large-size cardiac troponin ternary complex to the concentration of total cardiac troponin ternary complex;
preferably, the ratio parameter is the ratio of the concentration of large-size cardiac troponin ternary complex to the concentration of cTnI, cTnT or total cardiac troponin complex;
optionally, the ratio parameters further comprises the ratio of the concentration of total cardiac troponin ternary complex to the concentration of cTnI, cTnT or total cardiac troponin complex, and/or the ratio of the concentration of cTnT to the concentration of cTnI or total cardiac troponin complex.

6. The method according to any one of claims 1 to 5, wherein the method is used for the diagnosis of myocardial injury in the subject; preferably,
the characteristic parameter is used for the diagnosis of myocardial injury in the subject, wherein the characteristic parameter is obtained based on the large-size cardiac troponin ternary complex.

7. The method according to any one of claims 1 to 5, wherein the method is used for the evaluation of prognosis of myocardial injury in the subject; preferably, the characteristic parameter is used for the evaluation of prognosis of myocardial injury in the subject, wherein the characteristic parameter is obtained based on the large-size cardiac troponin ternary complex.

8. The method according to any one of claims 1 to 6, wherein the step of assessing myocardial injury of the subject based on the result of the comparison comprises:
staging myocardial infarction of the subject based on the result of the comparison;
preferably, when the characteristic parameter is higher than the reference value of the characteristic parameter, the subject is judged to be in the early stage of acute myocardial infarction.

9. The method according to claim 8, wherein the step of obtaining the characteristic parameter for assessment of myocardial injury based on the concentration of the one or more myocardial injury markers comprises:
determining the concentration of the large-size cardiac troponin ternary complex as the characteristic parameter for assessment of myocardial injury; or
obtaining the characteristic parameter for assessment of myocardial injury based on the concentration of the large-size cardiac troponin ternary complex and the concentration of at least one of cTnI, cTnT, total cardiac troponin ternary complex and total cardiac troponin complex, preferably based on the concentration of the large-size cardiac troponin ternary complex and the concentration of cTnT; or
obtaining the characteristic parameter for assessment of myocardial injury based on the ratio of the concentration of the large-size cardiac troponin ternary complex or the concentration of the total cardiac troponin ternary complex to the concentration of at least one of cTnI, cTnT and total cardiac troponin complex, and optionally the ratio of the concentration of cTnT to the concentration of cTnI or total cardiac troponin complex.

10. The method according to any one of claims 1 to 6, wherein the step of assessing the myocardial injury of the subject based on the result of the comparison comprises:
judging whether the subject has type I myocardial infarction or type II myocardial infarction based on the result of the comparison;
preferably, when the characteristic parameter is higher than the reference value of the characteristic parameter, judging that the subject has type I myocardial infarction; or
preferably, obtaining the characteristic parameter for assessment of myocardial injury based on the ratio of the concentration of large-size cardiac troponin ternary complex or the concentration of the total cardiac troponin ternary complex to the total cardiac troponin complex.

11. The method according to any one of claims 1 to 6, wherein the step of assessing the myocardial injury of the subject based on the result of the comparison comprises:
judging whether the subject has type I myocardial infarction or a chronic cardiac event based on the result of the comparison;
preferably, when the characteristic parameter is higher than the reference value of the characteristic parameter, judging that the subject has type I myocardial infarction.

12. The method according to claim 11, wherein the step of obtaining the characteristic parameter for assessment of myocardial injury based on the concentration of the one or more myocardial injury markers comprises:
determining the concentration of large-size cardiac troponin ternary complex or the concentration of total cardiac troponin ternary complex as the characteristic parameter for assessment of myocardial injury; or
obtaining the characteristic parameter for assessment of myocardial injury based on the concentration of large-size cardiac troponin ternary complex and the concentration of total cardiac troponin ternary complex; or
obtaining the characteristic parameter for assessment of myocardial injury based on the concentration of large-size cardiac troponin ternary complex or the concentration of total cardiac troponin ternary complex and the concentration of cTnT or total cardiac troponin complex;
preferably, obtaining the characteristic parameter for assessment of myocardial injury based on the ratio of the concentration of large-size cardiac troponin ternary complex or the concentration of total cardiac troponin ternary complex to the concentration of at least one of cTnI, cTnT and total cardiac troponin complex, and optionally the ratio of the concentration of cTnT to the concentration of cTnI or total cardiac troponin complex.

13. The method according to any one of claims 1 to 6, wherein the step of assessing the myocardial injury of the subject based on the result of the comparison comprises:
judging whether the subject has myocardial injury due to invasive procedure or chronic cardiac event based on the result of the comparison;
preferably, when the characteristic parameter is higher than the reference value of the characteristic parameter, the subject is judged to have myocardial injury due to invasive procedure.

14. The method according to claim 13, wherein the step of obtaining the characteristic parameter for assessment of myocardial injury based on the concentration of the one or more myocardial injury markers comprises:
determining the concentration of large-size cardiac troponin ternary complex or the concentration of total cardiac troponin ternary complex as the characteristic parameter for assessment of myocardial injury;
preferably, the characteristic parameter for assessment of myocardial injury is obtained based on the ratio of the concentration of large-size cardiac troponin ternary complex or the concentration of total cardiac troponin ternary complex to the concentration of at least one of cTnI, cTnT and total cardiac troponin complex, and optionally the ratio of the concentration of cTnT to the concentration of cTnI or total cardiac troponin complex.

15. The method according to any one of claims 1 to 6, comprising excluding the subject with chest pain who has not experienced myocardial injury event based on the result of the comparison;
preferably, excluding the subject with chest pain who has not experienced myocardial injury event when the characteristic parameter is lower than the reference value of the characteristic parameter;
preferably, the myocardial injury event is myocardial infarction or NSTEMI.

16. The method according to claim 15, wherein the step of obtaining the characteristic parameter for assessment of myocardial injury based on the concentration of the one or more myocardial injury markers comprises:
determining the concentration of large-size cardiac troponin ternary complex or the concentration of total cardiac troponin ternary complex as the characteristic parameter for assessment myocardial injury; or
obtaining the characteristic parameter for assessment of myocardial injury based on the concentration of large-size cardiac troponin ternary complex or the concentration of total cardiac troponin ternary complex and the concentration of total cardiac troponin complex.

17. The method according to claim 7, comprising assessing the prognosis of myocardial injury in the subject with acute myocardial injury; preferably, the subject is a subject who undergoes cardiac surgery or myocardial infarction;
preferably, judging the prognosis to be poor when the characteristic parameter is higher than the reference value of the characteristic parameter.

18. The method according to claim 17, wherein the step of obtaining the characteristic parameter for assessment of myocardial injury based on the concentration of the one or more myocardial injury markers comprises:
determining the concentration of large-size cardiac troponin ternary complex or total cardiac troponin ternary complex as the characteristic parameter for assessment of myocardial injury;
preferably, determining the change in the concentration of large-size cardiac troponin ternary complex or total cardiac troponin ternary complex in the plasma of the subject before and after cardiac surgery as a characteristic parameter for assessing prognosis risk of the cardiac surgery.

19. The method according to claim 7, comprising assessing prognosis of myocardial injury in the subject with chronic myocardial injury based on the result of the comparison;
preferably, the subject is a patient with cardiomyopathy, chronic heart failure, structural heart disease, infiltrative disease, stable coronary heart disease, or persistent arrhythmia;
preferably, judging the prognosis to be poor when the characteristic parameter is higher than the reference value of the characteristic parameter.

20. The method according to claim 19, wherein the step of obtaining the characteristic parameter for assessment of myocardial injury based on the concentration of the one or more myocardial injury markers comprises:
determining the concentration of large-size cardiac troponin ternary complex or total cardiac troponin ternary complex as the characteristic parameter for assessment of myocardial injury.

21. A device for obtaining a characteristic parameter for assessment of myocardial injury in a subject, comprising:
a data receiving module, configured to receive concentration data of one or more myocardial injury markers obtained from a sample from the subject, wherein the one or more myocardial injury markers comprise a large-size cardiac troponin ternary complex and/or a total cardiac troponin ternary complex;
a data processing module, configured to process the concentration data of the one or more myocardial injury markers received by the receiving module, and obtain the characteristic parameter for assessment of myocardial injury; and,
an output module, configured to output the characteristic parameter;
wherein, the large-size cardiac troponin ternary complex is a complex formed by full-length troponin C or any fragment thereof, full-length cardiac troponin I or any fragment thereof, one or more fragments of amino acid residues 223-287 of cardiac troponin T and one or more fragments of amino acid residues 1-222 of cardiac troponin T;
the total cardiac troponin ternary complex is a complex formed by full-length troponin C or any fragment thereof, full-length cardiac troponin I or any fragment thereof, one or more fragments of amino acid residues 223-287 of cardiac troponin T, and optionally one or more fragments of amino acid residues 1-222 of cardiac troponin T.

22. The device according to claim 21, wherein the data processing module is configured to process the concentration of the one or more myocardial injury markers to obtain the characteristic parameter for assessment of myocardial injury, comprising:
being configured to determine the concentration of cardiac troponin ternary complex or total cardiac troponin ternary complex as the characteristic parameter for assessment of myocardial injury; or
being configured to obtain the characteristic parameter for assessment of myocardial injury based on the concentration of cardiac troponin ternary complex and the concentration of total cardiac troponin ternary complex.

23. The device according to claim 21, wherein the myocardial injury markers further comprise one or more of the following:
a cTnI, comprising full-length cardiac troponin I or any fragment thereof;
a cTnT, comprising full-length cardiac troponin T or any fragment thereof;
a TnC, comprising full-length troponin C or any fragment thereof;
a cardiac troponin binary complex, comprising a binary complex consisting of full-length troponin C or any fragment thereof and full-length cardiac troponin I or any fragment thereof; and
a total cardiac troponin complex, comprising the total cardiac troponin ternary complex, a binary complex consisting of full-length troponin C or any fragment thereof and full-length cardiac troponin I or any fragment thereof;
preferably, the one or more myocardial injury markers further comprise at least one of cTnI, cTnT and total cardiac troponin complex.

24. The device according to any one of claims 21 to 23, wherein the data processing module being configured to process the concentration of the one or more myocardial injury markers to obtain the characteristic parameter for assessment of myocardial injury, comprising: being configured to input the concentration of the one or more myocardial injury markers into a preset function model to obtain an output of the preset function model as the characteristic parameter for assessment of myocardial injury.

25. The device according to any one of claims 21 to 24, wherein the data processing module being configured to process the concentration of the one or more myocardial injury markers to obtain the characteristic parameter for assessment of myocardial injury, comprising: being configured to use the data processing module to determine one of the following ratio parameters as the characteristic parameter for assessment of myocardial injury, or being configured to obtain the characteristic parameter for assessment of myocardial injury based on at least two of the following ratio parameters, or being configured to obtain the characteristic parameter for assessment of myocardial injury based on at least one of the following ratio parameters and the ratio of the concentration of cTnT to the concentration of total cardiac troponin complex;
the ratio parameters comprise: the ratio of the concentration of large-size cardiac troponin ternary complex to the concentration of cTnI, the ratio of the concentration of large-size cardiac troponin ternary complex to the concentration of cTnT, the ratio of the concentration of large-size cardiac troponin ternary complex to the concentration of total cardiac troponin complex, the ratio of the concentration of total cardiac troponin ternary complex to the concentration of cTnI, the ratio of the concentration of total cardiac troponin ternary complex to the concentration of cTnT, the ratio of the concentration of total cardiac troponin ternary complex to the concentration of total cardiac troponin complex, and the ratio of the concentration of large-size cardiac troponin ternary complex to the concentration of total cardiac troponin ternary complex;
preferably, the ratio parameter is the ratio of the concentration of large-size cardiac troponin ternary complex to the concentration of cTnI, cTnT or total cardiac troponin complex; optionally, the ratio parameters further comprise the ratio of the concentration of total cardiac troponin ternary complex to the concentration of cTnI, cTnT or total cardiac troponin complex, and/or the ratio of the concentration of cTnT to the concentration of cTnI or total cardiac troponin complex.

26. A sample analysis system, comprising:
a sample carrying portion, configured to carry a container containing a sample from a subject;
a sample dispensing portion, configured to pipette the sample from the subject from the sample carrying portion and discharge it into a reaction cup to be added with the sample;
a reagent carrying portion, configured to carry a detection reagent;
a reagent dispensing portion, configured to pipette the detection reagent from the reagent carrying portion and discharge it into a reaction cup to be added with the reagent;
a reaction portion, configured to place a reaction cup so as to incubate a test solution obtained by reacting the sample from the subject with the detection reagent in the reaction cup;
a detection portion, which has a signal detector and is configured to detect a signal of the test solution in the reaction cup to determine and output the concentration of one or more myocardial injury markers in the sample from the subject, wherein the one or more myocardial injury markers comprise a large-size cardiac troponin ternary complex and/or a total cardiac troponin ternary complex, wherein the large-size cardiac troponin ternary complex is a complex formed by full-length troponin C or any fragment thereof, full-length cardiac troponin I or any fragment thereof, one or more fragments of amino acid residues 223-287 of cardiac troponin T and one or more fragments of amino acid residues 1-222 of cardiac troponin T; the total cardiac troponin ternary complex is a complex formed by full-length troponin C or any fragment thereof, full-length cardiac troponin I or any fragment thereof, one or more fragments of amino acid residues 223-287 of cardiac troponin T, and optionally one or more fragments of amino acid residues 1-222 of cardiac troponin T;
a data processing portion, which comprises a processor and a computer-readable storage medium on which the computer-readable instructions stored, **characterized in that** the computer-readable instructions cause the processor to implement the following steps when executed by the processor:
receiving and processing the concentration of the one or more myocardial injury markers to obtain a characteristic parameter for assessment of myocardial injury; and
outputting the characteristic parameter.

27. Use of a reagent for quantitative detection of a large-size cardiac troponin ternary complex and/or a reagent for quantitative detection of a total cardiac troponin ternary complex in a sample in the manufacture of a kit, wherein the kit is used for assessment of myocardial injury in a subject,
wherein, the large-size cardiac troponin ternary complex is a complex formed by full-length troponin C or any fragment thereof, full-length cardiac troponin I or any fragment thereof, one or more fragments of amino acid residues 223-287 of cardiac troponin T and one or more fragments of amino acid residues 1-222 of cardiac troponin T;
the total cardiac troponin ternary complex is a complex formed by full-length troponin C or any fragment thereof, full-length cardiac troponin I or any fragment thereof, one or more fragments of amino acid residues 223-287 of cardiac troponin T, and optionally one or more fragments of amino acid residues 1-222 of cardiac troponin T.

28. The use according to claim 27, wherein the kit further comprises a reagent for quantitative detection of total cardiac troponin complex, a reagent for quantitative detection of cTnI and/or a reagent for quantitative detection of cTnT, wherein,
the cTnI comprises full-length cardiac troponin I or any fragment thereof;
the cTnT comprises full-length cardiac troponin T or any fragment thereof;
the total cardiac troponin complex comprises the total cardiac troponin ternary complex, a binary complex consisting of full-length troponin C or any fragment thereof and full-length cardiac troponin I or any fragment thereof.

29. The use according to claim 27 or 28, wherein the reagent for quantitative detection of large-size cardiac troponin ternary complex in the sample comprises a first group of antibodies and a second group of antibodies, wherein,
the first group of antibodies comprises one or more Antibodies **1-1**, each of the Antibodies **1-1** is independently selected from an antibody capable of specifically binding to any fragment of amino acid residues 67-222 of cTnT;
the second group of antibodies comprises one or more Antibodies **1-2**, each of the Antibodies **1-2** is independently selected from an antibody capable of specifically binding to any fragment of the amino acid sequence of TnC;
optionally, the second group of antibodies further comprises one or more Antibodies **1-3**, each of the Antibodies **1-3** is independently selected from an antibody capable of specifically binding to cTnIC; and/or,
one or more Antibodies **1-4**, each of the Antibodies **1-4** is independently selected from an antibody capable of specifically binding to any fragment of amino acid residues 18-210 of cTnI;
preferably, the first group of antibodies does not comprise an antibody capable of specifically binding to any fragment of amino acid residues 223-287 of cTnT;
preferably, the first group of antibodies are capture antibodies, and the second group of antibodies are detection antibodies.

30. The use according to any one of claims 27 to 29, wherein the reagent for quantitative detection of total cardiac troponin ternary complex in the sample comprises a first antibody group and a second antibody group, wherein:
the first detection reagent comprises one or more Antibodies **2-1**, each of the Antibodies **2-1** is independently selected from an antibody capable of specifically binding to any fragment of amino acid residues 223-287 of cTnT;
the second detection reagent comprises one or more Antibodies **2-2**, each of the Antibodies **2-2** is independently selected from an antibody capable of specifically binding to any fragment of the amino acid sequence of TnC;
optionally, the first detection reagent further comprises one or more Antibodies **2-3**, each of the Antibodies **2-3** is independently selected from an antibody capable of specifically binding to any fragment of amino acid residues 67-222 of cTnT;
optionally, the second detection reagent further comprises:
one or more Antibodies **2-4**, each of the Antibodies **2-4** is independently selected from an antibody capable of specifically binding to cTnIC; and/or
one or more Antibodies **2-5**, each of the Antibodies **2-5** is independently selected from an antibody capable of specifically binding to any fragment of amino acid residues 18-210 of cTnI;
preferably, the first antibody group is a capture antibody, and the second antibody group is a detection antibody.

31. Use according to any one of claims 27 to 30, wherein the kit is used for one or more of the following:
1) staging myocardial infarction, in particular judging whether the subject is in the early stage of myocardial infarction;
2) distinguishing between type I myocardial infarction and chronic cardiac event;
3) excluding a subject with chest pain who has not experienced myocardial injury event;
4) distinguishing between type I myocardial infarction and type II myocardial infarction;
5) distinguishing between myocardial injury due to invasive procedure and chronic cardiac event;
6) assessing prognosis of chronic myocardial injury; or
7) assessing prognosis of acute myocardial injury.
